# EUROPÄISCHE PATENTSCHRIFT

(11) **EP 3 854 388 B1**
(45) Veröffentlichungstag und Bekanntmachung des Hinweises auf die Patenterteilung: **04.10.2023**
(21) Anmeldenummer: 20153621.6
(22) Anmeldetag: 24.01.2020
(51) Int. Cl.: A61K 9/70, A61K 31/381, A61P 25/16

(54) **TRANSDERMALES THERAPEUTISCHES SYSTEM MIT DEM WIRKSTOFF ROTIGOTIN UND MINDESTENS EINEM NICHT AMINRESISTENTEN SILIKONKLEBER**
TRANSDERMAL THERAPEUTIC SYSTEM COMPRISING ROTIGOTINE AND AT LEAST ONE NON-AMINE-RESISTANT SILICONE ADHESIVE
SYSTÈME THÉRAPEUTIQUE TRANSDERMIQUE COMPORTANT LE PRINCIPE ACTIF ROTIGOTINE ET AU MOINS UNE COLLE SILICONE NON RÉSISTANT AUX AMINES

(43) Veröffentlichungstag der Anmeldung: 28.07.2021
(73) Patentinhaber: Luye Pharma Switzerland AG, 4051 Basel (CH)
(72) Erfinder: BENDA, Christian, 83607 Holzkirchen (DE); KOLLING, Ulrike, 83026 Rosenheim (DE); SCHURAD, Björn, 80897 München (DE); WAUER, Gabriel, 83727 Schliersee (DE)
(74) Vertreter: Lederer & Keller Patentanwälte Partnerschaft mbB

(56) Entgegenhaltungen:
- WO-A1-2011/076879
- WO-A1-2014/079573
- WO-A1-2014/198423
- WO-A1-2015/177209
- US-A1- 2005 260 254
- US-A1- 2009 299 304
- US-B1- 6 337 086

## Beschreibung

Die vorliegende Erfindung betrifft ein transdermales therapeutisches System (TTS) zur Verabreichung des Wirkstoffes Rotigotin umfassend einen oder mehrere nicht aminresistente Silikonkleber, sowie Verfahren zu dessen Herstellung. Das erfindungsgemäße transdermale therapeutische System ist insbesondere zur Behandlung der Parkinson-Erkrankung geeignet.

### Hintergrund der Erfindung

Rotigotin ist der INN der Verbindung (-)-5,6,7,8-Tetrahydro-6-[propyl-[2-(2-thienyl)ethyl]-amino]-1-naphthalenol der folgenden Strukturformel:

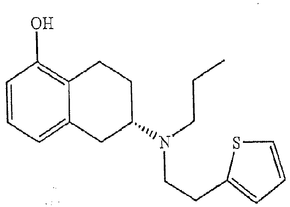

Gegenwärtig sind zwei kristalline Formen von Rotigotin bekannt: die polymorphe Form I und die polymorphe Form II (WO 2009/068520). Die polymorphe Form I und polymorphe Form II können durch ihre jeweiligen physikalisch-chemischen Parameter, wie Pulver-Röntgenbeugungsdiffraktogramm, Raman-Spektrum und Schmelzpunkt, unterschieden werden. Wie in der WO 2009/068520 beschrieben, ist die polymorphe Form II thermodynamisch stabiler als die polymorphe Form I und soll auch verbesserte Verarbeitungseigenschaften haben.

Rotigotin ist ein bekannter Dopaminrezeptoragonist, der erfolgreich zur Behandlung der Parkinson-Erkrankung eingesetzt wird. Die pharmazeutische Wirksamkeit von Rotigotin und Erkrankungen, bei denen Rotigotin bevorzugt eingesetzt werden kann sind beispielsweise in der WO 2002/089777, der WO 2005/092331, der WO 2005/009424, der WO 2003/092677 und der WO 2005/063237 beschrieben.

Aufgrund ihrer kurzen Halbwertzeit und ihres hohen First-Pass-Effekts ist die orale Verabreichung der Verbindung Rotigotin problematisch. Daher schlagen eine Reihe von Druckschriften die transdermale Verabreichung von Rotigotin vor und ein entsprechendes Arzneimittel ist unter der Bezeichnung Neupro^{®} auf dem Markt.

Ein transdermales therapeutisches System für die Verabreichung von Rotigotin wurde bereits früh in der WO 94/07468 beschrieben. In dem dort beschriebenen System wird der Wirkstoff als Hydrochlorid in einer Zweiphasenmatrix eingesetzt, die im Wesentlichen durch ein als durchgehende Phase vorliegendes hydrophobes Polymermaterial mit darin dispergiertem hydratisiertem Silikat zur Aufnahme des hydrophilen Arzneistoffsalzes gebildet wird. Die dort beschriebenen transdermalen therapeutischen Systeme sind jedoch schwierig herzustellen und die Hautpermeation des Wirkstoffes aus diesem System ist problematisch. Die in der WO 94/07468 beschriebenen transdermalen therapeutischen Systeme waren daher nicht marktfähig.

Es gibt eine Reihe von Druckschriften, die verbesserte transdermale therapeutische Systeme beschreiben. So offenbart die WO 99/49852 transdermale therapeutische Systeme mit einer Matrix auf der Basis eines nicht-wässrigen Polymerklebersystems auf Acrylat- bzw. Silikonbasis, die im Wesentlichen frei von anorganischen Silikatpartikeln ist. Die Matrixsysteme stellen in ihrer einfachsten Ausführungsform Einphasenmatrix-Systeme dar. Die Matrix besteht im Wesentlichen aus einem Acrylatkleber oder einem Silikonkleber und im Falle eines Silikonklebers kann die Matrix auch z.B. Polyvinylpyrrolidon, Copolymere von Vinylpyrrolidon und Vinylacetat, Polyethylenglykol, Glycerin, Fettsäureester von Glycerin oder Copolymere aus Ethylen und Vinylacetat enthalten.

Die WO 2004/012730, die WO 2004/012719 und die WO 2004/058247 offenbaren transdermale therapeutische Systeme zur Verabreichung von Rotigotin mit einer selbstklebenden Matrix, die mit dem Wirkstoff gesättigt ist und den Wirkstoff als Vielzahl von Mikroreservoirs bzw. amorphen Partikeln enthält. Bei der Matrix handelt es sich um eine selbstklebende Matrix auf der Basis von Silikonkleber.

Ein transdermales therapeutisches System zur Verabreichung von Rotigotin auf der Basis einer Silikonmatrix wird auch in der WO 02/089778 offenbart. Hier wird als wesentlich angesehen, dass das transdermale therapeutische System eine Fläche von 10-40 cm² aufweist und 0,1-3,15 mg/cm² Rotigotin als Wirkstoff enthält.

Ein wichtiger Aspekt bei der Formulierung von transdermalen therapeutischen Systemen mit dem Wirkstoff Rotigotin ist die Vermeidung des Auskristallisierens von Wirkstoff im transdermalen therapeutischen System. Auskristallisierter Wirkstoff kann eine Vielzahl von Problemen verursachen, einerseits wird die Verabreichungsrate beeinträchtigt, zum anderen können auch die Klebeeigenschaften der Klebematrix nachteilig beeinflusst werden. Unglücklicherweise wird ein solches Auskristallisieren im Stand der Technik für Rotigotin als ein häufig auftretendes Problem darstellt. Bei dem einzig derzeit vermarkteten transdermalen therapeutischen System, dem Produkt "Neupro^{®}" der Firma UCB liegt das Rotigotin eingebettet in eine Polymermatrix vor. Jedoch erwies sich eine erste Formulierung nicht als stabil und führte zu Kristallbildung im Produkt, weshalb eine Rückrufaktion durchgeführt werden musste.

Die WO 2012/072650 schlägt daher vor, den Wirkstoff in eine nicht-klebende Matrix einzubringen und auf der nicht-klebenden Matrix eine Klebstoffschicht vorzusehen, welche vorzugsweise selbstklebend ist und bevorzugt aus einem druckempfindlichen Polymerklebstoff ("pressure sensitive adhesive"), bevorzugt aus einem aminresistenten Silikonkleber, besteht.

Die WO 2012/084969 schlägt transdermale therapeutische Systeme zur Verabreichung von Rotigotin vor, bei denen die Klebstoffmatrix aus Polystyrol, Polyisobutylen und Gemischen davon besteht und neben dem Wirkstoff noch mindestens ein vernetztes Polyvinylpyrrolidon oder ein Copolymer von Vinylpyrrolidon und Vinylacetat enthält.

Die Druckschrift WO 2011/076879 beschreibt, dass Polyvinylpyrrolidon in einem bestimmten Gewichtsverhältnis zu Rotigotin zu verwenden ist, da ein solches Gewichtsverhältnis unerwarteterweise in der Lage sei, die nichtkristalline Form von Rotigotin zu stabilisieren und zu verhindern, dass Rotigotin in einer festen Dispersion, wie einer selbstklebenden Matrix eines transdermalen therapeutischen Systems, rekristallisiert, Die WO 2011/076879 schlägt deshalb vor, Polyvinylpyrrolidon, zur Stabilisierung einer festen Dispersion der nichtkristallinen Form von Rotigotin in einem Dispergiermittel, welches vorzugsweise mindestens einen druckempfindlichen Silikonkleber umfasst, in einem Gewichtsverhältnis von Rotigotin zu Polyvinylpyrrolidon von etwa 9: 3,5 bis etwa 9: 6 zu verwenden.

Schließlich beschreibt die Druckschrift WO 2011/057714 ein Verfahren zur Verhinderung der Kristallisation eines Arzneistoffs in einem Polymerfilm. Diese Polymerfilme der WO 2011/057714 sind unter anderem für die Herstellung transdermaler therapeutischer Systeme geeignet, wobei einer der beiden bevorzugten Arzneistoffe Rotigotin ist. Dabei muss die, bei der Herstellung des Polymerfilms ausgestrichene lösungsmittelhaltige, Beschichtungsmasse, umfassend ein matrixbildendes Polymer oder Polymergemisch und mindestens einen Arzneistoff, bei Temperaturen getrocknet wird, die zeitweise mindestens 10 °C über der Schmelztemperatur des in der Beschichtungsmasse enthaltenen Arzneistoffs liegen.

Ein weiterer wichtiger Aspekt bei der Formulierung von transdermalen therapeutischen Systemen ist die Wahl der druckempfindlichen Haftkleber. Bei der Wahl der druckempfindlichen Haftkleber sind verschiedene Faktoren zu berücksichtigen, insbesondere sollen eine optimale Klebkraft, Trennkraft und Klebrigkeit (Tack), konstante Wirkstofffreisetzung, Lagerstabilität, sowie minimale Kleberückstände und Hautreizungen erreicht werden. Außerdem sollen die druckempfindlichen Haftkleber nicht mit dem Wirkstoff reagieren.

Die "Klebkraft" (oder "Haftfestigkeit") beschreibt die Kraft, die erforderlich ist um ein transdermales therapeutisches System von einer Testoberfläche, auf die das transdermale therapeutische Systeme angebracht wurde, abzuziehen, d.h. die Eigenschaft dem Abziehen von einer Oberfläche zu widerstehen. Die "Trennkraft" beschreibt die Kraft die erforderlich ist um ein TTS von der Schutzfolie (4) abzuziehen. Die "Klebrigkeit" (oder der "Tack") ist die Eigenschaft Bindungen zu festen Oberflächen einzugehen, d.h. die Eigenschaft zur Anhaftung an eine feste Oberfläche bei kurzer Kontaktzeit und sehr leichtem Druck.

Verschiedene druckempfindliche Haftkleber zur Verwendung in transdermalen therapeutischen Systemen mit dem Wirkstoff Rotigotin sind im Stand der Technik vorgeschlagen worden, so insbesondere Silikonkleber, aber auch beispielsweise Polyacrylat, Polyisobutylen und Polystyrol basierte Polymerkleber.

Polyisobutylen und Polystyrol und Gemische davon werden beispielsweise in den transdermalen therapeutischen Systemen zur Verabreichung von Rotigotin der WO 2012/084969 verwendet. Polyisobutylene weisen gegenüber vielen pharmazeutischen Wirkstoffen grundsätzlich schlechte Lösungseigenschaften auf und haben außerdem den Nachteil, dass diese nur bei einer Abmischung mit geringer molekularen Polyisobutylenen ausreichende Klebrigkeit besitzen und dann auch einen höheren s.g. Kalten Fluss (Cold Flow) besitzen können. Styrole indessen benötigen in der Regel hohe Mengen an Weichmachern und Klebkraftvermittler (Tackifiern).

Hingegen weisen druckempfindliche Silikonkleber eine hohe Flexibilität, eine geringe Oberflächenspannung und minimale Eigenschaftsänderungen über einen weiten Temperaturbereich auf. Schließlich besitzen Silikonkleber eine hohe Luft- und Wasserdurchlässigkeit, eine gute Hautverträglichkeit und Beständigkeit gegenüber äußeren Einflüssen (Feuchtigkeit, UV-Strahlen, Stabilität unter sauren und basischen Bedingungen, etc.).

Bei druckempfindlichen Silikonklebern für transdermale therapeutische Systeme lassen sich grundsätzlich zwei Formen unterscheiden, nämlich "nicht aminresistente" Silikonkleber und "aminresistente" Silikonkleber. "Nicht aminresistente" Silikonkleber enthalten noch freie Silanolgruppen. Diese Silanolgruppen interagieren leicht mit den Amingruppen in Wirkstoffen wie Rotigotin, wodurch Wirkstoffabbauprodukte auftreten können. Darüberhinaus kann diese Reaktion die Eigenschaften der Adhesivschicht in transdermalen therapeutischen Systemen erheblich beeinträchtigen, beispielsweise durch Verringerung des Tacks und/oder durch Austrocknung bei Lagerung (siehe bspw. U.S. Patentschriften US RE35,474 und US 4,591,622).

Aus diesem Grund wird im Stand der Technik für silikonkleberbasierte transdermale therapeutische Systeme mit dem Wirkstoff Rotigotin in der Regel nur aminresistenter und kein nicht aminresistenter Silikonkleber verwendet. In "aminresistentem" Silikonkleber sind die Silanolgruppen durch Schutzgruppen, beispielsweise durch Trimethylsilyl-, abgekürzt (TMS)-Gruppen, geschützt.

So werden beispielsweise in dem derzeit einzigen auf dem Markt verfügbaren transdermalen therapeutischen System Neupro^{®} ausschließlich aminresistente Silikonkleber verwendet. Es handelt sich dabei um ein Einschichtlaminat, mit der Matrixschicht als ein Biphasensystem in der Form einer festen Dispersion, wobei eine innere Phase durch den Wirkstoff gelöst in einem Polymer und eine äußere Phase durch aminresistente Silikonkleber als Dispergiermittel gebildet werden.

Gleichermaßen werden in der WO 99/49852 aufgrund der basischen Natur von Rotigotin für einen diesen Wirkstoff enthaltenden Silikonkleber aminresistente Kleber eingesetzt. Wie die WO 99/49852 beschreibt, zeichnen sich solche aminresistenten Silikonkleber dadurch aus, dass sie über keine freien Silanolfunktionen (d.h. Silanolgruppen) verfügen.

Auch gemäß der WO 02/089778 muss das darin offenbarte transdermale therapeutische System auf Silikonbasis mindestens eine aminresistente Silikonverbindung als Hauptbestandteil enthalten. Dabei ist die Silikonverbindung gewöhnlich ein druckempfindlicher Kleber oder eine Mischung davon und bildet eine Matrix, in welcher die anderen Komponenten des transdermalen therapeutischen Systems eingebettet sind.

Gemäß der WO 2004/012730 (und auch gemäß der WO 2004/012719 und der WO 2011/076879) sind besonders bevorzugte druckempfindliche Kleber zur Verwendung in den darin offenbarten transdermalen therapeutischen Systemen von dem Typ, der ein Netzwerk aus löslichem polykondensiertem Polydimethylsiloxan (PDMS)/Harz bildet, worin die Hydroxygruppen z.B. mit Trimethylsilyl (TMS)-Gruppen geschützt sind.

Gemäß der WO 2004/058247 ist in einer bevorzugten Ausführungsform der Erfindung das Matrixpolymer ein Silikon, vorzugsweise ein aminresistentes Silikon oder eine Silikon-Mischung. Gleichermaßen ist gemäß der WO 2004/058247 das Matrixpolymer ein aminresistentes Silikon oder eine Mischung aminresistenter Silikone. Schließlich sind auch gemäß der WO 2011/057714 aminresistente Polysiloxane besonders bevorzugt.

Solche aminresistenten Silikonkleber, wie beispielsweise im vermarkteten Neupro^{®} Produkt verwendet, führen jedoch häufig zu unbefriedigenden Resultaten insbesondere hinsichtlich Klebkraft und Klebrigkeit.

Somit besteht trotz all den bekannten transdermalen therapeutischen Systemen mit dem Wirkstoff Rotigotin ein Bedarf nach einem transdermalen therapeutischen System für die Verabreichung des Wirkstoffs Rotigotin, das eine befriedigende Klebkraft und Klebrigkeit (Tack) und eine hervorragende Lagerstabilität aufweist, bei dem also der Wirkstoff nicht während der Lagerzeit auskristallisiert. Das transdermale therapeutische System soll aber dennoch eine ausreichende Hautpermeation für den Wirkstoff gewährleisten, möglichst einfach herzustellen sein, und eine gleichmäßige Verabreichung des Wirkstoffs über die gewünschte Verabreichungsdauer von mindestens einem Tag erlauben. Ferner sollte aus Kostengründen und im Hinblick auf Vorgaben bestimmter nationaler Arzneimittelbehörden (z.B. zur Missbrauchsvermeidung) der Restgehalt des Wirkstoffs in dem transdermalen therapeutischen System nach dessen Verwendung nicht zu hoch sein.

### Zusammenfassung der Erfindung

Zur Lösung dieser Aufgabe schlägt die Erfindung transdermale therapeutische Systeme vor, wie sie in den Ansprüchen definiert werden.

Die Urheber der vorliegenden Erfindung haben überraschenderweise gefunden, dass in transdermalen therapeutischen Systemen (TTS) mit dem Wirkstoff Rotigotin durch die Zugabe einer kleinen Menge von Paraffin zur Matrixschicht, trotz der Verwendung von Silikonklebern, die eine noch relevante Menge an freien Silanolgruppen aufweisen (d.h. nicht aminresistente Silikonkleber), im Vergleich zu einem TTS, bei dem aminresistente Silikonkleber verwendet werden, verbesserte Eigenschaften, insbesondere eine deutlich erhöhte Klebkraft und Klebrigkeit und eine verbesserte Lagerstabilität, erreicht werden können.

Weiterhin haben die vorliegenden Erfinder überraschenderweise gefunden, dass in einem solchen transdermalen therapeutischen System mit dem Wirkstoff Rotigotin und einem oder mehreren nicht aminresistenten Silikonkleber in einer Menge von mehr als 50 Gew.-% bezogen auf das Gesamtgewicht der druckempfindlichen Haftkleber der Matrixschicht (2), bei Verwendung von Rotigotin und einem Polyvinylpyrrolidon in der Matrixschicht in der dispergierten Phase einer festen Dispersion in einem Gewichtsverhältnis von 9 : 7 oder kleiner keine Auskristallisierung des Wirkstoffs erfolgt. Bei einem größeren Gewichtsverhältnis von Rotigotin und Polyvinylpyrrolidon, beispielsweise von 9 : 5 oder größer wurden dagegen nach längeren Lagerzeiten, bei Temperaturen von 25 °C oder höher, Kristalle gefunden.

Schließlich wurde überraschenderweise gefunden, dass eine Trocknungstemperatur beim Beschichtungsvorgang auf mindestens 10 °C oberhalb des Schmelzpunktes von Rotigotin, um eine spätere Auskristallisierung von Rotigotin zu verhindern, bei den erfindungsgemäßen transdermalen therapeutischen Systemen nicht erforderlich ist.

Damit stellt die Erfindung transdermale therapeutische Systeme, umfassend eine Rückschicht (1), eine Matrixschicht (2), die einen Arzneistoff enthält, und eine vor Gebrauch zu entfernende Schutzfolie (4), zur Verfügung, wobei es sich bei dem Arzneistoff um Rotigotin handelt, und wobei die Matrixschicht (2) einen oder mehrere nicht aminresistente druckempfindliche Silikonkleber in einer Menge von mehr als 50 Gew.-% bezogen auf das Gesamtgewicht der druckempfindlichen Haftkleber der Matrixschicht (2), und Paraffin in einer Menge von mindestens 0,1 Gew.-% bezogen auf das Gesamtgewicht der Matrixschicht (2), enthält, wobei die Rückschicht eine Polyesterfolie mit einer Dick von unter 35 µm umfasst oder aus einer Polyesterfolie mit einer Dicke von unter 70 µm besteht, und wobei das Rotigotin in der Matrixschicht (2) im Wesentlichen in einer nicht-kristallinen Form in der dispergierten Phase einer festen Dispersion umfassend ein Polyvinylpyrrolidon vorliegt.

Die Erfindung betrifft ebenfalls die Verwendung der transdermalen therapeutischen Systeme zur Behandlung von Krankheiten, bei denen eine transdermale Verabreichung von Rotigotin veranlasst ist, insbesondere zur Behandlung der Parkinson-Erkrankung. Schließlich betrifft die Erfindung Verfahren zur Herstellung der erfindungsgemäßen transdermalen therapeutischen Systeme.

### Kurze Beschreibung der Abbildungen

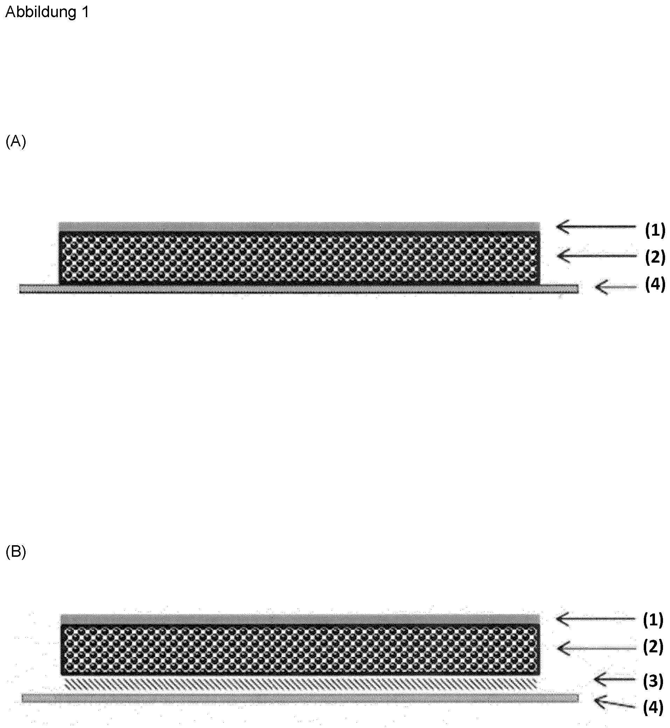

**Abb. 1:** (A) Monolayer-Formulierung des transdermalen therapeutischen Systems mit einer *Rückschicht* (1), einer *Matrixschicht* (2) und einer *Schutzfolie* (4). (B) Bilayer-Formulierung des transdermalen therapeutischen Systems mit einer *Rückschicht* (1), einer *Matrixschicht* (2), mindestens einer *zusätzlichen initial wirkstofffreien Haftkleberschicht* (3) und einer *Schutzfolie* (4).

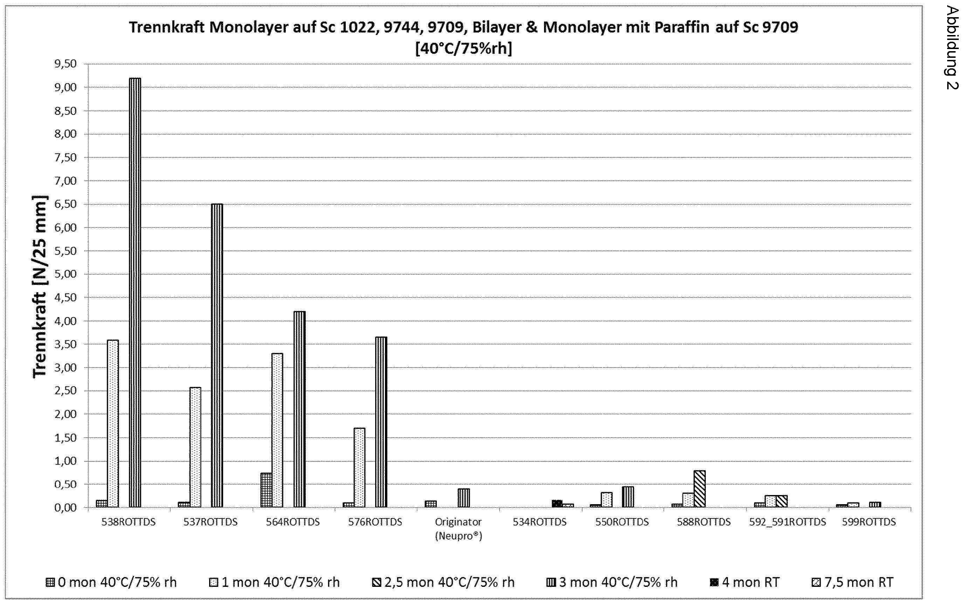

**Abb. 2****:** Trennkraft (Separation Force) verschiedener Monolayer- und Bilayer-Formulierungen bei Lagerung über 0 bis 3 Monate bei 40 °C / 75% rF (oder rh, relative Luftfeuchtigkeit).

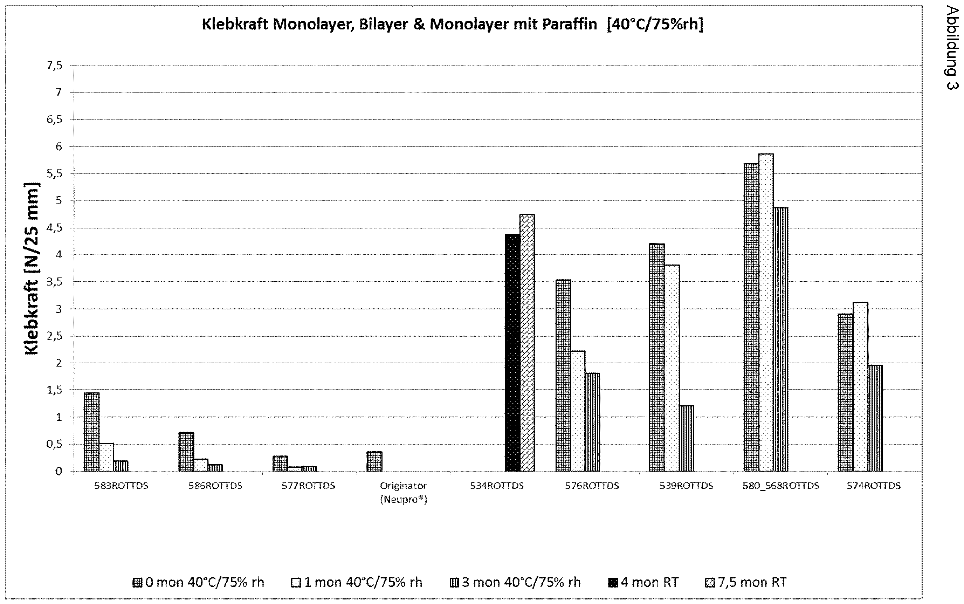

**Abb. 3****:** Klebkraft (Adhesion Strength) verschiedener Monolayer- und Bilayer-Formulierungen bei Lagerung über 0 bis 3 Monate bei 40 °C / 75% rF (oder rh, relative Luftfeuchtigkeit).

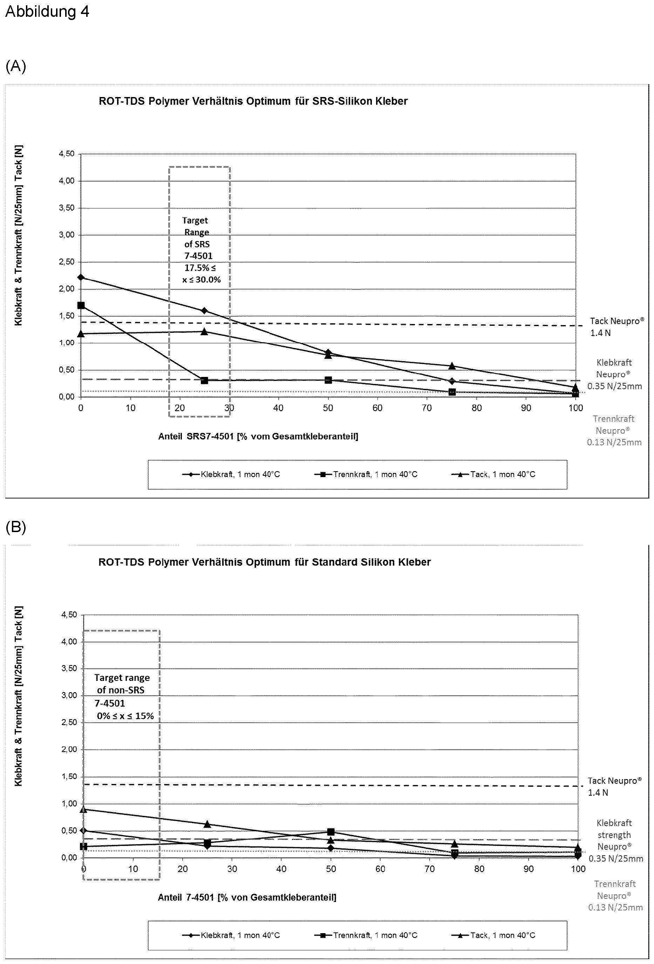

**Abb. 4:** (A) Ermittlung des optimalen Polymerkleberverhältnisses der silanolreduzierten Silikonkleber BIO-PSA SRS7-4501 (mittlere Klebrigkeit) : SRS7-4601 (hohe Klebrigkeit) unter Berücksichtigung von Trennkraft, Klebkraft und Klebrigkeit. (B) Ermittlung des optimalen Polymerkleberverhältnisses der nicht silanolreduzierten Silikonkleber BIO-PSA 7-4501 (mittlere Klebrigkeit) : 7-4601 (hohe Klebrigkeit) unter Berücksichtigung von Trennkraft, Klebkraft und Klebrigkeit.

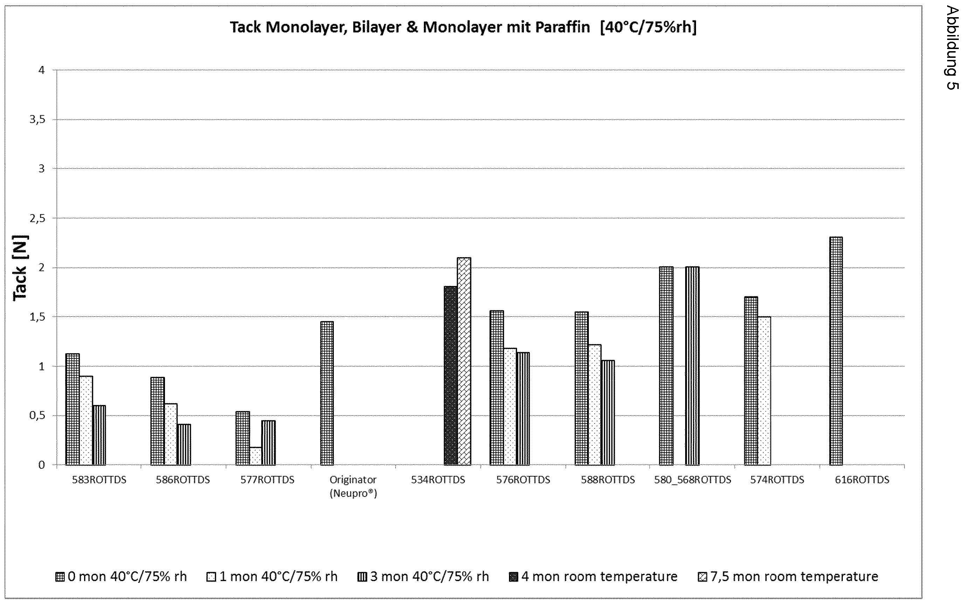

**Abb. 5****:** Klebrigkeit (Tack) verschiedener Monolayer- und Bilayer-Formulierungen bei Lagerung über 0 bis 3 Monate bei 40 °C / 75% rF (oder rh, relative Luftfeuchtigkeit).

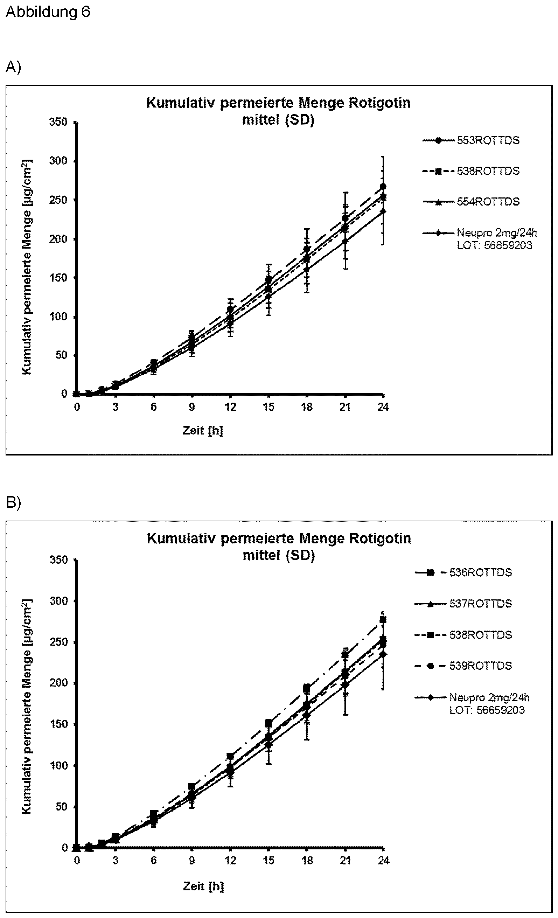

**Abb. 6:** A)) Kumulativ permeierte Menge an Rotigotin über 24 h, bei Monolayer-Formulierungen mit konstantem Gehalt an Rotigotin und variablem Gehalt an PVP K90. B) Kumulativ permeierte Menge an Rotigotin über 24 h, von Monolayer-Formulierungen mit verschiedenen Mischungsverhältnissen von BIO-PSA SRS7-4501 zu BIO-PSA SRS7-4601 (Dow Corning^{®}) bei konstantem Rotigotin und PVP K90 Gehalt.

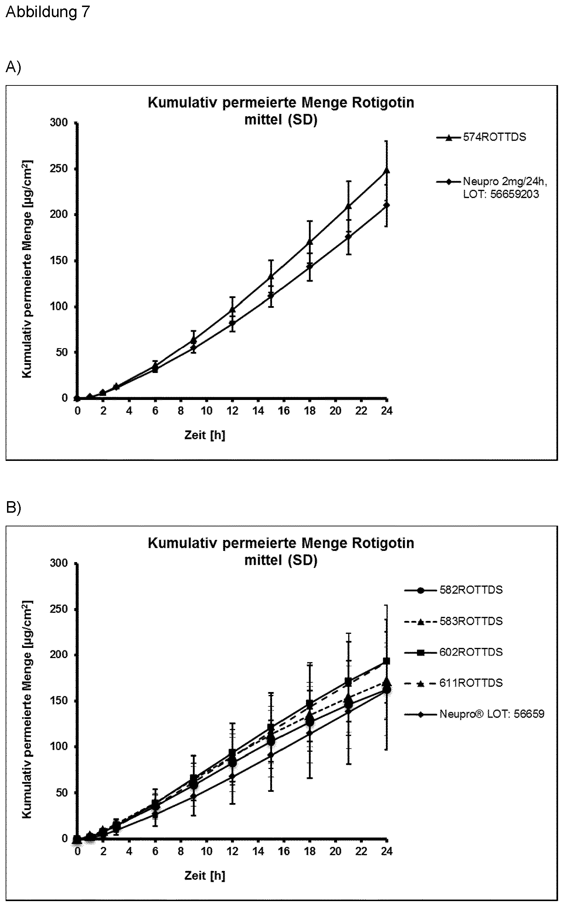

Abb. 7: (A) Kumulativ permeierte Menge an Rotigotin über 24 h, bei einer Monolayer-Formulierung mit silanolreduziertem nicht aminresistentem Silikonkleber und 2 Gew.-% Paraffin. (B) Kumulativ permeierte Menge an Rotigotin über 24 h, bei Monolayer-Formulierungen mit nicht silanolreduzierten nicht aminresistenten Silikonklebern, bei einer Paraffinmenge von 1-2 Gew.-%.

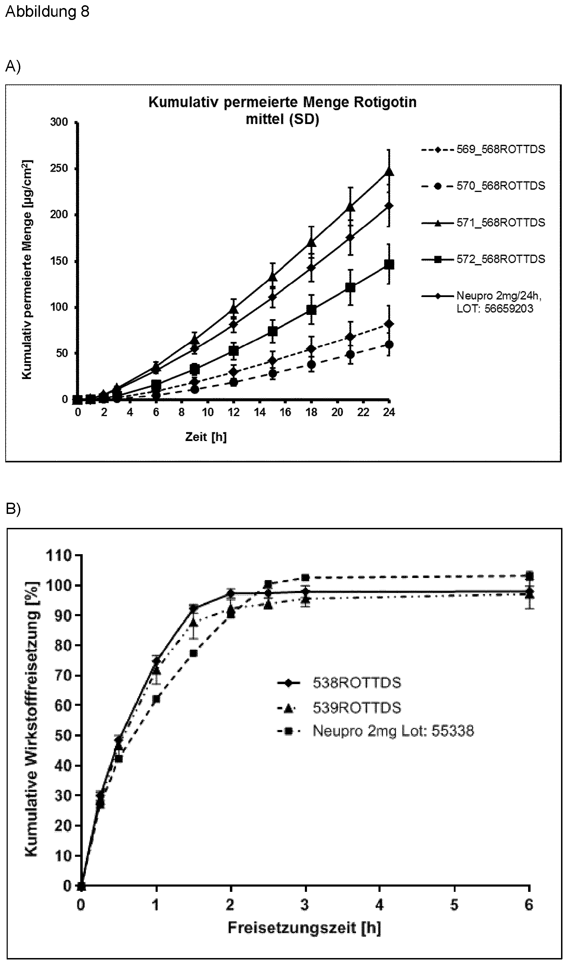

Abb. 8**:** A) Kumulativ permeierte Menge an Rotigotin über 24 h, bei Bilayer-Formulierungen. B) Kumulativ freigesetzte Menge an Rotigotin über 6 h bei Monolayer-Formulierungen.

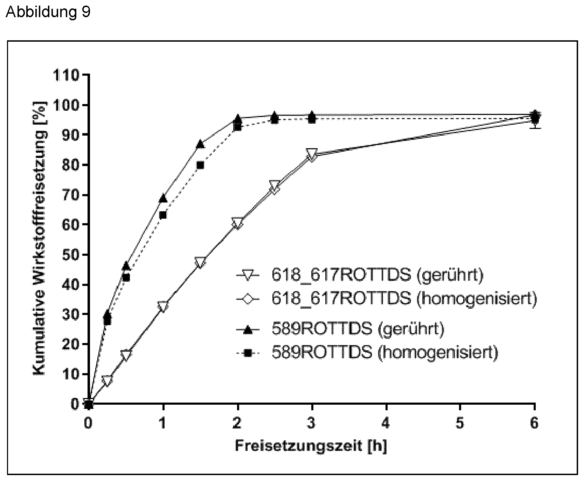

**Abb. 9** Kumulativ freigesetzte Menge an Rotigotin über 6 h, bei Bilayer-Formulierung per *in vitro* Dissolution (*in vitro* Wirkstofffreisetzung), bei der die Masse der wirkstoffhaltigen Matrixschicht (2) vor dem jeweiligen Beschichten zu ca. 50 g/m² jeweils nur gerührt wurde und dieselbe Masse vor dem jeweiligen Beschichten zu 50 g/m² zusätzlich homogenisiert wurde. 618_617ROTTDS: Bilayer Formulierung; 589ROTTDS: Monolayer Formulierung; gerührt: nur gerührt; homogenisiert: gerührt und homogenisiert.

### Detaillierte Beschreibung der Erfindung

In seiner einfachsten Ausgestaltung umfasst das erfindungsgemäße transdermale therapeutische System eine Rückschicht (1), auf die Rückschicht (1) folgt eine Matrixschicht (2), die den Wirkstoff enthält und auf die Matrixschicht (2) folgt eine vor Gebrauch zu entfernende Schutzfolie (4) (vgl. Abb. 1 (A)). Zwischen den einzelnen Schichten des erfindungsgemäßen transdermalen therapeutischen Systems, d.h. zwischen der Rückschicht (1) und der Matrixschicht (2) und/oder zwischen der Matrixschicht (2) und der Schutzfolie (4) können sich jeweils eine oder mehrere weitere Schichten befinden. Beispielsweise befindet sich in einer bevorzugten Ausführungsform der vorliegenden Erfindung zwischen der Matrixschicht (2) und der Schutzfolie (4) mindestens eine zusätzliche initial wirkstofffreie Haftkleberschicht (3).

Bei der Matrixschicht (2) der erfindungsgemäßen transdermalen therapeutischen Systeme handelt es sich um eine Haftkleberschicht die den Wirkstoff Rotigotin enthält und erfindungsgemäß ist wesentlich, dass diese Haftkleberschicht einen oder mehrere nicht aminresistente druckempfindliche Silikonkleber in einer Menge von mehr als 50 Gew.-%, bezogen auf das Gesamtgewicht der druckempfindlichen Haftkleber der Matrixschicht (2), und Paraffin in einer Menge von mindestens 0,1 Gew.-% bezogen auf das Gesamtgewicht der Matrixschicht (2), enthält, und dass das Rotigotin in der Matrixschicht (2) im Wesentlichen in einer nicht-kristallinen Form in der dispergierten Phase einer festen Dispersion umfassend ein Polyvinylpyrrolidon vorliegt.

Die vorliegenden Erfinder haben überraschenderweise gefunden, dass durch die Zugabe einer kleinen Menge von Paraffin zur Matrixschicht (2) trotz gleichzeitiger Verwendung nicht aminresistenter Silikonkleber und des Wirkstoffs Rotigotin in der Matrixschicht (2), im Vergleich zu einem transdermalen therapeutischen System bei dem aminresistente Silikonkleber und Rotigotin verwendet werden, eine deutliche Erhöhung der Klebkraft und der Klebrigkeit und eine verbesserte Lagerbeständigkeit, insbesondere bezüglich der Klebkraft und Klebrigkeit, erreicht werden kann.

Die Begriffe "Gesamtgewicht" und "Gesamtmenge" wie hierin verwendet, beziehen sich jeweils auf das Trockengewicht, d.h. das Gewicht der Bestandteile, auf welche sich der Ausdruck "Gesamtgewicht" oder "Gesamtmenge" im jeweiligen Kontext bezieht, im transdermalen therapeutischen System in seiner gebrauchsfertigen Form, falls nicht anderweitig ersichtlich.

Unter den "druckempfindlichen Haftklebern" eines transdermalen therapeutischen Systems, bzw. den "druckempfindlichen Haftklebern" einer Schicht eines transdermalen therapeutischen Systems, beispielsweise der Matrixschicht (2) und/oder der mindestens einen zusätzlichen initial wirkstofffreien Haftkleberschicht (3), im Sinne der vorliegenden Erfindung, sind all jene Bestandteile zu verstehen, insbesondere Polymerkleber, die aufgrund ihrer inhärenten Eigenschaft als druckempfindliche Haftvermittler/Kleber dem transdermalen therapeutischen System, bzw. der Schicht oder den Schichten des transdermalen therapeutischen Systems beigegeben werden. Derartige druckempfindliche Haftklebersind dem Fachmann bekannt. Der Wirkstoff, Paraffin, ein Kristallisationshemmer wie Polyvinylpyrrolidon, Penetrationsverstärker und weitere Zusatzstoffe wie Weichmacher und Antioxidantien zählen damit nicht zu den druckempfindlichen Haftklebern. Die Begriffe "Silikonkleber" und druckempfindlicher Silikonkleber", wie hierin verwendet sind gegeneinander austauschbar.

Die Matrixschicht (2) des erfindungsgemäßen transdermalen therapeutischen Systems umfasst einen oder mehrere nicht aminresistente Silikonkleber in einer Menge von mehr als 50 Gew.-% bezogen auf das Gesamtgewicht der druckempfindlichen Haftkleber der Matrixschicht (2). Die Matrixschicht (2) kann also einen, zwei, drei, vier etc. nicht aminresistente Silikonkleber umfassen.

Der Fachmann weiß was unter den Begriffen "nicht aminresistenter" Silikonkleber und "aminresistenter" Silikonkleber zu verstehen ist. Aminresistente Silikonkleber bzw. Verfahren zur Herstellung solcher aminresistenten Silikonkleber sind beispielsweise in den U.S. Patentschriften US RE35,474 und US 4,591,622 beschrieben. Ein "nicht aminresistenter" Silikonkleber zeichnet sich bspw. dadurch aus, dass es sich um einen druckempfindlichen Silikonkleber handelt der, im Gegensatz zu einem "aminresistenten" Silikonkleber, eine relevante Menge an freien Silanolgruppen aufweist (nicht durch Schutzgruppen geschützte OH-Gruppen), so dass unter gewöhnlichen Umständen die Gefahr einer Interaktion mit aminhaltigen Wirkstoffen besteht. Ein unvollständiges Schützen, s.g. capping oder (end-) blocking bzw. nur teilweise Entfernung dieser freien Silanolgruppen führt zu den sogenannten silanolreduzierten Silikonklebern. Silanolreduzierte nicht aminresistente Silikonkleber bzw. Verfahren zur Herstellung solcher silanolreduzierter nicht aminresistenter Silikonkleber sind dem Fachmann bekannt und beispielsweise in der U.S. Patentschrift US 6,337,086 beschrieben. Diese gehören immer noch zur Gruppe der nicht aminresistenten Silikonkleber.

Vorzugsweise ist unter dem Begriff "nicht aminresistenter" Silikonkleber im Sinne der vorliegenden Erfindung ein druckempfindlicher Silikonkleber zu verstehen, der ein Gehalt an freien Silanolgruppen (Gehalt an freien OH-Gruppen bzw. siliziumgebundener Hydroxylgehalt) von beispielsweise mindestens 7700 ppm oder mehr, bevorzugt mindestens um 8000 ppm oder mehr, und bevorzugt nicht mehr als 13000 ppm, aufweist.

Der Gehalt (bzw. die Konzentration) an freien Silanolgruppen in Silikonklebern kann mittels dem Fachmann geläufiger Methoden, wie beispielsweise mit Hilfe der Kernspinresonanzspektroskopie (NMR Spektroskopie) und/oder der Fourier-Transformations-Infrarotspektroskopie (FTIR-Spektroskopie), gemessen werden (siehe bspw. US 6,337,086). Der Silanolgehalt kann dabei mittels ²⁹Si-Kernspinresonanzspektroskopie (²⁹Si-NMR Spektroskopie) und Korrelation der Daten gegen normierte Referenzproben bestimmt werden, während die FTIR-Spektroskopie direkt ein Verhältnis von freien zu geschützten Silanolfunktionen liefert.

Der Silanolgehalt kann so, beispielsweise wie in US 6,337,086 beschrieben, mittels FTIR-Spektroskopie über das Peakflächenverhältnis A1/(A2 * 100) berechnet werden, wobei die A1-Fläche dem Peak für die dimere Dehnungsschwingung der O-H-Bindung aus den Silanolgruppen und die A2-Fläche der Fläche für einen Obertonpeak der Verformung des Wasserstoffs der Polydimethylsiloxan (PDMS)-Methylgruppe entspricht. In dieser Ausführungsform zeichnet sich ein nicht aminresistenter Silikonkleber dadurch aus, dass sein Verhältnis von ungeschützten zu geschützten Silanolfunktionen beispielsweise größer als 0,45, bevorzugt mindestens 0,46, mehr bevorzugt mindestens 0,5 oder mehr ist.

In einer bevorzugten Ausführungsform der vorliegenden Erfindung zeichnet sich ein nicht aminresistenter Silikonkleber dadurch aus, dass nach dessen mindestens zweistündiger, bevorzugt zwei- bis vierstündiger Umsetzung mit dem Wirkstoff Rotigotin bei ca. 50 °C, in einem geeigneten Lösemittel und in einem Mischungsverhältnis von Silikonkleber zu Rotigotin von bspw. 20:1 bis 4:1, bevorzugt bspw. 10:1, ein nicht unbeachtlicher Teil des Rotigotins, beispielsweise mindestens 0,5 Gew.-%, bevorzugt mindestens 1,0 Gew.-%,, bevorzugter mindestens 2,5 Gew.-% mit dem Silikonkleber reagiert und dadurch abgebaut bzw. umgewandelt wird. Geeignete Lösemittel sind dem Fachmann bekannt und hängen insbesondere vom Silikonkleber ab; Beispiele sind Heptan, Ethanol und Essigsäureethylester. Der Anteil an abgebautem bzw. umgewandeltem Rotigotin kann auf für den Fachmann bekannte Art und Weise bestimmt werden.

Hinsichtlich der Wahl des einen oder der mehreren nicht aminresistenten Silikonkleber ist die Erfindung ansonsten nicht besonders eingeschränkt. Nicht aminresistente Silikonkleber sind aus dem Stand der Technik bekannt.

In einer Ausführungsform der Erfindung ist der eine oder die mehreren nicht aminresistenten druckempfindlichen Silikonkleber auszuwählen aus der Gruppe umfassend nicht aminresistente druckempfindliche Silikonkleber mit mittlerer Klebrigkeit, wie beispielsweise Dow Corning^{®} BIO-PSA 7-4501, Dow Corning^{®} BIO-PSA 7-4502, Dow Corning^{®} BIO-PSA SRS7-4501, Dow Corning^{®} BIO-PSA SRS7-4502, nicht aminresistente druckempfindliche Silikonkleber mit hoher Klebrigkeit, wie beispielsweise Dow Corning^{®} BIO-PSA 7-4601, Dow Corning^{®} BIO-PSA 7-4602, Dow Corning^{®} BIO-PSA SRS7-4601, Dow Corning^{®} BIO-PSA SRS7-4602 und Kombinationen davon. Chemisch werden diese Kleber auch als dimethiconol trimethyl siloxysilicate crosspolymer bezeichnet. In einer weiteren Ausführungsform der Erfindung ist der eine oder die mehreren nicht aminresistenten druckempfindlichen Silikonkleber auszuwählen aus der Gruppe umfassend nicht silanolreduzierte nicht aminresistente druckempfindliche Silikonkleber mit mittlerer Klebrigkeit, nicht silanolreduzierte nicht aminresistente druckempfindliche Silikonkleber mit hoher Klebrigkeit, silanolreduzierte nicht aminresistente druckempfindliche Silikonkleber mit mittlerer Klebrigkeit, silanolreduzierte nicht aminresistente druckempfindliche Silikonkleber mit hoher Klebrigkeit und Kombinationen davon. Beispiele für bevorzugte nicht aminresistente druckempfindliche Silikonkleber im Sinne der vorliegenden Erfindung sind Dow Corning^{®} BIO-PSA 7-4501, Dow Corning^{®} BIO-PSA 7-4601, Dow Corning^{®} BIO-PSA SRS7-4501, Dow Corning^{®} BIO-PSA SRS7-4601, und Kombinationen davon.

Die Matrixschicht (2) der erfindungsgemäßen transdermalen therapeutischen Systeme, enthält einen oder mehrere nicht aminresistente druckempfindliche Silikonkleber in einer Menge von mehr als 50 Gew.-% bezogen auf das Gesamtgewicht der druckempfindlichen Haftkleber der Matrixschicht (2). D.h. die Matrixschicht (2) enthält einen Gesamtgewichtsanteil an nicht aminresistentem druckempfindlichem Silikonkleber von mehr als 50 Gew.-%, bezogen auf das Gesamtgewicht der druckempfindlichen Haftkleber der Matrixschicht (2).

In einer bevorzugten Ausführungsform der vorliegenden Erfindung enthält die Matrixschicht (2) des transdermalen therapeutischen Systems einen Gewichtsanteil an nicht aminresistentem druckempfindlichem Silikonkleber von mehr als 60 Gew.-%, bevorzugt mehr als 70 Gew.-%, bevorzugter mehr als 75 Gew.-%, noch bevorzugter mehr als 80 Gew.-%, noch bevorzugter mehr als 85 Gew.-% noch bevorzugter mehr als 90 Gew.-%, weiterhin bevorzugt mehr als 93 Gew.-%, weiterhin bevorzug mehr als 95 Gew.-%, weiterhin bevorzugt mindestens 99 Gew.-%, bezogen auf das Gesamtgewicht der druckempfindlichen Haftkleber der Matrixschicht (2). In einer weiteren bevorzugten Ausführungsform der vorliegenden Erfindung enthält die Matrixschicht (2) des transdermalen therapeutischen Systems als druckempfindliche Haftkleber ausschließlich nicht aminresistente druckempfindliche Silikonkleber; d.h. insbesondere, dass die Matrixschicht (2) keine anderen Polymerkleber sondern ausschließlich nicht aminresistente druckempfindliche Silikonkleber enthält.

Wenn in der Haftkleberschicht beziehungsweise in den Haftkleberschichten des TTS auf die Bezug genommen wird (bspw. die Matrixschicht (2) oder eine zusätzliche initial wirkstofffreie Haftkleberschicht (3)) genau ein nicht aminresistenter Silikonkleber enthalten ist, macht der Gewichtsanteil an diesem nicht aminresistenten Silikonkleber, bezogen auf das Gesamtgewicht der druckempfindlichen Haftkleber der Haftkleberschicht oder Haftkleberschichten des TTS auf die Bezug genommen wird, mehr als 50 Gew.-% aus; wenn in der Haftkleberschicht beziehungsweise in den Haftkleberschichten des TTS auf die Bezug genommen wird genau zwei nicht aminresistente Silikonkleber enthalten sind, macht der Gesamtgewichtsanteil an diesen zwei nicht aminresistenten Silikonklebern, bezogen auf das Gesamtgewicht der druckempfindlichen Haftkleber der Haftkleberschicht oder Haftkleberschichten des TTS auf die Bezug genommen wird, mehr als 50 Gew.-% aus; etc. Dabei muss bei mehreren Haftkleberschichten, in jeder dieser Schichten ein Gewichtsanteil von mehr als 50 Gew.-% jeweils bezogen auf das Gesamtgewicht der druckempfindlichen Haftkleberder jeweiligen Schicht vorliegen.

Die Matrixschicht (2) des erfindungsgemäßen transdermalen therapeutischen Systems enthält Paraffin, das auch als Weißöl bezeichnet wird, in einer Menge von mindestens 0,1 Gew.-%, bezogen auf das Gesamtgewicht der Matrixschicht (2). Es sind insbesondere zwei Paraffine bekannt, nämlich einerseits dickflüssiges Paraffin, dass im Ph. Eur. als Paraffin, liquid bzw. Paraffinum liquidum, in der USP als Mineral Oil, der JP als Liquid Paraffin und in gängiger Literatur auch als Paraffinum subliquidum bezeichnet wird und eine ölige Flüssigkeit mit einer relativen Dichte laut Ph. Eur. im Bereich von 0,827 bis 0,890 (Methode 2.2.5), laut USP im Bereich von 0,845 bis 0,905 (Methode <841>), und laut JP im Bereich von 0,860 bis 0,890 und einer Viskosität laut Ph. Eur. im Bereich von 110-230 mPas (Methode 2.2.9), laut USP im Bereich von 34,5 bis 150,0 mm²*s⁻¹ (Methode <911> capillary viscometer at 40 ± 0.1°) und laut JP im Bereich von nicht weniger als (nlt) 37 mm²/s (Methode 1, 37,8°C) darstellt.

Andererseits bekannt ist dünnflüssiges Paraffin, dass im Ph. Eur. als Paraffin, light liquid bzw. Paraffinum perliquidum, in der USP als Light Mineral Oil und der JP als Light liquid Paraffin bezeichnet wird und eine ölige Flüssigkeit mit einer Dichte laut Ph. Eur. im Bereich von 0,810 bis 0,875 (Methode 2.2.5), laut USP im Bereich von 0,818 bis 0,880 (Methode <841 >) und laut JP im Bereich von 0,830 bis 0,870 und einer Viskosität laut Ph. Eur. im Bereich von 25-80 mPas (Methode 2.2.9), laut USP im Bereich von 3,0 bis 34,4 mm^{2*}s⁻¹ (Methode <911> capillary viscometer at at 40 ± 0.1°) und laut JP im Bereich von weniger als (It) 37 mm²/s (Methode 1, 37,8°C) darstellt.

Bevorzugt ist dickflüssiges Paraffin. In einer weiteren Ausführungsform handelt es sich bei dem Paraffin um dünnflüssiges Paraffin.

Die Menge an Paraffin in der Matrixschicht (2) der erfindungsgemäßen transdermalen therapeutischen Systeme kann z.B. bis zu 50 Gew.-% bezogen auf das Gesamtgewicht der Matrixschicht (2) der erfindungsgemäßen transdermalen therapeutischen Systeme betragen, bevorzugt bis zu 40 Gew.-%, bevorzugter bis zu 30 Gew.-%, noch bevorzugter bis zu 20 Gew.-%, noch bevorzugter bis zu 15 Gew.-%, noch bevorzugter bis zu 10 Gew.-%.

In einer bevorzugten Ausführungsform der vorliegenden Erfindung liegt das Paraffin in der Matrixschicht (2) in einer Menge von mindestens 0,2 Gew.-%, bevorzugt mindestens 0,3 Gew.-%, bevorzugter mindestens 0,5 Gew.-%,noch bevorzugter mindestens 0,8 Gew.-%, noch bevorzugter mindestens 1,0 Gew.-%. bezogen auf das Gesamtgewicht der Matrixschicht (2) vor. In einer weiteren bevorzugten Ausführungsform der Erfindung liegt das Paraffin in der Matrixschicht (2) in einer Menge von mindestens von 0,1 - 30,0 Gew.-%, bevorzugt 0,1 - 20,0 Gew.-%, bevorzugter 0,2 - 20,0 Gew.-%, noch bevorzugter 0,5 - 10,0 Gew.-%, noch bevorzugter 0,8 - 5,0 Gew.-%, noch bevorzugter 1,0 - 5,0 Gew.-%, noch bevorzugter 1,0 - 3,0 Gew.-%, bezogen auf das Gesamtgewicht der Matrixschicht (2), vor.

Da die Matrixschicht bei den erfindungsgemäßen transdermalen therapeutischen Systemen haftklebend ist, ist es in der Regel nicht erforderlich, dass eine zusätzliche haftklebende Schicht auf der Matrixschicht (2) vorhanden ist, wie sie beispielsweise in der WO 2012/072650 verlangt wird. Es ist aber erfindungsgemäß auch nicht ausgeschlossen, dass beispielsweise zur Verbesserung der Klebkraft und Klebrigkeit mindestens eine derartige zusätzliche initial wirkstofffreie Haftkleberschicht (3) vorgesehen ist. In einer bevorzugten Ausführungsform der Erfindung enthält das erfindungsgemäße transdermale therapeutische System keine zusätzliche initial wirkstofffreie Haftkleberschicht (3) zwischen der Matrixschicht (2) und der vor Gebrauch zu entfernenden Schutzfolie (4). Nach dieser Ausführungsform ist die Matrixschicht (2) ausreichend klebrig um eine vorteilhafte Haftung auf der Haut für den gewünschten Anwendungszeitraum zu gewährleisten.

In einer weiteren bevorzugten Ausführungsform der Erfindung enthält das erfindungsgemäße transdermale therapeutische System mindestens eine zusätzliche initial wirkstofffreie Haftkleberschicht (3) zwischen der Matrixschicht (2) und der vor Gebrauch zu entfernenden Schutzfolie (4) (vgl. Abb. 1 B). Die mindestens eine zusätzliche initial wirkstofffreie Haftkleberschicht (3) kann insbesondere bei einer Matrixschicht (2) mit mehreren Phasen zu einer höheren Robustheit gegenüber unterschiedlichen Sphärengrößen-Verteilungen der wirkstoffhaltigen inneren Phase bei der in-vitro Dissolution führen, da die Diffusionstrecke von der wirkstoffhaltigen Matrixschicht (2) in Richtung Haut durch die mindestens eine initial wirkstofffreie Haftkleberschicht (3) auf eine feste Schichtdicke gebracht wird.

In einer weiteren bevorzugten Ausführungsform enthält das erfindungsgemäße transdermale therapeutische System deshalb zwischen der Matrixschicht (2) und der vor Gebrauch zu entfernenden Schutzfolie (4) mindestens eine zusätzliche initial wirkstofffreie Haftkleberschicht (3), wobei die mindestens eine zusätzliche initial wirkstofffreie Haftkleberschicht (3) einen oder mehrere nicht aminresistente druckempfindliche Silikonkleber in einer Menge von mehr als 50 Gew.-% bezogen auf das Gesamtgewicht der druckempfindlichen Haftkleber der mindestens einen zusätzlichen initial wirkstofffreien Haftkleberschicht (3), und Paraffin in einer Menge von mindestens 0,1 Gew.-%, bevorzugt 0,2 - 20,0 Gew.-%, bevorzugter 1,0 - 5,0 Gew.-%, bezogen auf das Gesamtgewicht der mindestens einen zusätzlichen initial wirkstofffreien Haftkleberschicht (3), enthält. Die mindestens eine zusätzliche initial wirkstofffreie Haftkleberschicht (3) kann also einen, zwei, drei, vier etc. nicht aminresistente Silikonkleber umfassen.

In einer weiteren bevorzugten Ausführungsform der vorliegenden Erfindung weist die mindestens eine zusätzliche initial wirkstofffreie Haftkleberschicht (3) des transdermalen therapeutischen Systems einen Gewichtsanteil an nicht aminresistentem druckempfindlichem Silikonkleber von mehr als 60 Gew.-%, bevorzugt mehr als 70 Gew.-%, bevorzugter mehr als 75 Gew.-%, noch bevorzugter mehr als 80 Gew.-%, noch bevorzugter mehr als 85 Gew.-% noch bevorzugter mehr als 90 Gew.-%, weiterhin bevorzugt mehr als 93 Gew.-%, weiterhin bevorzugt mehr als 95 Gew.-%, weiterhin bevorzugt mindestens 99 Gew.-%, bezogen auf das Gesamtgewicht der druckempfindlichen Haftkleber der mindestens einen zusätzlichen initial wirkstofffreien Haftkleberschicht (3), auf. In einer weiteren Ausführungsform der vorliegenden Erfindung weist die mindestens eine zusätzliche initial wirkstofffreie Haftkleberschicht (3) des transdermalen therapeutischen Systems als druckempfindliche Haftkleber ausschließlich nicht aminresistente druckempfindliche Silikonkleber auf; d.h. insbesondere dass die mindestens eine zusätzliche initial wirkstofffreie Haftkleberschicht (3) keine anderen Polymerkleber sondern ausschließlich nicht aminresistente druckempfindliche Silikonkleber enthält.

In einer weiteren bevorzugten Ausführungsform weist das erfindungsgemäße transdermale therapeutische System einen Gewichtsanteil an nicht aminresistentem druckempfindlichem Silikonkleber von mehr als 50 Gew.-%, bevorzugt mehr als 60 Gew.-%, bevorzugter mehr als 70 Gew.-%, noch bevorzugter mehr als 75 Gew.-%, noch bevorzugter mehr als 80 Gew.-%, noch bevorzugter mehr als 85 Gew.-% noch bevorzugter mehr als 90 Gew.-%, weiterhin bevorzugt mehr als 93 Gew.-%, weiterhin bevorzugt mehr als 95 Gew.-%, weiterhin bevorzugt mindestens 99 Gew.-%, bezogen auf das Gesamtgewicht der druckempfindlichen Haftkleber des transdermalen therapeutischen Systems, auf (wobei das transdermale therapeutische System einen einzelnen nicht aminresistenten druckempfindlichem Silikonkleber, oder auch eine Mischung aus zwei, drei, vier etc. nicht aminresistenten Silikonkleber, aufweisen kann). In einer weiteren Ausführungsform weist das transdermale therapeutische System als druckempfindliche Haftkleber ausschließlich nicht aminresistente druckempfindliche Silikonkleber auf; d.h. insbesondere, dass das transdermale therapeutische System keine anderen Polymerkleber sondern ausschließlich nicht aminresistente druckempfindliche Silikonkleber enthält.

In einer weiteren bevorzugten Ausführungsform weist das transdermale therapeutische System mindestens eine zusätzliche initial wirkstofffreie Haftkleberschicht (3) auf, wobei die mindestens eine zusätzliche initial wirkstofffreie Haftkleberschicht (3), oder die Matrixschicht (2) und die mindestens eine zusätzliche initial wirkstofffreie Haftkleberschicht (3), einen Gewichtsanteil an nicht aminresistentem druckempfindlichem Silikonkleber von mehr als 60 Gew.-%, bevorzugter mehr als 70 Gew.-%, noch bevorzugter mehr als 75 Gew.-%, noch bevorzugter mehr als 80 Gew.-%, noch bevorzugter mehr als 85 Gew.-% noch bevorzugter mehr als 90 Gew.-%, weiterhin bevorzugt mehr als 93 Gew.-%, weiterhin bevorzugt mehr als 95 Gew.-%, weiterhin bevorzugt mindestens 99 Gew.-%, bezogen auf das Gesamtgewicht der druckempfindlichen Haftkleber der mindestens einen zusätzlichen initial wirkstofffreien Haftkleberschicht (3), beziehungsweise bezogen auf das Gesamtgewicht der druckempfindlichen Haftkleber der Matrixschicht (2) und der mindestens einen zusätzlichen initial wirkstofffreien Haftkleberschicht (3), aufweist.

Das Flächengewicht der Matrixschicht (2) des erfindungsgemäßen transdermalen therapeutischen Systems ist nicht besonders beschränkt. In einer allgemeinen Ausführungsform der Erfindung weist die Matrixschicht (2) ein Flächengewicht von 30-70 g/m², bevorzugt 30-60 g/m², auf. Der Begriff "Flächengewicht" in Bezug auf eine Schicht des erfindungsgemäßen transdermalen therapeutischen Systems, wie bspw. der Matrixschicht (2) oder der mindestens einen zusätzlichen initial wirkstofffreien Haftkleberschicht (3), bezieht sich auf das Flächengewicht der trockenen Schicht, d.h. das Flächengewicht der Schicht nachdem bei der Herstellung des TTS das Lösemittel durch Trocknen entfernt wurde.

In einer bevorzugten Ausführungsform, in der das transdermale therapeutische System keine andere Haftkleberschicht außer der Matrixschicht (2) enthält, weist die Matrixschicht (2) ein Flächengewicht von 40-70 g/m², bevorzugt 45-65 g/m², bevorzugter um 50-60 g/m², noch bevorzugter 50-60 g/m², auf. In einer weiteren bevorzugten Ausführungsform, in der das transdermale therapeutische System keine andere Haftkleberschicht außer der Matrixschicht (2) enthält, weist die Matrixschicht (2) ein Flächengewicht von um 50 g/m², oder von um 60 g/m² auf.

Der Begriff "um", wie er hier vor Zahlenwerten und Zahlenbereichen verwendet wird, bedeutet, dass ein damit bezeichneten Wert oder Bereich, alle Werte, welche innerhalb von ± 10% des angegebenen Werts oder Bereichs, oder innerhalb von ± 5% des Werts oder Bereichs, oder in einigen Ausführungsformen innerhalb von ± 1% des Werts oder Bereichs, liegen, miteinschließt.

In einer Ausführungsform der Erfindung, in der das transdermale therapeutische System, zusätzlich zu der Matrixschicht (2), mindestens eine zusätzliche initial wirkstofffreie Haftkleberschicht (3) enthält, weist die Matrixschicht (2) ein Flächengewicht von 30-70 g/m², bevorzugter 40-70 g/m², noch bevorzugter 45-65 g/m², noch bevorzugter um 50-60 g/m², noch bevorzugter 50-60 g/m², bspw. um 50 g/m², oder um 60 g/m²; und die mindestens eine zusätzliche initial wirkstofffreie Haftkleberschicht (3) ein Flächengewicht von 15-40 g/m², bevorzugt 20-40 g/m², bevorzugter 20-35 g/m², noch bevorzugter 25-35 g/m², weiterhin bevorzugt um 30 g/m², auf.

In den erfindungsgemäßen transdermalen therapeutischen Systemen enthält die Matrixschicht (2) und, falls mindestens eine zusätzliche initial wirkstofffreie Haftkleberschicht (3) vorhanden ist, die mindestens eine zusätzliche initial wirkstofffreie Haftkleberschicht (3), einen oder mehrere nicht aminresistente druckempfindliche Silikonkleber in einer Menge von mehr als 50 Gew.-% bezogen auf das Gesamtgewicht der druckempfindlichen Haftkleber der Matrixschicht (2), und, falls mindestens eine zusätzliche initial wirkstofffreie Haftkleberschicht (3) vorhanden ist, bezogen auf das Gesamtgewicht der druckempfindlichen Haftkleber der Matrixschicht (2) beziehungsweise bezogen auf das Gesamtgewicht der mindestens einen zusätzlichen initial wirkstofffreien Haftkleberschicht (3).

In einer bevorzugten Ausführungsform enthält die Matrixschicht (1) und/oder die mindestens eine zusätzliche initial wirkstofffreie Haftkleberschicht (3), falls eine zusätzliche initial wirkstofffreie Haftkleberschicht (3) vorhanden ist, genau einen nicht aminresistenten druckempfindlichen Silikonkleber. D.h. in dieser Ausführungsform ist in der Matrixschicht (2), in der mindestens einen zusätzlichen initial wirkstofffreien Haftkleberschicht (3), oder in der Matrixschicht (2) als auch in der mindestens einen zusätzlichen initial wirkstofffreien Haftkleberschicht (3) genau ein nicht aminresistenter druckempfindlicher Silikonkleber enthalten. In einer weiteren bevorzugten Ausführungsform bestehen die druckempfindlichen Haftkleber der Matrixschicht (2) und/oder der mindestens einen zusätzlichen initial wirkstofffreien Haftkleberschicht (3), falls vorhanden, ausschließlich aus genau einem nicht aminresistenten druckempfindlichen Silikonkleber.

In einer weiteren bevorzugten Ausführungsform umfasst der eine oder die mehreren nicht aminresistenten druckempfindlichen Silikonkleber der Matrixschicht (2) und/oder der mindestens einen zusätzlichen initial wirkstofffreien Haftkleberschicht (3), falls mindestens eine zusätzliche initial wirkstofffreie Haftkleberschicht (3) vorhanden ist, zwei oder mehr, oder drei oder mehr, oder vier oder mehr, etc., unterschiedliche nicht aminresistente druckempfindliche Silikonkleber, wobei, vorzugsweise, zumindest einer (d.h. einer oder mehrere) der nicht aminresistenten druckempfindlichen Silikonkleber eine mittlere Klebrigkeit und zumindest einer (d.h. einer oder mehrere) der nicht aminresistenten druckempfindlichen Silikonkleber eine hohe Klebrigkeit aufweist. In einer weiteren bevorzugten Ausführungsform enthält die Matrixschicht (2), und/oder die mindestens eine zusätzliche initial wirkstofffreie Haftkleberschicht (3), falls mindestens eine zusätzliche initial wirkstofffreie Haftkleberschicht (3) vorhanden ist, genau zwei, oder genau drei, oder genau vier, etc., unterschiedliche nicht aminresistente druckempfindliche Silikonkleber. In noch einer weiteren bevorzugten Ausführungsform enthält die Matrixschicht (2) genau einen, und die mindestens eine zusätzliche initial wirkstofffreie Haftkleberschicht (3), falls vorhanden, genau zwei nicht aminresistente druckempfindliche Silikonkleber.

In noch einer weiteren bevorzugten Ausführungsform, umfasst der eine oder die mehreren nicht aminresistenten druckempfindlichen Silikonkleber der Matrixschicht (2) und/oder der mindestens einen zusätzlichen initial wirkstofffreien Haftkleberschicht (3), falls mindestens eine zusätzliche initial wirkstofffreie Haftkleberschicht (3) vorhanden ist, mindestens oder genau zwei, mindestens oder genau drei, mindestens oder genau vier, etc., nicht aminresistente druckempfindliche Silikonkleber, mit unterschiedlichem Molekulargewicht.

In einer bevorzugten Ausführungsform des transdermalen therapeutischen Systems der Erfindung, umfasst der eine oder die mehreren nicht aminresistenten Silikonkleber der Matrixschicht (2), und/oder der mindestens einen zusätzlichen initial wirkstofffreien Haftkleberschicht (3), falls vorhanden, eine Mischung aus zumindest einem nicht aminresistenten druckempfindlichen Silikonkleber mit mittlerer Klebrigkeit, wie beispielsweise Dow Corning^{®} BIO-PSA 7-4501, und zumindest einem nicht aminresistenten druckempfindlichen Silikonkleber mit hoher Klebrigkeit, wie beispielsweise Dow Corning^{®} BIO-PSA 7-4601. In einer weiteren bevorzugten Ausführungsform des transdermalen therapeutischen Systems der Erfindung, besteht der eine oder die mehreren nicht aminresistenten Silikonkleber der Matrixschicht (2), und/oder der mindestens einen zusätzlichen initial wirkstofffreien Haftkleberschicht (3), falls vorhanden, aus einer Mischung aus einem nicht aminresistenten druckempfindlichen Silikonkleber mit mittlerer Klebrigkeit, wie beispielsweise Dow Corning^{®} BIO-PSA 7-4501, und einem nicht aminresistenten druckempfindlichen Silikonkleber mit hoher Klebrigkeit, wie beispielsweise Dow Corning^{®} BIO-PSA 7-4601.

In einer bevorzugten Abwandlung der obigen Ausführungsformen der Erfindung, besteht der eine oder die mehreren nicht aminresistenten Silikonkleber der Matrixschicht (2), und/oder der mindestens einen zusätzlichen initial wirkstofffreien Haftkleberschicht (3), falls vorhanden, aus einer Mischung aus einem nicht aminresistenten druckempfindlichen Silikonkleber mit mittlerer Klebrigkeit, wie beispielsweise Dow Corning^{®} BIO-PSA 7-4501, und einem nicht aminresistenten druckempfindlichen Silikonkleber mit hoher Klebrigkeit, wie beispielsweise Dow Corning^{®} BIO-PSA 7-4601, wobei die Matrixschicht (2), und/oder die mindestens eine zusätzliche initial wirkstofffreie Haftkleberschicht (3), falls vorhanden, keine anderen druckempfindlichen Haftkleber aufweisen.

In einer bevorzugten Ausführungsform der vorliegenden Erfindung, in welcher der eine oder die mehreren nicht aminresistenten Silikonkleber der Matrixschicht (2) und/oder der mindestens einen zusätzlichen initial wirkstofffreien Haftkleberschicht (3), falls vorhanden, eine Mischung aus zumindest einem nicht aminresistenten druckempfindlichen Silikonkleber mit mittlerer Klebrigkeit, und zumindest einem nicht aminresistenten druckempfindlichen Silikonkleber mit hoher Klebrigkeit, enthält, oder aus einer solchen Mischung besteht, liegt der nicht aminresistente Silikonkleber mit mittlerer Klebrigkeit in einem Gewichtsbereich von bevorzugt 0,0-55,0 Gew.-%, bevorzugter 0,0-45,0 Gew.-%, noch bevorzugter um 0,0-35,0 Gew.-%, noch bevorzugter 0,0-25,0 Gew.-%, und der nicht aminresistente Silikonkleber mit hoher Klebrigkeit in einem Gewichtsbereich von bevorzugt 45,0-100,0 Gew.-%, bevorzugter 55,0-100,0 Gew.-%, noch bevorzugter 65,0-100,0 Gew.-%, noch bevorzugter 75,0-100,0 Gew.-%, bezogen auf das Gesamtgewicht an Silikonkleber in der Matrixschicht (2) und/oder in der mindestens einen zusätzlichen initial wirkstofffreien Haftkleberschicht (3), falls vorhanden, vor. Dabei kann bei Vorliegen einer Matrixschicht (2) und mindestens einer zusätzlichen initial wirkstofffreien Haftkleberschicht (3) im TTS das Gewichtsverhältnis von nicht aminresistentem Silikonkleber mit mittlerer Klebrigkeit zu nicht aminresistentem Silikonkleber mit hoher Klebrigkeit in beiden Schichten gleich oder unterschiedlich sein.

Bei den nicht aminresistenten Silikonklebern im Sinne der vorliegenden Erfindung kann es sich um nicht silanolreduzierte Silikonkleber (d.h. nicht aminresistente Silikonkleber, wobei die Silanolgruppen nicht durch Schutzgruppen geschützt sind) oder um silanolreduzierte Silikonkleber (d.h. nicht aminresistente Silikonkleber, wobei die Silanolgruppen nur teilweise durch Schutzgruppen geschützt sind) handeln. Solche nicht silanolreduzierten bzw. nur teils silanolreduzierten Silikonkleber, sind aus dem Stand der Technik bekannt. Bevorzugte nicht aminresistente Silikonkleber sind nicht silanolreduzierte nicht aminresistente Silikonkleber wie beispielsweise Dow Corning^{®} BIO-PSA 7-4501, Dow Corning^{®} BIO-PSA 7-4601, Dow Corning^{®} BIO-PSA 7-4502, Dow Corning^{®} BIO-PSA 7-4602 oder die silanolreduzierten nicht aminresistenten Silikonkleber die aufgrund ihrer geringen Silanolreduzierung noch zu den nicht aminresistenten Silikonklebern gehören, wie Dow Corning^{®} BIO-PSA SRS7-4501 und Dow Corning^{®} BIO-PSA SRS7-4601. Besonders bevorzugt sind die nicht aminresistenten Silikonkleber Dow Corning^{®} BIO-PSA 7-4501, Dow Corning^{®} BIO-PSA 7-4601, Dow Corning^{®} BIO-PSA SRS7-4501 und Dow Corning^{®} BIO-PSA SRS7-4601, noch bevorzugter sind Dow Corning^{®} BIO-PSA SRS7-4501 und Dow Corning^{®} BIO-PSA SRS7-4601.

Die Erfinder der vorliegenden Erfindung haben außerdem überraschenderweise gefunden, dass durch Einsatz von silanolreduzierten nicht aminresistenten Silikonklebern, anstatt von nicht silanolreduzierten nicht aminresistenten Silikonklebern, die Klebkraft der resultierenden transdermalen therapeutischen Systeme gesteigert werden kann. Der Begriff "silanolreduziert" im Sinne der vorliegenden Erfindung bedeutet, dass im so bezeichneten nicht aminresistenten Silikonkleber ein Teil der Silanolgruppen durch Schutzgruppen geschützt ist, d.h. ein solcher silanolreduzierter Silikonkleber ist immer noch ein nicht aminresistenter Silikonkleber im Sinne der vorliegenden Erfindung. Silanolreduzierte nicht aminresistente Silikonkleber sind aus dem Stand der Technik bekannt, und beispielsweise in der U.S. Patentschrift US 6,337,086 beschrieben. Dow Corning^{®} BIO-PSA SRS7-4501, Dow Corning^{®} BIO-PSA SRS7-4601, Dow Corning^{®} BIO-PSA SRS7-4502, Dow Corning^{®} BIO-PSA SRS7-4602 sind Beispiele silanolreduzierter nicht aminresistenter Silikonkleber. Bevorzugt sind die silanolreduzierten nicht aminresistenten Silikonkleber Dow Corning^{®} BIO-PSA SRS7-4501 und Dow Corning^{®} BIO-PSA SRS7-4601. Gemäß einer bevorzugten Ausführungsform der vorliegenden Erfindung liegt der Silanolgehalt eines silanolreduzierten nicht aminresistenten Silikonklebers in ppm bevorzugt zwischen um 8000 ppm und 13000 ppm und der Silanolgehalt eines nicht silanolreduzierten nicht aminresistenten Silikonklebers bevorzugt über 13000 ppm, und kann, beispielsweise wie in US 6,337,086 beschrieben, beispielsweise mittels ²⁹Si-NMR Spektroskopie und/oder FTIR-Spektroskopie bestimmt werden.

In einer bevorzugten Ausführungsform der vorliegenden Erfindung umfasst der eine oder die mehreren nicht aminresistenten Silikonkleber der Matrixschicht (2) einen oder mehrere silanolreduzierte nicht aminresistente Silikonkleber, vorzugsweise mit einem Gewichtsanteil an silanolreduziertem nicht aminresistentem Silikonkleber von mehr als 50 Gew.-%, bevorzugter mehr als 60.-%, noch bevorzugter mehr als 75 Gew.-%, noch bevorzugter mehr als 85 Gew.-%, noch bevorzugter mehr als 90 Gew.-%, noch bevorzugter mehr als 95 Gew.-%, noch bevorzugter mindestens 99 Gew.-%, bezogen auf das Gesamtgewicht der nicht aminresistenten Silikonkleber der Matrixschicht (2).

In einer bevorzugten Ausführungsform der vorliegenden Erfindung, in der das transdermale therapeutische Systeme mindestens eine zusätzliche initial wirkstofffreie Haftkleberschicht (3) umfasst, umfasst der eine oder die mehreren nicht aminresistenten Silikonkleber der mindestens einen zusätzlichen initial wirkstofffreien Haftkleberschicht (3) einen oder mehrere silanolreduzierte nicht aminresistente Silikonkleber, vorzugsweise mit einem Gewichtsanteil an silanolreduziertem nicht aminresistentem Silikonkleber von mehr als 50 Gew.-%, bevorzugter mehr als 60.-%, noch bevorzugter mehr als 75 Gew.-%, noch bevorzugter mehr als 85 Gew.-%, noch bevorzugter mehr als 90 Gew.-%, noch bevorzugter mehr als 95 Gew.-%, noch bevorzugter mindestens 99 Gew.-%, bezogen auf das Gesamtgewicht der nicht aminresistenten Silikonkleber der mindestens einen zusätzlichen initial wirkstofffreien Haftkleberschicht (3).

Beispielsweise kann der eine oder die mehreren nicht aminresistenten Silikonkleber der Matrixschicht (2) auch zwei, drei, vier, etc. silanolreduzierte nicht aminresistente Silikonkleber, vorzugsweise mit den oben beschriebenen Gewichtsanteilen am Gesamtgewicht der nicht aminresistenten Silikonkleber der Matrixschicht (2), umfassen. Gleichermaßen kann beispielsweise auch der eine oder die mehreren nicht aminresistenten Silikonkleber der mindestens einen zusätzlichen initial wirkstofffreien Haftkleberschicht (3) auch zwei, drei, vier, etc. silanolreduzierte nicht aminresistente Silikonkleber, vorzugsweise mit den oben beschrieben Gewichtsanteilen aber bezogen auf das Gesamtgewicht des nicht aminresistenten Silikonklebers der mindestens einen zusätzlichen initial wirkstofffreien Haftkleberschicht (3), umfassen.

Wie die Erfinder der vorliegenden Erfindung beobachtet haben, kann die Verwendung von einem silanolreduzierten nicht aminresistenten Silikonklebers mit hoher Klebrigkeit wie Dow Corning^{®} BIO-PSA SRS7-4601, auch bei Einsatz einer Schutzfolie (4) aus fluorosilikonisierter Folie wie beispielsweise Scotchpak 9709 (3M Corporation) zu einem deutlichen Trennkraftanstieg während der Lagerung führen. Überraschenderweise haben die vorliegenden Erfinder gefunden, dass durch die Verwendung eines Gemisches von einem silanolreduzierten nicht aminresistenten Silikonklebers mit mittlerer Klebrigkeit, wie beispielsweise Dow Corning^{®} BIO-PSA SRS7-4501, und einem silanolreduzierten nicht aminresistenten Silikonklebers mit hoher Klebrigkeit, wie beispielsweise Dow Corning^{®} BIO-PSA SRS7-4601, ein solcher Trennkraftanstieg während der Lagerung deutlich reduziert werden kann.

Deshalb enthält in einer erfindungsgemäßen Ausführungsform, in welcher der eine oder die mehreren nicht aminresistenten Silikonkleber der Matrixschicht (2) silanolreduzierte nicht aminresistenten Silikonkleber entweder umfasst oder ausschließlich aus solchen besteht, die Matrixschicht (2) vorzugsweise eine Mischung aus einem oder mehreren silanolreduzierten nicht aminresistenten druckempfindlichen Silikonkleber mit mittlerer Klebrigkeit, wie beispielsweise Dow Corning^{®} BIO-PSA SRS7-4501, und einem oder mehreren silanolreduzierten nicht aminresistenten druckempfindlichen Silikonkleber mit hoher Klebrigkeit, wie beispielsweise Dow Corning^{®} BIO-PSA SRS7-4601.

In einer weiteren Ausführungsform der vorliegenden Erfindung, in der das transdermale therapeutische System mindestens eine zusätzliche initial wirkstofffreie Haftkleberschicht (3) umfasst, und in der der eine oder die mehreren nicht aminresistenten Silikonkleber der mindestens einen zusätzlichen initial wirkstofffreien Haftkleberschicht (3) silanolreduzierte nicht aminresistente Silikonkleber umfassen oder ausschließlich aus solchen besteht, enthält die mindestens eine zusätzliche initial wirkstofffreie Haftkleberschicht (3) bevorzugt eine Mischung aus einem oder mehreren silanolreduzierten nicht aminresistenten druckempfindlichen Silikonkleber mit mittlerer Klebrigkeit, wie beispielsweise Dow Corning^{®} BIO-PSA SRS7-4501, und einem oder mehreren silanolreduzierten nicht aminresistenten druckempfindlichen Silikonkleber mit hoher Klebrigkeit, wie beispielsweise Dow Corning^{®} BIO-PSA SRS7-4601.

In einer bevorzugten Ausführungsform der vorliegenden Erfindung, in der welcher der eine oder die mehreren nicht aminresistenten Silikonkleber der Matrixschicht (2) und/oder der mindestens einen zusätzlichen initial wirkstofffreien Haftkleberschicht (3), falls vorhanden, eine Mischung aus einem oder mehreren silanolreduzierten nicht aminresistenten druckempfindlichen Silikonkleber mit mittlerer Klebrigkeit, und einem oder mehreren silanolreduzierten nicht aminresistenten druckempfindlichen Silikonkleber mit hoher Klebrigkeit, enthält, oder aus einer solchen Mischung besteht, liegt der silanolreduzierte nicht aminresistente Silikonkleber mit mittlerer Klebrigkeit in einem Gewichtsbereich von bevorzugt 10,0-40,0 Gew.-%, bevorzugter 15,0-35,0 Gew.-%, noch bevorzugter um 17,5-30,0 Gew.-%, noch bevorzugter 17,5-30,0 Gew.-%, und der silanolreduzierte nicht aminresistente Silikonkleber mit hoher Klebrigkeit in einem Gewichtsbereich von bevorzugt 60,0-90,0 Gew.-%, bevorzugter 65,0-85,0 Gew.-%, noch bevorzugter um 70,0-82,5 Gew.-%, noch bevorzugter 70,0-82,5 Gew.-%, bezogen auf das Gesamtgewicht an Silikonkleber in der Matrixschicht (2) und/oder in der mindestens einen zusätzlichen initial wirkstofffreien Haftkleberschicht (3), falls vorhanden, vor. Dabei kann bei Vorliegen einer Matrixschicht (2) und mindestens einer zusätzlichen initial wirkstofffreien Haftkleberschicht (3) im TTS das Gewichtsverhältnis von silanolreduziertem nicht aminresistentem Silikonkleber mit mittlerer Klebrigkeit zu silanolreduziertem nicht aminresistentem Silikonkleber mit hoher Klebrigkeit in beiden Schichten gleich oder unterschiedlich sein.

In einer bevorzugten Abwandlung der obengenannten Ausführungsformen des transdermalen therapeutischen Systems mit silanolreduziertem nicht aminresistentem druckempfindlichem Silikonkleber, besteht der eine oder die mehreren nicht aminresistenten druckempfindlichen Silikonkleber der Matrixschicht (2) und/oder der mindestens einen zusätzlichen initial wirkstofffreien Haftkleberschicht (3), falls vorhanden, ausschließlich aus silanolreduziertem nicht aminresistentem druckempfindlichem Silikonkleber. In einer weiteren bevorzugten Abwandlung der obengenannten Ausführungsformen, weist die Matrixschicht (2) und/oder die mindestens eine zusätzliche initial wirkstofffreie Haftkleberschicht (3), falls vorhanden, als druckempfindliche Haftkleber ausschließlich silanolreduzierte nicht aminresistente druckempfindliche Silikonkleber auf.

In noch einer weiteren bevorzugten Abwandlung der obengenannten Ausführungsformen des transdermalen therapeutischen Systems mit silanolreduzierten nicht aminresistenten druckempfindlichen Silikonkleber, bestehen die druckempfindlichen Haftkleber der Matrixschicht (2) und/oder der mindestens einen zusätzlichen initial wirkstofffreien Haftkleberschicht (3), falls vorhanden, ausschließlich aus einer Mischung aus einem silanolreduzierten nicht aminresistenten druckempfindlichen Silikonkleber mit mittlerer Klebrigkeit, wie beispielsweise Dow Corning^{®} BIO-PSA SRS7-4501, und einem silanolreduzierten nicht aminresistenten druckempfindlichen Silikonkleber mit hoher Klebrigkeit, wie beispielsweise Dow Corning^{®} BIO-PSA SRS7-4601.

In einer bevorzugten erfindungsgemäßen Ausführungsform einer Bilayer-Formulierung, besteht der eine oder die mehreren nicht aminresistenten Silikonkleber der Matrixschicht (2) ausschließlich aus einem silanolreduzierten nicht aminresistenten druckempfindlichen Silikonkleber mit mittlerer Klebrigkeit, wie beispielsweise Dow Corning^{®} BIO-PSA SRS7-4501, und der eine oder die mehreren nicht aminresistenten Silikonkleber der mindestens einen zusätzlichen initial wirkstofffreien Haftkleberschicht (3) ausschließlich aus einer Mischung aus einem silanolreduzierten nicht aminresistenten druckempfindlichen Silikonkleber mit mittlerer Klebrigkeit, wie beispielsweise Dow Corning^{®} BIO-PSA SRS7-4501, und einem silanolreduzierten nicht aminresistenten druckempfindlichen Silikonkleber mit hoher Klebrigkeit, wie beispielsweise Dow Corning^{®} BIO-PSA SRS7-4601. In weiteren einer bevorzugten erfindungsgemäßen Ausführungsform einer Bilayer-Formulierung, bestehen die druckempfindlichen Haftkleber der Matrixschicht (2) ausschließlich aus einem silanolreduzierten nicht aminresistenten druckempfindlichen Silikonkleber mit mittlerer Klebrigkeit, und die druckempfindlichen Haftkleber der mindestens einen zusätzlichen initial wirkstofffreien Haftkleberschicht (3) ausschließlich aus einer Mischung aus einem silanolreduzierten nicht aminresistenten druckempfindlichen Silikonkleber mit mittlerer Klebrigkeit und einem silanolreduzierten nicht aminresistenten druckempfindlichen Silikonkleber mit hoher Klebrigkeit.

In einer bevorzugten Abwandlung der obigen Ausführungsformen mit einem oder mehreren silanolreduzierten nicht aminresistenten Silikonkleber wird anstelle des einen oder mehreren silanolreduzierten nicht aminresistenten Silikonkleber, ein oder mehrere nicht silanolreduzierte nicht aminresistente Silikonkleber verwendet. D.h. bspw. in einer bevorzugten Ausführungsform der vorliegenden Erfindung umfasst der eine oder die mehreren nicht aminresistenten Silikonkleber der Matrixschicht (2), und/oder der mindestens einen zusätzlichen initial wirkstofffreien Haftkleberschicht (3), falls vorhanden, einen oder mehrere nicht silanolreduzierten nicht aminresistenten Silikonkleber, vorzugsweise mit einem Gewichtsanteil an nicht silanolreduziertem nicht aminresistentem Silikonkleber von mehr als 50 Gew.-%, bevorzugter mehr als 60.-%, noch bevorzugter mehr als 75 Gew.-%, noch bevorzugter mehr als 85 Gew.-%, noch bevorzugter mehr als 90 Gew.-%, noch bevorzugter mehr als 95 Gew.-%, noch bevorzugter mindestens 99 Gew.-%, bezogen auf das Gesamtgewicht der nicht aminresistenten Silikonkleber der Matrixschicht (2), und/oder der mindestens einen zusätzlichen initial wirkstofffreien Haftkleberschicht (3), falls vorhanden. Oder bspw. in einer bevorzugten Abwandlung der obengenannten bevorzugten Ausführungsformen, besteht der eine oder die mehreren nicht aminresistenten druckempfindlichen Silikonkleber der Matrixschicht (2) und/oder der mindestens einen zusätzlichen initial wirkstofffreien Haftkleberschicht (3), falls vorhanden, ausschließlich aus nicht silanolreduziertem nicht aminresistentem druckempfindlichem Silikonkleber.

Beispielsweise kann der eine oder die mehreren nicht aminresistenten Silikonkleber der Matrixschicht (2), und/oder der eine oder die mehreren nicht aminresistenten Silikonkleber der mindestens einen zusätzlichen initial wirkstofffreien Haftkleberschicht (3), falls mindestens eine solche initial wirkstofffreien Haftkleberschicht (3) vorhanden ist, auch zwei, drei, vier, etc. nicht silanolreduzierte nicht aminresistente Silikonkleber, vorzugsweise mit den oben beschrieben Gewichtsanteilen am Gesamtgewicht der nicht aminresistenten Silikonkleber der Matrixschicht (2), bzw. der mindestens einen initial wirkstofffreien Haftkleberschicht (3), umfassen. Nicht silanolreduzierte nicht aminresistente Silikonkleber sind dem Fachmann. Bevorzugte nicht silanolreduzierte nicht aminresistente Silikonklebern sind Dow Corning^{®} BIO-PSA 7-4501, Dow Corning^{®} BIO-PSA 7-4601, Dow Corning^{®} BIO-PSA 7-4502 und Dow Corning^{®} BIO-PSA 7-4602.

Sämtliche vorstehenden Ausführungsformen können bevorzugt so ausgestaltet sein, dass die Matrixschicht (2) des transdermalen therapeutischen Systems, und/oder die mindestens eine zusätzliche initial wirkstofffreie Haftkleberschicht (3), falls vorhanden, und/oder das gesamte transdermale therapeutische System als druckempfindliche Haftkleber ausschließlich Silikonkleber enthält. Sämtliche vorstehenden Ausführungsformen können außerdem bevorzugt so ausgestaltet sein, dass die Matrixschicht (2) des transdermalen therapeutischen Systems, und/oder die mindestens eine zusätzliche initial wirkstofffreie Haftkleberschicht (3), falls vorhanden, und/oder das gesamte transdermale therapeutische System als druckempfindliche Silikonkleber ausschließlich nicht aminresistente druckempfindliche Silikonkleber enthält; d.h. insbesondere dass die Matrixschicht (2) und/oder die mindestens eine zusätzliche initial wirkstofffreie Haftkleberschicht (3), falls vorhanden, und/oder das gesamte transdermale therapeutische System keine aminresistenten druckempfindlichen Silikonkleber sondern ausschließlich nicht aminresistente druckempfindliche Silikonkleber enthält.

Die Anwendungsdauer des erfindungsgemäßen transdermalen therapeutischen Systems beträgt bevorzugt einen Tag, d.h. das erfindungsgemäße transdermale therapeutische System wird nach einem Tag von der Haut entfernt. Da die erfindungsgemäßen transdermalen therapeutischen Systeme in der Regel für die Langzeitbehandlung (mehrere Monate oder auch mehrere Jahre) verwendet werden, wird nach dem Entfernen eines transdermalen therapeutischen Systems nach einem Tag ein neues erfindungsgemäßes transdermales therapeutisches System auf die Haut aufgeklebt.

Die transdermalen therapeutischen Systeme der Erfindung können aber auch für mehr als einen Tag angewendet werden, z.B. 2 Tage oder 3 Tage. In diesen Fällen erfolgt ein Ersatz eines erfindungsgemäßen transdermalen therapeutischen Systems durch ein neues transdermales therapeutisches System nach 2 bzw. 3 Tagen.

Auf der der menschlichen Haut beim Gebrauch abgewandten Seite der Klebstoffmatrix befindet sich eine Rückschicht (1), die in einer bevorzugter Ausführungsform für den Wirkstoff okklusiv, also nicht durchlässig ist. Besonders bevorzugt ist auch, dass die Rückschicht weitestgehend lichtundurchlässig ist. Derartige Rückschichten können in einer Ausführungsform aus Polyester, Polyolefinen, insbesondere Polyethylen, oder Polyurethanen bestehen. Auch Rückschichten, die mehrere verschiedene Polymere übereinander angeordnet beinhalten, sind vorteilhafterweise einsetzbar. Bevorzugt weist die Rückschicht eine hohe Wasserdampfundurchlässigkeit auf.

Ein bevorzugtes Material für die Rückschicht ist Polyester in der Form einer Verbundfolie mit Polyester innen, einer zentralen Aluminiumbarriere und pigmentiertem Polyethylen außen. Besonders bevorzugte Rückschichten sind beispielsweise die Polyester-basierten Folien die von der Firma 3M unter der Bezeichnung Scotchpak 1109 oder Scotchpak 9738 vertrieben werden oder die Polyester-basierten Folien die von der Firma Mitsubishi Polyester Film unter der Bezeichnung Hostaphan^{®} MN19, Hostaphan^{®} MN 19 Med und Hostaphan^{®} MN 15 Med vertrieben werden; besonders bevorzugt für eine Monolayer-Formulierung ist bspw. Scotchpak 9738 und für eine Bilayer-Formulierung bspw. Hostaphan^{®} MN 19 Med.

Andere geeignete Materialien umfassen Cellophan, Celluloseacetat, Ethylcellulose, mit Weichmachern versehene Vinylacetatvinylchlorid-copolymere, Ethylenvinyl-acetatcopolymere, Polyethylenterephthalat, Nylon, Polyethylen, Polypropylen, Polyvinylidenchlorid, Ethylenmethacrylatcopolymer, Papier, das gegebenenfalls beschichtet sein kann, textile Gewebe, wie Polyethylentherephthalatfolien, Aluminiumfolien und Polymer-Metall-Kompositmaterialien.

Die Rückschicht der erfindungsgemäßen umfasst eine Polyesterfolie mit einer Dick von unter 35 µm oder besteht aus einer Polyesterfolie mit einer Dicke von unter 70 µm. In einer bevorzugten Ausführungsform umfasst die Rückschicht (1) eine Polyesterfolie, mit einer Dicke 5-30 µm, bevorzugt 10-25 µm, bevorzugter 15-23 µm. In einer weiteren Ausführungsform besteht die Rückschicht (1) aus einer Polyesterfolie, mit einer Dicke 15 - 65 µm, bevorzugt 25- 60 µm, bevorzugter 30 - 60 µm, alternativ besonders bevorzugt 49 - 60 µm, weiter alternativ besonders bevorzugt 31 - 37 µm.

Auf der Rückschicht (1) des Pflasters kann sich noch eine Abdeckschicht befinden, die insbesondere verhindern soll, dass das Pflaster mit der Verpackung verklebt, falls geringe Mengen des Matrixmaterials austreten. Die Abdeckschicht liegt bevorzugt lose auf der Rückschicht auf und wird durch elektrostatische Kräfte gehalten. Derartige Abdeckschichten sind im Stand der Technik bekannt, z.B. aus der EP 1 097 090, auf die insoweit vollinhaltlich Bezug genommen wird. Die Abdeckschicht ist zumindest auf der auf der Rückschicht aufliegenden Seite antihaftbeschichtet, z.B. fluoriert oder fluorsilikonisiert.

Weiterhin umfasst das erfindungsgemäße transdermale therapeutische System eine vor Gebrauch zu entfernende Schutzfolie (4) (Release Liner). Die Schutzfolie (4) folgt auf die Matrixschicht (2), bzw. falls mindestens eine zusätzliche initial wirkstofffreie Haftkleberschicht (3) vorhanden ist auf die mindestens eine zusätzliche initial wirkstofffreie Haftkleberschicht (siehe bspw. Abbildung 1 (A) bzw. (B)). Dabei ist die vor Gebrauch zu entfernende Schutzfolie (4) bevorzugt die Außenschicht des TTS, so dass eine Seite der vor Gebrauch zu entfernenden Schutzfolie (4) eine Außenseite bildet. Falls keine zusätzliche initial wirkstofffreie Haftkleberschicht (3) vorhanden ist, ist die vor Gebrauch zu entfernende Schutzfolie (4) bevorzugt mit der Matrixschicht (2) in direktem Kontakt, so dass die gegenüberliegende Seite der vor Gebrauch zu entfernenden Schutzfolie (4) die Außenseite des TTS darstellt. Falls eine zusätzliche initial wirkstofffreie Haftkleberschicht (3) vorhanden ist, befindet sich die zusätzliche initial wirkstofffreie Haftkleberschicht (3) zwischen der Matrixschicht (2) und der vor Gebrauch zu entfernenden Schutzfolie (4) und ist bevorzugt in direktem Kontakt mit der Matrixschicht (2) und/oder der vor Gebrauch zu entfernenden Schutzfolie (4). Falls mehr als eine zusätzliche initial wirkstofffreie Haftkleberschicht (3) vorhanden ist, befinden sich die mehreren zusätzlichen initial wirkstofffreien Haftkleberschichten (3) zwischen der Matrixschicht (2) und der vor Gebrauch zu entfernenden Schutzfolie (4), so dass die vor Gebrauch zu entfernende Schutzfolie (4) bevorzugt mit jener zusätzlichen initial wirkstofffreien Haftkleberschicht (3) in direktem Kontakt ist, welche am weitesten von der Matrixschicht (2) entfernt ist.

Die vor Gebrauch zu entfernende Schutzfolie (4) ist bevorzugt aus polymerem Material hergestellt, das gegebenenfalls auch metallisiert sein kann. Beispiele für bevorzugt eingesetzte polymere Materialien sind Polyester, Polyurethane, Polyvinylacetat, Polyvinylidenchlorid, Polypropylen, Polycarbonat, Polystyrol, Polyethylen, Polyethylenterephthalat, Polybutylenterephthalat sowie gegebenenfalls mit entsprechenden Polymeren oberflächenbeschichtetes Papier. Bevorzugt handelt es sich hierbei um eine einseitig oder beidseitig fluorsilikonisierte Schutzfolie (4). Besonders bevorzugt sind handelsübliche fluorsilikonisierte Polyesterfolien, wie das fluorsilikonisierte Handelsprodukt Scotchpak 9709 (Firma 3M). In einer bevorzugten Ausführungsform umfasst das transdermale therapeutische System weiterhin eine vor Gebrauch zu entfernende Schutzfolie (4) bestehend aus einer fluorsilikonisierten Folie, vorzugsweise einer fluorsilikonisierten Polyesterfolie.

In einer bevorzugten Ausführungsform besteht das transdermale therapeutische System gemäß der Erfindung aus der Rückschicht (1); der Matrixschicht (2), die sich auf der Rückschicht befindet; und der vor Gebrauch zu entfernenden Schutzfolie (4), die sich auf der Matrixschicht befindet.

In einer weiteren bevorzugten Ausführungsform besteht das transdermale therapeutische System aus der Rückschicht (1); der Matrixschicht (2), die sich auf der Rückschicht befindet; der mindestens einen zusätzlichen initial wirkstofffreien Haftkleberschicht (3), die sich zwischen der Matrixschicht und der vor Gebrauch zu entfernenden Schutzfolie (4) befindet; und der vor Gebrauch zu entfernenden Schutzfolie (4), die sich auf der mindestens einen zusätzlichen initial wirkstofffreien Haftkleberschicht befindet.

Erfindungsgemäß befindet sich der Wirkstoff in der Matrixschicht (2). Bei dem Wirkstoff handelt es sich um Rotigotin oder ein pharmazeutisch verträgliches Salz von Rotigotin, bevorzugt um Rotigotin. Hinsichtlich der verwendbaren polymorphen Formen des Rotigotins ist die Erfindung nicht eingeschränkt, aus Stabilitätsgründen wird jedoch Rotigotin der polymorphen Form II, wie sie in der WO 2009/068520 beschrieben ist, bevorzugt. Hinsichtlich der Herstellung und der Charakterisierung des Rotigotins der polymorphen Form II wird vollumfänglich auf die WO 2009/068520 Bezug genommen.

Der Wirkstoff liegt in der Matrixschicht (der Klebermatrix) bevorzugt vollständig gelöst vor, d.h. die Matrixschicht enthält bevorzugt keine festen Wirkstoffteilchen.Der Rotigotingehalt in der Matrixschicht (2) ist bevorzugt im Bereich von 5 Gew.-% bis 25 Gew.-%, bevorzugter im Bereich von 6 Gew.-% bis 20 Gew.-%, noch bevorzugter im Bereich von 6 Gew.-% bis 15 Gew.-%, noch bevorzugter im Bereich von 6,5 Gew.-% bis 11,5 Gew.-%, beispielsweise 6,875-9 Gew.-%, insbesondere um 7,5-9 Gew.-%, an Rotigotin bezogen auf das Gesamtgewicht der Matrixschicht (2).

Rotigotin liegt erfindungsgemäß in der Matrixschicht (der Klebermatrix) im Wesentlichen in der dispergierten Phase einer festen Dispersion in einer nicht-kristallinen Form vor, wobei der eine oder die mehreren nicht aminresistenten Silikonkleber sowie mögliche andere Polymerkleber bevorzugt das Dispersionsmittel bilden. Erfindungsgemäß umfasst die dispergierte Phase neben nicht-kristallinem Rotigotin ein Polyvinylpyrrolidon.

Rotigotin ist in Silikonklebern sehr schlecht löslich, z.B. in einem Kristallisationshemmer wie Polyvinylpyrrolidon aber gut löslich. Bei den erfindungsgemäßen transdermalen therapeutischen Systemen weist die Matrixschicht (2) daher neben den nicht aminresistenten Silikonklebern somit noch darin dispergiertes Polyvinylpyrrolidon auf. Das Rotigotin ist in der Matrixschicht vorzugsweise vollständig gelöst, d.h. der Gehalt an Rotigotin im Silikonkleber ist so gering, dass vorzugsweise kein Rotigotin ausfällt/kristalisiert und der größte Teil des Rotigotins liegt vorzugsweise gelöst (oder zumindest in nicht-kristalliner Form) in dem dispergierten Polyvinylpyrrolidon vor.

Polyvinylpyrrolidon (PVP) ist ein Polymer aus dem Monomer N-Vinylpyrrolidon. Es ist bekannt dafür, dass es die Kohäsion von Silikonklebern erhöhen kann. Polyvinylpyrrolidon kann auch als Kristallisationshemmer für den Wirkstoff Rotigotin dienen. Das Molekulargewicht des Polyvinylpyrrolidons kann im Bereich von 2.000 bis 2.500.000 Dalton (g / mol) (angegeben als Durchschnittsgewicht), bevorzugt in einem Bereich von 700.000 bis 1.500.000 Dalton, noch bevorzugter in einem Bereich von 900.000 bis 1.500.000 Dalton, liegen. Verschiedene PVP-Qualitäten sind im Handel erhältlich, beispielsweise von BASF AG, Ludwigshafen, Deutschland, z.B. unter dem Namen Kollidon. Beispielsweise sind die folgenden Kollidonsorten wasserlösliche Formen von PVP: K-12 PF (Molekulargewicht = 2.000 - 3.000 Dalton); K-17 PF (Molekulargewicht = 7.000 - 11.000 Dalton); K-25 (Molekulargewicht = 28.000 - 34.000 Dalton); K-30 (Molekulargewicht = 44.000 - 54.000 Dalton); und K-90 (Molekulargewicht = 900.000 - 1.500.000 Dalton). In einer bevorzugten Ausführungsform liegt das Molekulargewicht des Polyvinylpyrrolidons im Bereich von. 28.000 bis 1.500.000 Dalton (g / mol).

Die vorliegenden Erfinder haben überraschenderweise gefunden, dass in einem transdermalen therapeutischen System mit dem Wirkstoff Rotigotin und einem oder mehreren nicht aminresistenten Silikonklebern in der Matrixschicht in einer Menge von mehr als 50 Gew.-% bezogen auf das Gesamtgewicht der druckempfindlichen Haftkleber der Matrixschicht (2), bei Verwendung von Rotigotin und einem Polyvinylpyrrolidon in der Matrixschicht in der dispergierten Phase einer festen Dispersion in einem Gewichtsverhältnis von 9 : 7 oder kleiner keine Kristallbildung auch nach längeren Lagerungszeiten bei 25 °C oder höher erfolgt. Bei einem größeren Gewichtsverhältnis von Rotigotin und Polyvinylpyrrolidon, beispielsweise von 9 : 5 oder größer wurden dagegen nach längeren Lagerzeiten bei Temperaturen von 25 °C oder höher Kristalle gefunden.

In einer bevorzugten Ausführungsform der erfindungsgemäßen transdermalen therapeutischen Systeme liegt deshalb das Rotigotin in der Matrixschicht im Wesentlichen in einer nicht-kristallinen Form in der dispergierten Phase einer festen Dispersion umfassend ein Polyvinylpyrrolidon (PVP) vor, wobei das Gewichtsverhältnis von Rotigotin zu Polyvinylpyrrolidon höchstens 9 : 7, vorzugsweise 9 : 7 bis 9 : 10, beträgt. Bei einem solchen Gewichtsverhältnis kann die Matrixschicht beispielsweise 5.14-12,86 Gew.-% Rotigotin und 4-10 Gew.-% Polyvinylpyrrolidon, oder 6,88-9 Gew.-% Rotigotin und 5,35-7 Gew.-% Polyvinylpyrrolidon, bezogen auf das Gesamtgewicht der Matrixschicht, enthalten. In weiteren bevorzugten Ausführungsformen der vorliegenden Erfindung liegen mindestens 70 Gew.-%, mindestens 80 Gew.-%, mindestens 85 Gew.-%, mindestens 90 Gew.-%, mindestens 95 Gew.-%, mindesten 97,5 Gew.-%, des Rotigotins in der Matrixschicht in einer nicht-kristallinen Form in der dispergierten Phase einer festen Dispersion umfassend ein Polyvinylpyrrolidon (PVP) vor.

Passende Polyvinylpyrrolidone zur Verwendung zusammen mit Rotigotin in der Matrixschicht eines transdermalen therapeutischen Systems sind aus dem Stand der Technik bekannt. Solche Polyvinylpyrrolidone sind beispielweise in der WO 2011/076879 beschrieben. Bevorzugte Polyvinylpyrrolidone in Bezug auf die vorliegende Erfindung sind PVP K90 (BASF SE) Besonders bevorzugt ist der Polyvinylpyrrolidon-Typ K-90 (PVP K90).

In der Matrixschicht (2) und/oder der mindestens einen zusätzlichen initial wirkstofffreien Haftkleberschicht (3) können weitere Polymerkleber (d.h. druckempfindliche Haftkleber) enthalten sein die keine nicht aminresistenten Silikonkleber sind. Der Gewichtsanteil an weiteren Polymerkleber die keine nicht aminresistenten Silikonkleber sind in der Matrixschicht (2) ist kleiner als 50 Gew.-%, bevorzugt kleiner als 40 Gew.-%, bevorzugter kleiner als 30 Gew.-%, noch bevorzugter kleiner als 20 Gew.-%, noch bevorzugter kleiner als 10 Gew.-%, noch bevorzugter kleiner als 5 Gew.-%, bezogen auf das Gesamtgewicht der druckempfindlichen Haftkleber der Matrixschicht (2). Falls im TTS mindestens eine zusätzliche initial wirkstofffreie Haftkleberschicht (3) vorhanden ist, ist der Gewichtsanteil an weiteren Polymerkleber die keine nicht aminresistenten Silikonkleber sind in der mindestens einen zusätzlichen initial wirkstofffreien Haftkleberschicht (3) kleiner als 50 Gew.-%, bevorzugt kleiner als 40 Gew.-%, bevorzugter kleiner als 30 Gew.-%, noch bevorzugter kleiner als 20 Gew.-%, noch bevorzugter kleiner als 10 Gew.-%, noch bevorzugter kleiner als 5 Gew.-%, bezogen auf das Gesamtgewicht der mindestens einen zusätzlichen initial wirkstofffreien Haftkleberschicht (3). Solche weiteren Polymerkleber sind beispielsweise Polyacrylate, Polymethacrylate, SBS-Blockcopolymere und Polyisobutylene.

Polyacrylate und Polymethacrylate sind im Stand der Technik bekannt (vgl. bspw. US 2002/0077437) und werden vielfältig für transdermale therapeutische Systeme eingesetzt. Polyacrylate bzw. Polymethacrylate werden in der Regel durch radikalische Polymerisation von Acryl- bzw. Methacrylsäurederivaten, insbesondere Acryl- bzw. Methacrylsäureestern hergestellt, wobei auch andere geeignete Verbindungen wie z.B. Vinylacetat als zusätzliche Monomere copolymerisiert werden können. Es ist möglich, die Polyacrylate bzw. -methacrylate beispielsweise durch mehrwertige Metallionen zu vernetzen um die Eigenschaften der Polyacrylate bzw. -methacrylate zu verändern. Sowohl vernetzte als auch unvernetzte Polyacrylate bzw. -methacrylate sind kommerziell erhältlich, eine der wichtigsten Anbieter ist die Firma Henkel (bzw. National Starch) die die Polyacrylate und -methacrylate unter der Bezeichnung "DURO-TAK" vertreibt.

Beispiele sind Polyacrylate bzw. Polymethacrylate Copolymere oder Terpolymere aus Monomeren, die beispielsweise aus Acrylsäure, Methacrylsäure, Methoxyethylacrylat, Ethylacrylat, Ethylmethacrylat, Butylacrylat, Butylmethacrylat, Hexylacrylat, Hexylmethacrylat, Methylacrylat, Methylmethacrylat, 2-Ethylbutylacrylat, 2-Ethylbutylmethacrylat, Isooctylacrylat, losoctylmethacrylat, 2-Ethylhexylacrylat, 2-Ethylhexylmethacrylat, Decylacrylat, Decylmethacrylat, Dodecylacrylat, Dodecylmetacrylat, Tridecylacrylat, Tridecylmethacrylat, Hydroxyethylacrylat, Hydroxypropylacrylat, Dimethylaminoethylacrylat, Dimethylaminoethylmethacrylat, Tertbutylaminoethylacrylat, Tertbutylaminoethylmethacrylat, Methoxyethylacrylat, Methoxyethylmethacrylat, usw. ausgewählt sind. Gegebenenfalls können als Comonomere auch Acrylamid, Dimethylamid, Acrylnitril und Vinylacetat verwendet werden.

Weitere Beispiele geeigneter Acrylklebstoffe sind beispielsweise in Satas, "Acrylic Adhesives, Handbook of Pressure Sensitive Adhesives Technology, 2. Auflage, Seite 396-456 (D. Satas, Herausgeber) van Nostrand Reinhold, New York (1989) genannt. Sofern hierin von Polyacrylaten gesprochen wird, sind immer auch die entsprechenden Polymethacrylate gemeint.

Polyisobutylene sind im Stand der Technik bekannt und kommerziell erhältlich. Beispielsweise die Produkte Oppanol, die von der Firma BASF, Ludwigshafen, Deutschland vertrieben werden. Geeignete Polyisobutylene sind beispielsweise Oppanol B50, N50, B80, N80, B100, N100, B150, N150, B200 und N200 oder auch Oppanol B10SFN oder Oppanol B15SFN B10, B15. Es können beispielsweise auch Gemische aus einem Polyisobutylen, das beispielsweise ausgewählt wird aus Oppanol B80, Oppanol B100, Oppanol B150 und Oppanol B200, bevorzugt Oppanol B80 oder Oppanol B100, mit einem zweiten Polyisobutylen, das ausgewählt wird aus den Produkten Oppanol B10SFN und Oppanol B15SFN eingesetzt werden.

Soweit im Rahmen dieser Erfindung von dem Molekulargewicht von Polymeren gesprochen wird, handelt es sich immer um das gewichtsgemittelte Molekulargewicht M_{w}, sofern nichts anderes ausdrücklich erwähnt oder aufgrund des Zusammenhanges offensichtlich ist. Das gewichtsgemittelte Molekulargewicht M_{w} kann beispielsweise über GPC bestimmt werden, wie es dem Fachmann bekannt ist.

Bei den erfindungsgemäßen transdermalen therapeutischen Systemen kann die Matrixschicht, die den Wirkstoff enthält, neben den genannten druckempfindlichen Haftklebern, dem Rotigotin und gegebenenfalls dem Polyvinylpyrrolidon, gegebenenfalls noch weitere Bestandteile enthalten.

Beispielsweise kann der Matrixschicht ein Penetrationsverstärker zugesetzt sein, um eine ausreichende Permeation des Wirkstoffs durch die Haut zu gewährleisten. Geeignete Penetrationsverstärker sind bekannt. Hierbei handelt es sich beispielsweise um Fettalkohole, Fettsäuren, Fettsäureester, Fettsäureamide, Glycerin und Glycerinderivate, n-Methylpyrrolidon, Terpen und Terpenderivate, wie D-Limonen, α-Pinen, α-Terpineol, Carvone, Carveol, Limonenoxid, Pinenoxid, 1,8-Eukalyptol. Bevorzugt enthält das erfindungsgemäße transdermale therapeutische System jedoch keine derartigen Penetrationsverstärker.

Weiterhin können gegebenenfalls der Matrixschicht (2) ein oder mehrere Weichmacher zugesetzt werden. Geeignete Weichmacher sind ebenfalls im Stand der Technik bekannt, und hier können beispielsweise Weichmacher auf Grundlage von Mineralöl oder von Polybuten genannt werden.

In einer Ausführungsform enthält die Matrixschicht (2) und/oder die mindestens eine zusätzliche initial wirkstofffreie Haftkleberschicht (3), falls vorhanden, einen oder mehrere Zusatzstoffe, vorzugsweise einen Weichmacher

In einer bevorzugten Ausführungsform der Erfindung, enthält die Matrixschicht (2) noch einen oder mehrere Zusatzstoffe zur Verbesserung der chemischen Stabilität von Rotigotin, z.B. Antioxidantien wie Tocopherol und dessen Derivate, insbesondere Ester, Butylhydroxytoluol (BHT), Butylhydroxyanisol (BHA), Ascorbinsäure und ihre Derivate, insbesondere Ester und/oder Natrium-Metabisulfit. In einer Ausführungsform enthält die Matrixschicht (2) Tocopherol, Ascorbylpalmitat und Natrium-Metabisulfit, beispielsweise um 0,05-0,125 Gew.-% Tocopherol, 0,0-0,1 Gew.-% Ascorbylpalmitat und 0,0-0,0021 Gew.-% Natrium-Metabisulfit bezogen auf das Gesamtgewicht der Matrixschicht (2). Der mindestens einen zusätzlichen initial wirkstofffreien Haftkleberschicht (3) werden bevorzugt keine Antioxidantien oder Natrium-Metabisulfit zugegeben.

Das erfindungsgemäße transdermale therapeutische System kann zur Behandlung von allen Erkrankungen verwendet werden, bei denen eine Verabreichung des Wirkstoffs Rotigotin angezeigt ist. Insbesondere bevorzugt wird das erfindungsgemäße transdermale therapeutische System jedoch zur Behandlung der Parkinson-Erkrankung verwendet.

Das erfindungsgemäße transdermale therapeutische System kann auf an sich bekannte Art und Weise hergestellt werden. Für eine Monolayer-Formulierung werden beispielsweise alle Bestandteile der Matrixschicht des transdermalen therapeutischen Systems in einem geeigneten Lösemittel zusammengegeben und bis zur gewünschten Homogenität gerührt. Anschließend wird die homogenisierte Beschichtungsmasse auf die Rückschicht (1) oder bevorzugt auf eine Schutzfolie (4) aufgebracht und das Lösemittel wird durch Trocknen entfernt. Schließlich wird die noch verbleibende Schicht, d.h. die Schutzfolie (4) oder bevorzugt die Rückschicht (1), auf die Matrixschicht (2) kaschiert und transdermale therapeutische Systeme geeigneter Größe werden ausgestanzt.

Somit ist die vorliegende Erfindung gemäß einer bevorzugten Ausführungsform weiterhin gerichtet auf ein Verfahren zur Herstellung eines transdermalen therapeutischen Systems als Monolayer-Formulierung gemäß einer der oben beschriebenen Ausführungsformen des erfindungsgemäßen transdermalen therapeutischen Systems, umfassend a) das Herstellen einer homogenisierten Beschichtungsmasse durch Zusammengegeben aller Bestandteile der Matrixschicht (2) des transdermalen therapeutischen Systems in einem geeigneten Lösemittel und Vermischen bis zur gewünschten Homogenität; b) das Aufbringen der homogenisierten Beschichtungsmasse auf eine Rückschicht (1) oder bevorzugt auf eine Schutzfolie (4) und Entfernen des Lösemittels durch Trocknen; und c) das Kaschieren der verbleibenden Schicht, also eine Schutzfolie (4) oder bevorzugt eine Rückschicht (1), auf die Matrixschicht (2), und Ausstanzen transdermaler therapeutischer Systeme geeigneter Größe.

Für eine Bilayer-Formulierung bzw. Multilayer-Formulierung werden beispielsweise alle Bestandteile der wirkstoffhaltigen Matrixschicht (2) des transdermalen therapeutischen Systems in einem geeigneten Lösemittel zusammengegeben und bis zur gewünschten Homogenität gerührt. Anschließend wird diese erste gerührte oder homogenisierte Beschichtungsmasse auf die Rückschicht (1), oder bevorzugt auf eine vorläufige Schutzfolie (4), aufgebracht und das Lösemittel wird durch Trocknen entfernt. Schließlich wird die noch verbleibende Schicht, d.h. die vorläufige Schutzfolie (4) oder bevorzugt die Rückschicht (1) auf die Matrixschicht (2) kaschiert (erste Vorstufe des TTS). Danach werden die Bestandteile der mindestens einen zusätzlichen initial wirkstofffreien Haftkleberschicht (3) des transdermalen therapeutischen Systems in einem geeigneten Lösemittel zusammengegeben und bis zur gewünschten Homogenität gerührt. Anschließend wird diese weitere Beschichtungsmasse auf eine Schutzfolie (4) aufgebracht und das Lösemittel wird durch Trocknen entfernt (zweite Vorstufe des TTS). Schließlich wird die vorläufige Schutzfolie (4) von der ersten Vorstufe des TTS abgezogen und mit der mindestens einen zusätzlichen initial wirkstofffreien Haftkleberschicht (3) samt Schutzfolie (4) (zweite Vorstufe des TTS) zu einem Gesamtlaminat umfassend, oder bestehend, in dieser Reihenfolge aus Rückschicht (1), wirkstoffhaltiger Matrixschicht (2), mindestens einer zusätzlichen initial wirkstofffreien Haftkleberschicht (3) und Schutzfolie (4) zusammen kaschiert bzw. laminiert und aus dem Gesamtlaminat werden transdermale therapeutische Systeme geeigneter Größe ausgestanzt.

Somit ist die vorliegende Erfindung gemäß einer zweiten bevorzugten Ausführungsform weiterhin gerichtet auf ein Verfahren zur Herstellung eines transdermalen therapeutischen Systems, umfassend a) das Herstellen einer ersten Vorstufe des transdermalen therapeutischen Systems, umfassend a1) das Herstellen einer ersten homogenisierten Beschichtungsmasse durch Zusammengegeben aller Bestandteile der Matrixschicht (2) in einem geeigneten Lösemittel und Vermischen bis zur gewünschten Homogenität; a2) das Aufbringen der ersten homogenisierten Beschichtungsmasse auf eine Rückschicht (1), oder bevorzugt auf eine vorläufige Schutzfolie (4), und Entfernen des Lösemittels durch Trocknen; und a3) das Kaschieren der verbleibenden Schicht, also eine vorläufige Schutzfolie (4) oder bevorzugt eine Rückschicht (1), auf die Matrixschicht (2); b) das Herstellen einer zweiten Vorstufe des transdermalen therapeutischen Systems, umfassend b1) das Herstellen einer weiteren homogenisierten Beschichtungsmasse durch Zusammengegeben aller Bestandteile der mindestens einen zusätzlichen initial wirkstofffreien Haftkleberschicht (3) in einem geeigneten Lösemittel und Vermischen bis zur gewünschten Homogenität; b2) das Aufbringen der weiteren homogenisierten Beschichtungsmasse auf eine Schutzfolie (4) und Entfernen des Lösemittels durch Trocknen; und c) das Entfernen der vorläufigen Schutzfolie (4) von der ersten Vorstufe des transdermalen therapeutischen Systems aus a), das Kaschieren (kaschieren bzw. laminieren) der ersten Vorstufe und der zweiten Vorstufe des transdermalen therapeutischen Systems zu einem Gesamtlaminat umfassend, oder bestehend aus, in dieser Schichtreihenfolge, einer Rückschicht (1), einer wirkstoffhaltigen Matrixschicht (2), mindestens einer zusätzlichen initial wirkstofffreien Haftkleberschicht (3) und einer Schutzfolie (4), und das Ausstanzen transdermaler therapeutischer Systeme geeigneter Größe.

In den oben beschriebenen Verfahren zur Herstellung eines transdermalen therapeutischen Systems können weitere Schichten durch weitere Verfahrenszwischenschritte auf an sich bekannte Art und Weise eingefügt werden. Beispielsweise kann zwischen der Matrixschicht und der mindestens einen zusätzlichen initial wirkstofffreien Haftkleberschicht (3) eine Membran zur Regulierung der Wirkstofffreisetzung eingefügt werden. Oder ein TTS umfassend mindestens zwei zusätzliche initial wirkstofffreie Haftkleberschichten (3) kann beispielsweise hergestellt werden durch Entfernen der Schutzfolie (4) von bspw. einer Bilayer-Formulierung des TTS, hergestellt gemäß der vorstehenden zweiten bevorzugten Ausführungsform des Verfahrens zur Herstellung des erfindungsgemäßen TTS, und Kaschieren (kaschieren bzw. laminieren) dieser Bilayer-Formulierung mit einer weiteren zweiten Vorstufe des TTS, beispielsweise wie in Schritt b) (der vorstehenden zweiten bevorzugten Ausführungsform des Verfahrens zur Herstellung des erfindungsgemäßen TTS) hergestellt, zu einem Gesamtlaminat umfassend, in dieser Schichtreihenfolge, eine Rückschicht (1), eine wirkstoffhaltige Matrixschicht (2), eine erste und eine zweite zusätzliche initial wirkstofffreie Haftkleberschicht (3) und eine Schutzfolie (4), und Ausstanzen transdermaler therapeutischer Systeme geeigneter Größe. In dieser Weise können weitere Schichten in das TTS eingefügt werden.

Die folgenden Beispiele erläutern die Erfindung. Prozentangaben bedeuten immer Gewichtsprozent.

### BEISPIEL 1 a: Monolayer (Herstellung der Testformulierungen am Beispiel von 616ROTTS)

| Rohstoff | Zusammensetzung der getrockneter Matrix [%] | Zusammensetzung der nassen Matrix [g/m²] |
|---|---|---|
| Rotigotin | 7,50 | 4,50 |
| PVP K90 | 5,83 | 3,50 |
| Ethanol | --- | 19,82 |
| Natriummetabisulfit | 0,0021 | 0,0013 |
| Wasser, dest. | --- | 0,1247 |
| Ascorbylpalmitat | 0,02 | 0,01 |
| Tocopherol | 0,05 | 0,03 |
| Paraffin, dickflüssig | 2,10 | 1,26 |
| BIO-PSA^{®} SRS7-4501 (z.B. 66 % Feststoffanteil) | 21,12 | 19,22 |
| BIO-PSA^{®} SRS7-4601 (z.B. 63 % Feststoffanteil) | 63,37 | 60,51 |
| n-Heptan | --- | 2,14 |
| Summe | 100,0 | 111,1 |

Eine wässrige 1% (Gew.%)-Natriummetabisulfit-Lösung wurde hergestellt. Eine ethanolische 25% (Gew.%)-PVP K90-Lösung wurde hergestellt. Die entsprechende Menge an Natriummetabisulfit-Lösung wurde zur entsprechenden Menge an PVP-Lösung in ein geeignetes Glasgefäß gewogen und für ca. 15 min gerührt. Ascorbylpalmitat und Tocopherol wurden zugegeben und verrührt. Dann wurde der Wirkstoff Rotigotin unter Rühren und Erhitzung im Wasserbad langsam zugegeben und bei ungefähr 60°C gerührt bis zur vollständigen Lösung. Nachdem die Masse wieder abgekühlt war, wurden die Silikonkleber nacheinander hinzugewogen und kurz verrührt. Dann wurde das Ausgleichslösemittel Heptan zugegeben und verrührt. Zum Schluss wird Paraffin hinzugewogen. Die Beschichtungsmasse wurde gerührt bis alles visuell homogen verteilt war. Im Anschluss wurde die Beschichtungsmasse für z.B. ungefähr 3 Minuten bei ca. 10000 rpm mit einem geeigneten Dispergiergerät (Ultra-Turrax) bearbeitet. Die so homogenisierte Beschichtungsmasse wurde auf einer fluorbeschichteten Folie, z.B. Scotchpak^{™} 9709 /1022 / 9744, als dünner Film ausgestrichen und anschließend für z.B. 10 min bei 85°C erwärmt, so dass die Lösemittel nahezu vollständig entfernt wurden. Die getrocknete Matrix lag um 60 g/m² und wurde mit einer Schutzfolie, z.B. aus Polyethylenterephthalat (PET) in einer Dicke von 19 µm oder aus Polyethylen-Aluminium-Polyester, kaschiert.

### BEISPIEL 1 b: Monolayer (Herstellung der Testformulierungen am Beispiel von IMPD 631 ROTTDS)

| Rohstoff | Zusammensetzung der getrockneter Matrix [%] | Zusammensetzung der nassen Matrix [g/m²] |
|---|---|---|
| Rotigotin | 7,50 | 3,75 |
| PVP K90 | 5,83 | 2,92 |
| Ethanol | --- | 16,53 |
| Natriummetabisulfit | 0,0001 | 0,0005 |
| Wasser, dest. | --- | 0,0495 |
| Ascorbylpalmitat | 0,05 | 0,03 |
| Tocopherol | 0,05 | 0,03 |
| Paraffin, dickflüssig | 2,10 | 1,05 |
| BIO-PSA^{®} SRS7-4501 (z.B. 68 % Feststoffgehalt) | 21,12 | 15,51 |
| BIO-PSA^{®} SRS7-4601 (z.B. 65 % Feststoffgehalt) | 63,35 | 48,99 |
| n-Heptan | --- | 0,45 |
| Summe | 100,0 | 89,3 |

Eine wässrige 1% (Gew.%)-Natriummetabisulfit-Lösung wurde hergestellt. Eine ethanolische 25% (Gew.%)-PVP K90-Lösung wurde hergestellt. Die entsprechende Menge an Natriummetabisulfit-Lösung wurde zur entsprechenden Menge an PVP-Lösung in ein geeignetes Glasgefäß gewogen und für ca. 30 min gerührt. Ascorbylpalmitat und Tocopherol wurden zugegeben und verrührt. Dann wurde der Wirkstoff Rotigotin unter Rühren und Erhitzung im Wasserbad langsam zugegeben und bei ungefähr 60°C gerührt bis zur vollständigen Lösung. Nachdem die Masse wieder abgekühlt war, wurden die Silikonkleber nacheinander hinzugewogen und kurz verrührt. Dann wurde das Ausgleichslösemittel Heptan zugegeben und verrührt. Zum Schluss wurde Paraffin hinzugewogen. Die Beschichtungsmasse wurde gerührt bis alles visuell homogen verteilt war. Im Anschluss wurde die Beschichtungsmasse für z.B. ungefähr 3 Minuten bei ca. 10000 rpm mit einem geeigneten Dispergiergerät (Ultra-Turrax) bearbeitet. Die so homogenisierte Beschichtungsmasse wurde auf einer fluorbeschichteten Folie, z.B. Scotchpak^{™} 9709, als dünner Film ausgestrichen und anschließend in einem Trockentunnel von ca. 52 cm Länge mit 4 separaten Sektionen bei je ca. 45, 60, 80, 99°C mit einer Geschwindigkeit von ca. 0,16 m/min erwärmt, so dass die Lösemittel nahezu vollständig entfernt wurden. Die getrocknete Matrix lag um 50 g/m² und wurde mit einer Schutzfolie, z.B. aus Polyethylenterephthalat (PET) in einer Dicke von 19 µm, kaschiert.

### BEISPIEL 2 a: Bilayer (Herstellung der Testformulierungen am Beispiel von 618_617ROTTDS)

| Rohstoff | Zusammensetzung der getrockneter Matrix [%] | Zusammensetzung der nassen Matrix [g/m²] |
|---|---|---|
| Matrixschicht | | |
| Rotigotin | 9,00 | 4,50 |
| PVP K90 | 7,00 | 3,50 |
| Ethanol | --- | 19,83 |
| Natriummetabisulfit | 0,0021 | 0,0011 |
| Wasser, dest. | --- | 0,1040 |
| Ascorbylpalmitat | 0,02 | 0,01 |
| Tocopherol | 0,05 | 0,03 |
| BIO-PSA^{®} SRS7-4501 (z.B. 66 % Feststoffgehalt) | 83,93 | 63,63 |
| n-Heptan | --- | 0,99 |
| Summe | 100,0 | 92,6 |

| Initial wirkstofffreie Haftklebeschicht | | |
|---|---|---|
| Paraffin, dickflüssig | 2,40 | 0,66 |
| BIO-PSA^{®} SRS7-4501 (z.B. 66 % Feststoffgehalt) | 24,40 | 10,17 |
| BIO-PSA^{®} SRS7-4601 (z.B. 63 % Feststoffgehalt) | 73,20 | 32,03 |
| n-Heptan | --- | 2,97 |
| Summe | 100,0 | 45,8 |

Zunächst wurde die Matrixschicht (2) hergestellt. Hierzu wurde eine wässrige 1% (Gew.%)-Natriummetabisulfit-Lösung sowie eine ethanolische 25% (Gew.%)-PVP K90-Lösung hergestellt.

Die entsprechende Menge an Natriummetabisulfit-Lösung wurde zur entsprechenden Menge an PVP-Lösung in ein geeignetes Glasgefäß gewogen und für mindestens 15 min gerührt. Ascorbylpalmitat und Tocopherol wurden zugegeben und verrührt. Dann wurde der Wirkstoff Rotigotin unter Rühren und Erhitzung im Wasserbad langsam zugegeben und bei ungefähr 60°C gerührt bis zur vollständigen Lösung. Nachdem die Masse wieder abgekühlt war, wurden die Silikonkleber nacheinander hinzugewogen und kurz verrührt. Dann wurde das Ausgleichslösemittel Heptan zugegeben und verrührt. Die Beschichtungsmasse wurde gerührt bis alles visuell homogen verteilt war. Im Anschluss wird Beschichtungsmasse auf einer fluorbeschichteten Folie, z.B. Scotchpak^{™} 9709 / 1022 / 9744, als dünner Film ausgestrichen und anschließend für z.B. 10 min bei 85 °C erwärmt, so dass die Lösemittel nahezu vollständig entfernt wurden. Die getrocknete Matrix lag bei ca. 50 g/m² und wurde mit einer Schutzfolie, z.B. Polyethylenterephthalat (PET) in einer Dicke von 19 µm, kaschiert.

Für die Herstellung der initial wirkstofffreien Haftklebeschicht (3) wurden die Silikonkleber in ein geeignetes Glasgefäß abgewogen und kurz verrührt. Dann wurden nacheinander Paraffin und das Ausgleichslösemittel Heptan hinzugewogen und verrührt. Die Beschichtungsmasse wurde gerührt bis alles visuell homogen erschien. Im Anschluss wurde die Beschichtungsmasse auf einer fluorbeschichteten Folie, z.B. Scotchpak^{™} 9709 / 1022 / 9744, als dünner Film ausgestrichen und anschließend z.B. bei 85°C für 10 min erwärmt, so dass die Lösemittel nahezu vollständig entfernt wurden. Die getrocknete initial wirkstofffreie Haftklebeschicht (3) besaß ein Matrixgewicht im Bereich von 25 - 30 g/m³ und wurde mit der Matrixschicht, unter Abzug ihres Release Liners, auf die offene Matrix der initial wirkstofffreien Haftklebeschicht (3) kaschiert.

### BEISPIEL 2 b: Bilayer (Herstellung der Testformulierungen am Beispiel von 629_628ROTTDS)

| Rohstoff | Zusammensetzung der getrockneter Matrix [%] | Zusammensetzung der nassen Matrix [g/m²] |
|---|---|---|
| Matrixschicht | | |
| Rotigotin | 9,00 | 4,50 |
| PVP K90 | 7,00 | 3,50 |
| Ethanol | --- | 19,83 |
| Natriummetabisulfit | 0,0021 | 0,0011 |
| Wasser, dest. | --- | 0,1040 |
| Ascorbylpalmitat | 0,10 | 0,05 |
| Tocopherol | 0,05 | 0,03 |
| BIO-PSA^{®} SRS7-4501 (z.B. 67 % Feststoffgehalt) | 83,85 | 62,57 |
| n-Heptan | --- | 0,32 |
| Summe | 100,0 | 90,9 |
| Initial wirkstofffreie Haftklebeschicht | | |
| Paraffin, dickflüssig | 2,40 | 0,66 |
| BIO-PSA^{®} SRS7-4501 (z.B. 68 % Feststoffgehalt) | 24,40 | 9,85 |
| BIO-PSA^{®} SRS7-4601 (z.B. 65 % Feststoffgehalt) | 73,20 | 31,20 |
| n-Heptan | --- | 0,59 |
| Summe | 100,0 | 42,3 |

Zunächst wurde die Matrixschicht (2) hergestellt. Hierzu wurde eine wässrige 1% (Gew.%)-Natriummetabisulfit-Lösung sowie eine ethanolische 25% (Gew.%)-PVP K90-Lösung hergestellt. Die entsprechende Menge an Natriummetabisulfit-Lösung wurde zur entsprechenden Menge an PVP-Lösung in ein geeignetes Glasgefäß gewogen und für ca. 30 min gerührt. Ascorbylpalmitat und Tocopherol wurden zugegeben und verrührt. Dann wurde der Wirkstoff Rotigotin unter Rühren und Erhitzung im Wasserbad langsam zugegeben und bei ungefähr 60°C gerührt bis zur vollständigen Lösung. Nachdem die Masse wieder abgekühlt war, wurden die Silikonkleber nacheinander hinzugewogen und kurz verrührt. Dann wurde das Ausgleichslösemittel Heptan zugegeben und verrührt. Die Beschichtungsmasse wurde gerührt bis alles visuell homogen verteilt war. Im Anschluss wurde die Beschichtungsmasse auf einer fluorbeschichteten Folie, z.B. Scotchpak^{™} 9709, als dünner Film ausgestrichen und anschließend in einem Trockentunnel von ca. 52 cm Länge mit 4 Sektionen, bei je ca. 45-60-80-99°C mit einer Geschwindigkeit von 0,16 m/min erwärmt, so dass die Lösemittel nahezu vollständig entfernt wurden. Die getrocknete Matrix lag bei ca. 50 g/m² und wurde mit einer Schutzfolie, z.B. Polyethylenterephthalat (PET) in einer Dicke von 19 µm, kaschiert.

Für die Herstellung der initial wirkstofffreien Haftklebeschicht (3) wurden die Silikonkleber in ein geeignetes Glasgefäß abgewogen und kurz verrührt. Dann wurden nacheinander Paraffin und das Ausgleichslösemittel Heptan hinzugewogen und verrührt. Die Beschichtungsmasse wurde gerührt bis alles visuell homogen erschien. Im Anschluss wurde die Beschichtungsmasse auf einer fluorbeschichteten Folie, z.B. Scotchpak^{™} 9709, als dünner Film ausgestrichen und anschließend z.B. bei 80°C für 5 min erwärmt, so dass die Lösemittel nahezu vollständig entfernt wurden. Die getrocknete initial wirkstofffreie Haftklebeschicht (3) besaß ein Matrixgewicht zwischen ca. 25 - 30 g/m² und wurde mit der Matrixschicht, unter Abzug ihres Release Liners, auf die offene Matrix der initial wirkstofffreien Haftklebeschicht (3) kaschiert.
- FG [g/m²]:: Flächengewicht
- PVP K90:: Polyvinylpyrrolidon K-90 (BASF SE)
- rF (oder rh):: relative Luftfeuchtigkeit
- RSD:: relative Standardabweichung
- RS:: Matrixschicht
- HS:: initial wirkstofffreie Haftschicht
- mon:: Monate
- RT:: Raumtemperatur

### Die so hergestellten transdermalen therapeutischen Systeme haben beispielsweise die folgende allgemeine Zusammensetzung:

Monolayer-Formulierungen mit wirkstoffhaltiger Matrixschicht, umfassend Rotigotin und PVP K90 in bestimmten Gewichtsverhältnissen und Gewichtsanteilen bezogen auf das Gesamtgewicht der Matrixschicht; 0-3 Gew.-% dickflüssiges Paraffin; verschiedene Kombinationen von einem oder zwei nicht-silanolreduzierten oder silanolreduzierten nicht aminresistenten Silikonklebern (Dow Corning^{®}); mindestens ein Antioxidant, z.B. Tocopherol 0,05-0,1 Gew.-%, Ascorbylpalmitat 0,02-0,1 Gew.-% und Na-Metabisulfit 0,0006-0,0021 Gew.-%; und ein Matrixgewicht von ca. 50-60 g/m2.

Bilayer-Formulierungen mit wirkstoffhaltiger Matrixschicht, umfassend Rotigotin und PVP K90 in bestimmten Gewichtsverhältnissen und Gewichtsanteilen bezogen auf das Gesamtgewicht der Matrixschicht; nicht- silanolreduzierte oder silanolreduzierte nicht aminresistente Silikonkleber BIO-PSA 7-4501 oder BIO-PSA SRS7-4501 (Dow Corning^{®}); mindestens ein Antioxidant, z.B. Tocopherol 0,05-0,1 Gew.-%, und/oder Ascorbylpalmitat 0,02-0,1 Gew.-% und 0,0006-0,0021 Gew.-% Na-Metabisulfit; einem Matrixgewicht von ca. 2550 g/m²; und einer zusätzlichen initial wirkstofffreien Haftkleberschicht (3) umfassend verschiedene Kombinationen von einem oder zwei nicht-silanolfreien nicht aminresistenten Silikonklebern (Dow Corning^{®}) und 0-3 Gew.-% dickflüssigem Paraffin.

### Die folgenden spezifischen Monolayer- und Bilayer-Formulierungen wurden hergestellt:

### A) Monolayer-Formulierungen mit unterschiedlichen Mischungsverhältnissen an nicht aminresistenten Silikonklebern ohne Paraffin

Es wurden verschiedene Monolayer-Formulierungen ohne Paraffin mit unterschiedlichen Mischungsverhältnissen von BIO-PSA SRS7-4501 zu BIO-PSA SRS7-4601 und BIO-PSA 7-4501 zu BIO-PSA 7-4601 in der Matrixschicht hergestellt. Für diese Monolayer-Formulierungen wurden verschiedene Kombinationen der Rückschichten ("Backing Layer") Scotchpak 1109 (3M Corporation) und Hostaphan^{®} MN19 (Mitsubishi Polyester Film) und der Schutzfolien ("Release Liner") Scotchpak 1022, Scotchpak 9744 und Scotchpak 9709 (3M Corporation) verwendet. Die resultierenden TTS wurden bei 25 °C und 40 °C über verschiedene Zeiträume gelagert und die Trennkraft, Klebkraft und Klebrigkeit (Tack) wurden untersucht. Zusätzlich wurden von einigen Formulierungen die *in vitro* Permeation auf humaner hitzeseparierter Epidermis (HSE: heat separated epidermis) bestimmt.

Die Trennkraft ("separation force") kann bestimmt werden als die Kraft, die erforderlich ist, um eine Probe in einem spezifizierten Winkel und bei definierter Geschwindigkeit von ihrem Release Liner abzulösen. Für ihre Bestimmung werden TDS einer definierten Größe, z.B. 10 cm², ausgestanzt und bei 23 ± 1°C und 50 ± 5% rF konditioniert. Es wird ein Leitstreifen, in der Breite des TDS, an dem TDS angebracht. Dann wird das TDS mit dem Release Liner nach unten auf einem Geräteschlitten mit Hilfe eines doppelseitigen Klebebandes fixiert. Dieser wird so in eine Zugprüfmaschine, z.B. Texture Analyser plus von Stable Micro Systems, eingebracht, dass das TDS in einem Winkel von 90° abgezogen wird. Die Geschwindigkeit liegt üblicherweise bei 300 ± 30 mm/min und erfolgt in der Regel bei 23 ± 1°C und 50 ± 5% rF. Die mittlere, auf eine Probenbreite von 25 mm normierte Kraft [N/25 mm], die über die Trennstrecke gemessen wird, ist die Trennkraft.

Die Klebkraft ("adhesion strength") kann bestimmt werden als die Kraft, die erforderlich ist, um eine Probe in einem spezifizierten Winkel und bei definierter Geschwindigkeit von einem geeigneten Träger zu lösen. Für die Bestimmung der Klebkraft werden TDS einer definierten Größe, z.B. 10 cm², ausgestanzt und bei 23 ± 1°C und 50 ± 5% rF konditioniert. Es wird ein Leitstreifen, z.B. aus doppelseitigem Klebeband, in der Breite des TDS angebracht. Das TDS wird unter Abzug seines Release Liners auf eine Prüfplatte, z.B. aus Stahl, aufgeklebt und im Anschluss für z.B. 1 min zwischen zwei Glasplatten liegend mit einem 2 kg Gewicht angepresst.

Die Prüfplatte wird waagerecht in einer Zugprüfmaschine, z.B. Texture Analyser plus von Stable Micro Systems, befestigt und der Leitstreifen so eingespannt, dass das TDS in einem 90° Winkel von abgezogen wird. Die Messung erfolgt mit einer spezifizierten Geschwindigkeit von in der Regel 300 ± 30 mm/min bei üblicherweise 23 ± 1°C und 50 ± 5% rF. Die mittlere, auf eine Probenbreite von 25 mm normierte Kraft [N/25 mm], die über die Strecke gemessen wird, ist die Klebkraft.

Tack kann bestimmt werden als die maximale Kraft, die erforderlich ist, um einen Edelstahlprüfkörper vollständig von der Adhäsivschicht eines TDS zu trennen. Für die Bestimmung des Tack wird ein Pflaster oder Laminat bei 23 ± 1°C und 50 ± 5% rF konditioniert und im Anschluss unter Abzug seines Release Liner mit der offenen Adhäsivmatrix auf einer gelochten Trägerplatte fixiert. Die Platte wird in einer Zugprüfmaschine, z.B. Texture Analyser plus von Stable Micro Systems, befestigt. Bei üblicherweise 23 ± 1°C und 50 ± 5% rF wird der Prüfkörper auf die Probenoberseite gedrückt und nach einer definierten Kontaktzeit von in der Regel 2 sec abgezogen. Eine Messreihe sollte nach Abziehen des Release Liners von der ersten Probe innerhalb von 30 min abgeschlossen sein. Die Maximalkraft (Tack; [N]) zum Trennen der Bindung zwischen Prüfkörper und Adhäsivschicht wird ermittelt.

**Tabelle 1: Monolayer-Formulierungen ohne Paraffin**

| Charge | Zusammensetzung der Matrix | Polymerverhältnis | FG [g/m²] | Release Liner / Backing Layer |
|---|---|---|---|---|
| Batches mit silanolreduzierten (SRS) nicht aminresistenen Silikonklebern BIO-PSA SRS7-4501 & SRS7-4601 | | | | |
| 538ROTTDS | Rotigotin 9%, PVP K90 7%, Tocopherol 0,05%, BIO-PSA SRS7-4501 41,975%, BIO-PSA SRS7-4601 41,975% | 1:1 | 50 | Scotchpak 1022, Scotchpak 1109 |
| 536ROTDS | Rotigotin 9%, PVP K90 7%, Tocopherol 0,05%, BIO-PSA SRS7-4501 33,58%, BIO-PSA SRS7-4601 50,37% | 1:1,5 | 50 | Scotchpak 1022, Scotchpak 1109 |
| 537ROTDS | Rotigotin 9%, PVP K90 7%, Tocopherol 0,05%, BIO-PSA SRS7-4501 27,98%, BIO-PSA SRS7-4601 55,97% | 1:2 | 50 | Scotchpak 9744, Scotchpak 1109 |
| 539ROTTDS | Rotigotin 9%, PVP K90 7%, Tocopherol 0,05%, BIO-PSA SRS7-4501 20,99%, BIO-PSA SRS7-4601 62,96% | 1:3 | 50 | Scotchpak 1022, Scotchpak 1109 |
| 534ROTTDS | Rotigotin 0%, PVP K90 7,7%, Tocopherol 0,05%, BIO-PSA SRS7-4501 46,13%, BIO-PSA SRS7-4601 46,13% | 1:1 | 45,5 | Scotchpak 9744, Scotchpak 1109 |
| Neupro nach EP1524975 | Rotigotin 9%, PVP K90 2%, Tocopherol 0,05%, Ascorbylpalmitat 0,02%, Na-Metabisulfit 0,0006%, BIO-PSA 7-4201 44,465%, BIO-PSA 7-4301 44,465% | 1:1 | 50 | Scotchpak 9744, Scotchpak 1109 |
| Neupro nach EP2515887 | Rotigotin 7,5%, PVP K90 3,33%, Tocopherol 0,05%, Ascorbylpalmitat 0,02%, Na-Metabisulfit 0,0006%, BIO-PSA 7-4201 35,64-44,55%, BIO-PSA 7-4301 44,55 - 53,46% | 1:1 oder 1:2 | 60 | Scotchpak 9744, Scotchpak 1109 |
| 576ROTTDS | Rotigotin 9%, PVP K90 7%, Tocopherol 0,05%, Ascorbylpalmitat 0,02%, Na-Metabisulfit 0,00006% BIO-PSA SRS7-4601 83,929% | 0:1 | 50 | Scotchpak 9709, Scotchpak 1109 |
| 564ROTTDS | Rotigotin 9%, PVP K90 7%, Tocopherol 0,05%, Ascorbylpalmitat 0,02%, Na-Metabisulfit 0,00006% BIO-PSA SRS7-4601 83,929% | 0:1 | 50 | Eastmen, Scotchpak 1109 |
| 588ROTTDS | Rotigotin 9%, PVP K90 7%, Tocopherol 0,05%, Ascorbylpalmitat 0,02%, Na-Metabisulfit 0,00021% BIO-PSA SRS7-4501 20,982%, BIO-PSA SRS7-4601 62,946% | 1:3 | 50 | Scotchpak 9709, Scotchpak 1109 |
| 550ROTTDS | Rotigotin 9%, PVP K90 7%, Tocopherol 0,05%, Ascorbylpalmitat 0,02%, Na-Metabisulfit 0,00006% BIO-PSA SRS7-4501 41,965%, BIO-PSA SRS7-4601 41,965% | 1:1 | 50 | Scotchpak 9709, Scotchpak 1109 |
| 589ROTTDS | Rotigotin 9%, PVP K90 7%, Tocopherol 0,05%, Ascorbylpalmitat 0,02%, Na-Metabisulfit 0,00021% BIO-PSA SRS7-4501 62,946%, BIO-PSA SRS7-4601 20,982% | 3:1 | 50 | Scotchpak 9709, Scotchpak 1109 |
| 577ROTTDS | Rotigotin 9%, PVP K90 7%, Tocopherol 0,05%, Ascorbylpalmitat 0,02%, Na-Metabisulfit 0,00021% BIO-PSA SRS7-4501 83,928% | 1:0 | 50 | Scotchpak 9709, Scotchpak 1109 |

| Batches mit nicht silanolreduzierten nicht aminresistenen Silikonklebern BIO-PSA 7-4501 & 7-4601 | | | | |
|---|---|---|---|---|
| 583ROTTDS | Rotigotin 9%, PVP K90 7%, Tocopherol 0,05%, Ascorbylpalmitat 0,02%, Na-Metabisulfit 0,00021 % BIO-PSA 7-4601 83,929% | 0:1 | 50 | Scotchpak 9709, Scotchpak 1109 |
| 586ROTTDS | Rotigotin 9%, PVP K90 7%, Tocopherol 0,05%, Ascorbylpalmitat 0,02%, Na-Metabisulfit 0,00021% BIO-PSA SRS7-4501 20,982%, BIO-PSA SRS7-4601 62,946% | 1:3 | 50 | Scotchpak 9709, Scotchpak 1109 |
| 581ROTTDS | Rotigotin 9%, PVP K90 7%, Tocopherol 0,05%, Ascorbylpalmitat 0,02%, Na-Metabisulfit 0,00021% BIO-PSA SRS7-4501 41,964%, BIO-PSA SRS7-4601 41,964% | 1:1 | 50 | Scotchpak 9709, Scotchpak 1109 |
| 587ROTTDS | Rotigotin 9%, PVP K90 7%, Tocopherol 0,05%, Ascorbylpalmitat 0,02%, Na-Metabisulfit 0,00021% BIO-PSA SRS7-4501 62,946%, BIO-PSA SRS7-4601 20,982% | 3:1 | 50 | Scotchpak 9709, Scotchpak 1109 |
| 582ROTTDS | Rotigotin 9%, PVP K90 7%, Tocopherol 0,05%, Ascorbylpalmitat 0,02%, Na-Metabisulfit 0,00021% BIO-PSA SRS7-4501 83,928% | 1:0 | 50 | Scotchpak 9709, Scotchpak 1109 |

### B) Monolayer-Formulierungen mit unterschiedlichen Mischungsverhältnissen nicht aminresistenter Silikonkleber mit Paraffin

Es wurden verschiedene Monolayer-Formulierungen analog zu A) mit einer geringen Menge an Paraffin mit unterschiedlichen Mischungsverhältnissen von BIO-PSA SRS7-4501 zu BIO-PSA SRS7-4601 und BIO-PSA 7-4501 zu BIO-PSA 7-4601 (Dow Corning^{®}) in der Matrixschicht hergestellt. Für diese Monolayer-Formulierungen wurden verschiedene Kombinationen der Rückschichten ("Backing Layer") Scotchpak 1109 (3M Corporation) und Hostaphan^{®} MN19 (Mitsubishi Polyester Film) und der Schutzfolien ("Release Liner") Scotchpak 9744 und Scotchpak 9709 (3M Corporation) verwendet. Die resultierenden TTS wurden bei 25 °C und 40 °C über verschiedene Zeiträume gelagert und die Trennkraft, Klebkraft und Klebrigkeit (Tack) wurden untersucht. Zusätzlich wurden von einigen Formulierungen die *in vitro* Permeation auf humaner hitzeseparierter Epidermis (HSE) bestimmt.

**Tabelle 2: Monolayer-Formulierungen mit Paraffin**

| Charge | Zusammensetzung der Matrix | Polymerverhältnis | FG [g/m²] | Release Liner / Backing Layer |
|---|---|---|---|---|
| Batches mit silanolreduzierten (SRS) nicht aminresistenen Silikonklebern BIO-PSA SRS7-4501 & SRS7-4601 oder nicht silanolreduzierten nicht aminresistenen Silikonklebern BIO-PSA 7-4501 & 7-4601 mit einem geringen Zusatz an dickflüssigem Paraffin | | | | |
| 574ROTTDS | Rotigotin 9%, PVP K90 7%, Tocopherol 0,05%, Ascorbylpalmitat 0,02% Na-Metabisulfit 0,00006%, Paraffin 2%, BIO-PSA SRS7-4501 82 % | 0:1 | 50 | Scotchpak 9744, Scotchpak 1109 |
| 599ROTTDS | Rotigotin 9%, PVP K90 7%, Tocopherol 0,05%, Ascorbylpalmitat 0,02%, Na-Metabisulfit 0,00021 %, Paraffin 1 %, BIO-PSA SRS7-4501 82,93% | 0:1 | 50 | Scotchpak 9709, Scotchpak 1109 |
| 611ROTTDS | Rotigotin 9%, PVP K90 7%, Tocopherol 0,1% Na-Metabisulfit 0,0021%, Paraffin 2%, BIO-PSA 7-4501 20,474%, BIO-PSA 7-4601 61,423% | 1:3 | 50 | Scotchpak 9709, Hostapahn MN19 |
| 602ROTTDS | Rotigotin 9%, PVP K90 7%, Tocopherol 0,1%, Na-Metabisulfit 0,0021%, Paraffin 1%, BIO-PSA 7-4501 20,724%, BIO-PSA 7-4601 62,173% | 1:3 | 50 | Scotchpak 9709 Hostaphan MN19 |
| 614ROTTDS | Rotigotin 9%, PVP K90 7%, Tocopherol 0,1%, Na-Metabisulfit 0,0021%, Paraffin 2%, BIO-PSA 7-4601 81,8979% | 0:1 | 50 | Scotchpak 9709 Hostaphan MN19 |
| 616ROTTDS | Rotigotin 7,5%, PVP K90 5,83%, Tocopherol 0,05%, Na-Metabisulfit 0,0021%, Ascorbylpalmitat 0,02%, Paraffin 2,1%, BIO-PSA SRS7-4501 21,12%, BIO-PSA SRS7-4601 63,37% | 1:3 | 60 | Scotchpak 9709 Hostaphan MN19 |
| 616ROTTDS | Rotigotin 7,5%, PVP K90 5,83%, Tocopherol 0,05%, Na-Metabisulfit 0,0021%, Ascorbylpalmitat 0,02%, Paraffin 2,1%, BIO-PSA SRS7-4501 21,12%, BIO-PSA SRS7-4601 63,37% | 1:3 | 50 | Scotchpak 9709 Hostaphan MN19 |

### C) Bilayer-Formulierungen mit unterschiedlichen Mischungsverhältnissen an nicht aminresistenten Silikonklebern mit und ohne Paraffin

Es wurden verschiedene Bilayer-Formulierungen mit unterschiedlichen Mengen an Rotigotin und BIO-PSA SRS7-4501 oder BIO-PSA 7-4501 (Dow Corning^{®}) in der Matrixschicht hergestellt. Die Bilayer-Formulierungen wiesen zusätzlich, auf der Seite der Matrixschicht die der Seite, welche mit der Rückschicht in Kontakt ist, gegenüber liegt, eine zusätzliche initial wirkstofffreie Haftkleberschicht ("adhesive layer") auf. Die zusätzliche initial wirkstofffreie Haftkleberschicht wurde mit oder ohne einer geringen Menge an Paraffin und mit Mengen von BIO-PSA SRS7-4501 und/oder BIO-PSA SRS7-4601 oder BIO-PSA 7-4501 (Dow Corning^{®}) formuliert.

Das Verhältnis von Silikonkleber mit mittlerer Klebrigkeit und Silikonkleber mit hoher Klebrigkeit war dabei so zu wählen, dass sowohl eine gute Klebkraft und Klebrigkeit (Tack) als auch möglichst geringer Cold Flow (kalter Fluss) und ausreichend hohe Kohäsion resultierte.

Es wurden verschiedene Kombinationen der Rückschichten ("Backing Layer") Scotchpak 1109 (3M Corporation) und Hostaphan^{®} MN19 (Mitsubishi Polyester Film) und der Schutzfolien ("Release Liner") Scotchpak 9744, Scotchpak 1022 und Scotchpak 9709 (3M Corporation) und Primeliner 100 µm 78BT, Primeliner 75 µm 78HL (Loparex International B.V.) verwendet. Die resultierenden TTS wurden bei 25 °C und 40 °C über verschiedene Zeiträume hinweg gelagert und die Trennkraft, Klebkraft und Klebrigkeit (Tack) wurden untersucht. Zusätzlich wurden von einigen Formulierungen die *in vitro* Permeation auf hitzeseparierter Epidermis (HSE) von Humanhaut bestimmt.

Bei den Bilayer-Formulierungen sollte der Einfluss einer zusätzlichen initial wirkstofffreien Haftkleberschicht auf Trennkraft, Klebkraft, Klebrigkeit (Tack) und *in vitro* Permeation untersucht werden.

**Tabelle 3: Bilayer-Formulierungen mit und ohne Paraffin**

| Charge | Zusammensetzung der Matrixschicht | Zusammensetzung der zusätzlichen initial wirkstofffreien Haftkleberschicht | FG Matrix / zusätzliche wirkstofffreie Haftklebersc hicht [g/m²] | Release Liner / Backing Layer |
|---|---|---|---|---|
| 569_568RO TTDS | Rotigotin 15%, PVP K90 8,5%, BIO-PSA SRS7-4501 76,5% | Duro-Tak 87-4098 100% | 30/30 | Primeliner^{™} 100µm 78BT / Scotchpak 1109 |
| 570_568RO TTDS | Rotigotin 15%, PVP K90 8,5%, BIO-PSA SRS7-4501 76,5% | Duro-Tak 87-626A 53,8%, Duro-Tak 87-625A 23,1%, Indopol H-1900 23,1% | 30/30 | Primeliner^{™} 100µm 78BT / Scotchpak 1109 |
| 571_568RO TTDS | Rotigotin 15%, PVP K90 8,5%, BIO-PSA SRS7-4501 76,5% | BIO-PSA SRS7-4601 100% | 30/30 | Scotchpak 9744, Scotchpak 1109 |
| 572_568RO TTDS | Rotigotin 15%, PVP K90 8,5%, BIO-PSA SRS7-4501 76,5 | SBS 45%, Paraffin 20%, Saturated Alicyclic Resin 35% | 30/30 | Primeliner^{™} 100µm 78BT / Scotchpak 1109 |
| 592_591 RO TTDS | Rotigotin 9%, PVP K90 7%, Tocopherol 0,05%, Na-Metabisulfit 0,00021%, Ascorbylpalmitat 0,02%, BIO-PSA SRS7-4501 83,928% | BIO-PSA SRS7-4601 100% | 50/30 | Scotchpak 9709, Scotchpak 1109 |
| 593_590RO TTDS | Rotigotin 9%, PVP K90 7%, Tocopherol 0,05%, Na-Metabisulfit 0,00021%, Ascorbylpalmitat 0,02%, BIO-PSA 7-4501 83,928% | BIO-PSA 7-4601 100% | 50 / 30 | Scotch pak 9709, Hostaphan MN15 |
| 612_613RO TTDS | Rotigotin 11,25%, PVP K90 8,75%, Tocopherol 0,1%, Na-Metabisulfit 0,0021%, BIO-PSA SRS7-4501 79,898% | BIO-PSA SRS7-4601 100% | 40 / 27,5 | Scotchpak 9709 Hostaphan MN19 |
| 618_617RO TTDS | Rotigotin 9%, PVP K90 7%, Tocopherol 0,05%, Na-Metabisulfit 0,0021%, Ascorbylpalmitat 0,02%, BIO-PSA SRS7-4501 83,93% | BIO-PSA SRS7-4501 24,4%, BIO-PSA SRS7-4601 73,2%, Paraffin 2,4% | 50 / 27,5 | Scotchpak 9709 Hostaphan MN19 |

### D) Monolayer-Formulierungen mit nicht aminresistenten Silikonklebern und unterschiedlichen Konzentrationen an Polyvinylpyrrolidon

Es wurden verschiedene Monolayer-Formulierungen mit unterschiedlichen Mengen an PVP K90 bei einer festen Menge von 9 Gew.-% Rotigotin, bezogen auf das Gesamtgewicht der Matrixschicht, und BIO-PSA SRS7-4501 zu BIO-PSA SRS7-4601 (Dow Corning^{®}) in einem Mischungsverhältnis von 1:1 in der Matrixschicht hergestellt.

Es wurden die Rückschicht ("Backing Layer") Scotchpak 1109 (3M Corporation) und die Schutzfolie ("Release Liner") Scotchpak 9744 verwendet. Die resultierenden TTS wurden bei 25 °C und 40 °C über verschiedene Zeiträume hinweg gelagert und das Aussehen, wurde untersucht. Zusätzlich wurden von einigen Formulierungen die *in vitro* Permeation auf humaner hitzeseparierter Epidermis (HSE) bestimmt. Ziel war es den Einfluss der Menge an PVP K90 auf die Rekristallisierung von Rotigotin und die *in vitro* Permeation zu untersuchen.

**Tabelle 4: Monolayer-Formulierungen mit unterschiedlichen Mengen an PVP K90 bei gleichbleibender Menge von 9 Gew.-% Rotigotin.**

| Charge | Zusammensetzung der Matrixschicht | Polymerverhältnis | FG [g/m²] | Release Liner / Backing Layer |
|---|---|---|---|---|
| 506ROTTDS | Rotigotin 9%, PVP K90 3,2%, Tocopherol 0,05%, BIO-PSA SRS7-4501 43,875%, BIO-PSA SRS7-4601 43,875% | 1:1 | 50 | Scotchpak 9744, Scotchpak 1109 |
| 525ROTTDS | Rotigotin 9%, PVP K90 5%, Tocopherol 0,05% BIO-PSA SRS7-4501 42,975%, BIO-PSA SRS7-4601 42,975% | 1:1 | 50 | Scotchpak 9744, Scotchpak 1109 |
| 526ROTTDS | Rotigotin 9%, PVP K90 6%, Tocopherol 0,05%, BIO-PSA SRS7-4501 42,475%, BIO-PSA SRS7-460142,475% | 1:1 | 50 | Scotchpak 9744, Scotchpak 1109 |
| 511ROTTDS | Rotigotin 9%, PVP K90 7%, Tocopherol 0,05%, BIO-PSA SRS7-4501 41,975%, BIO-PSA SRS7-4601 41,975% | 1:1 | 50 | Scotchpak 9744, Scotchpak 1109 |
| 513ROTTDS | Rotigotin 9%, PVP K90 8%, Tocopherol 0,05%, BIO-PSA SRS7-4501 41,475%, BIO-PSA SRS7-4601 41,475% | 1:1 | 50 | Scotchpak 9744, Scotchpak 1109 |
| 554ROTTDS | Rotigotin 9%, PVP K90 9%, Tocopherol 0,05%, BIO-PSA SRS7-4501 40,975%, BIO-PSA SRS7-4601 40,975% | 1:1 | 50 | Scotchpak 9744, Scotchpak 1109 |

### BEISPIEL 2 (Trennkraft, Klebkraft und Klebrigkeit)

Ziel war es den Einfluss der Mischungsverhältnisse der nicht aminresistenten Silikonkleber BIO-PSA SRS7-4501 zu BIO-PSA SRS7-4601 und BIO-PSA 7-4501 zu BIO-PSA 7-4601 (Dow Corning^{®}) auf Trennkraft, Klebkraft und Klebrigkeit (Tack) zu untersuchen, um dadurch ein optimales Mischungsverhältnis für die Matrixschicht festlegen zu können. Ziel war es außerdem den Einfluss von Paraffin auf Trennkraft, Klebkraft und Klebrigkeit (Tack) zu untersuchen.

### A) Trennkraft (Separation Force)

Wie in Tabelle 5 und Abbildung 2 deutlich zu erkennen ist, führten TTS-Formulierungen mit Rotigotin in Kombination mit einem oder mehreren Silikonklebern mit hauptsächlich freien Silanolgruppen (nicht aminresistente Silikonkleber) in der Matrixschicht von Monolayer-Formulierungen und einer mit Fluoropolymer beschichteten Schutzfolie, wie z.B Scotchpak 1022 oder Scotchpak 9744 (3M Corporation) bei Lagerung zu einem großen Anstieg der Trennkraft.

Bei der Placebo Formulierung ohne Rotigotin wurde bei Nutzung von Silikonklebern mit noch freien Silanolgruppen und denselben Schutzfolientypen kein Anstieg der Trennkraft beobachtet.

Eine mit Fluorsilikon beschichtet Schutzfolie wie z.B. Scotchpak 9709 (3M Corporation) zeigte bei derselben oder ähnlichen Formulierungen einen deutlich geringeren Anstieg der Trennkraft bei Verwendung von Rotigotin und einem oder mehreren Silikonklebern mit noch freien Silanolgruppen (vgl. Abb. 2 und Tabelle 5).

**Tabelle 5: Trennkraft von Silikonkleberformulierungen mit nicht silanolreduzierten Silikonklebern und mit silanolreduzierten nicht aminresistenten Silikonklebern in verschieden Polymerkleber-Mischungsverhältnissen mit oder ohne Paraffin bei Lagerung über 0-3 Monaten bei 40 °C/ 75% rF.**

| Batch / Release Liner | Trennkraft nach 0 mon | Trennkraft nach 1 mon bei 40°C/75% rF (rh) | Trennkraft nach 3 mon bei 40°C/75% rF (rh) | Trennkraft nach 4 mon bei RT | Trennkraft nach 75 mon bei RT |
|---|---|---|---|---|---|
| 538ROTTDS / Scotchpak^{™} 1022 | 0.15 (48%RSD) | 3.58 (16%RSD) | 9.18 (8%RSD) | ./. | ./. |
| 537ROTTDS / Scotchpak^{™} 9744 | 0.10 (36%RSD) | 2.56 (8%RSD) | 6.51 (8%RSD) | ./. | ./. |
| 564ROTTDS / Eastmen Release Liner | 0.74 (8%RSD) | 3.3 (12%RSD) | 4.2 (8%RSD) | ./. | ./. |
| 576ROTTDS / Scotchpak^{™} 9709 | 0.09 (35%RSD) | 170 (2%RSD) | 3.65 11%RSD) | ./. | ./. |
| Neupro^{®} lot: 56659203 / Scotchpak^{™} 9744 | 0.125 (29%RSD) | ./. | 0.389 (27%RSD) | ./. | ./. |
| 534ROTTDS / Scotchpak^{™} 9744 | ./. | 0.05 (54%RSD) | 0.08 (34%RSD) | 0.16 (54%RSD) | 0.07 (40%RSD) |
| 550ROTTDS / Scotchpak^{™} 9709 | 0.05 (42%RSD) | 0.31 (37%RSD) | 0.44 (14%RSD) | ./. | ./. |
| 588ROTTDS / Scotchpak^{™} 9709 | 0.06 (9%RSD) | 0.30 (17%RSD) | 0.79 (25%RSD) (2.5 mon) | ./. | ./. |
| 592_591ROTTDS / Scotchpak^{™} 9709 | 0.09 (31%RSD) | 0.25 (24%RSD) | 0.25 (9%RSD) 2.5 mon | ./. | ./. |
| 618_617ROTTDS / Scotchpak^{™} 9709 | 0.07 (54%RSD) | 0.14 (31 %RSD) | ./. | ./. | ./. |
| 599ROTTDS / Scotchpak^{™} 9709 | 0.05 (15%RSD) | 0.09 (29%RSD) | 0.14 (16%RSD) | ./. | ./. |
| 602ROTTDS / Scotchpak^{™} 9709 | 0.07 (58%RSD) | 0.20 (28%RSD) | 0.39 (23%RSD) | ./. | ./. |
| 611ROTTDS / Scotchpak^{™} 9709 | 0.24 (39%RSD) | 0.37 (22%RSD) | 0.46 (34%RSD) | ./. | ./. |
| 614ROTTDS / Scotchpak^{™} 9709 | 0.62 (9%RSD) | 0.45 (11%RSD) | ./. | ./. | ./. |
| 616ROTTDS / Scotchpak^{™} 9709 | 0.15 (10%RSD) | ./. | ./. | ./. | ./. |

Auch wiesen überraschenderweise Formulierungen mit Rotigotin und Mischungen der silanolreduzierten nicht aminresistenten Silikonkleber BIO-PSA SRS7-4501 und BIO-PSA SRS7-4601 (Dow Corning^{®}) im Gegensatz zu Rotigotin mit dem silanolreduzierten nicht aminresistenten Silikonkleber BIO-PSA SRS7-4601 (Dow Corning^{®}) alleine einen akzeptablen Trennkraftanstieg auf.

### B) Klebkraft

Außerdem wurde die Klebkraft von verschiedenen TTS-Formulierungen umfassend Rotigotin und unterschiedliche nicht-aminreistenten Silikonkleber und -klebergemische mit einer Placebo-Formulierung und dem auf dem Markt erhältlichen Neupro^{®} TTS, nach Lagerung über 0 bis 3 Monate, verglichen.

Wie in Tabelle 6 und Abbildung 3 zu erkennen ist, führten TTS-Formulierungen mit Rotigotin in Kombination mit einem oder mehreren nicht silanolreduzierte Silikonklebern (BIO-PSA 7-4501 und BIO-PSA 7-4601; Dow Corning^{®}) (nicht silanolreduzierte nicht aminresistente Silikonkleber) zu einer relativ geringen Klebkraft. Diese relativ geringe Klebkraft nahm bei Lagerungszeiten von 1 und 3 Monaten bei 40 °C/ 75% rF noch stark ab, so dass sie nach 1 Monat Lagerung bereits zu einer geringeren Klebkraft als das Neupro^{®} Produkt führte. Bei der Placebo Formulierung erfolgte kein Abfall der Klebkraft trotz Verwendung von nicht aminresistenten Silikonklebern.

Überraschenderweise zeigten Formulierungen von silanolreduzierten nicht aminresistenten Silikonklebern (BIO-PSA SRS7-4501 und BIO-PSA SRS7-4601 von Dow Corning^{®}) mit Rotigotin, obwohl diese weniger freie Silanolgruppen aufweisen und damit weniger Interaktionen mit Oberflächen eingehen können als die nicht-silanolreduzierten -Silikonkleber, eine deutlich höhere Klebkraft und auch einen geringeren relevanten Klebkraftabfall, so dass der absolute Klebkraftwert nach 0 bis 3 Monaten Lagerung bei 40 °C/ 75% rF immer noch höher war als bei dem Neupro^{®} Produkt.

Des Weiteren wiesen Formulierungen mit Silikonkleberverhältnissen von ca. 17,5-30,0 Gew.-% BIO-PSA SRS7-4501 : 82,5-70,0 Gew.-% BIO-PSA SRS7-4601 bezogen auf den Gesamtanteil der Silikonkleber BIO-PSA SRS7-4501 und BIO-PSA SRS7-4601 eine hohe Klebkraft auf und führten nicht zu einem relevanten Trennkraftanstieg (vgl. Abb. 4 und Tabelle 6), im Gegensatz zu Formulierungen mit nur BIO-PSA SRS7-4601 (Dow Corning^{®}) (vgl. Abb. 3).

Überraschenderweise führte die Zugabe von Paraffin in geringen Mengen von ca. 1-3 Gew.-% zu den Formulierungen zu einer deutlich höheren Klebkraft, die einen etwas geringeren Abfall während der Lagerung über 1-3 Monate aufwies (vgl. Abb. 3 und Tabelle 6).

**Tabelle 6: Klebkraft (adhesion strength) von Silikonkleberformulierungen mit nicht silanolreduzierten Silikonklebern und mit silanolreduzierten Silikonklebern in verschieden Polymerkleber-Mischungsverhältnissen mit oder ohne Paraffin bei Lagerung über 0-3 Monaten bei 40 °C/ 75% rF.**

| Batch | Klebertyp / Mischungsverhältnis | Klebkraft nach 0 mon | Klebkraft nach 1 mon bei 40°C/75% rF (rh) | Klebkraft nach 3 mon bei 40°C/75% rF (rh) | Klebkraft nach 4 mon bei RT | Klebkraft nach 7.5 mon bei RT |
|---|---|---|---|---|---|---|
| 583ROTTDS | 7-4601 | 1.44 (7%RSD) | 0.51 (12%RSD) | 0.19 (45%RSD) | ./. | ./. |
| 586ROTTDS | 7-4501 :4601 / 1:3 | 0.71 (10%RSD) | 0.22 (67%RSD) | 0.12 (48%RSD) | ./. | ./. |
| 577ROTTDS | SRS7-4501 | 0.28 (24%RSD) | 0.07 (55%RSD) | 0.09 (28%RSD) | ./. | ./. |
| Neupro^{®} lot: 55338204 | | 0.35 (56%RSD) | ./. | ./. | ./. | ./. |
| 534ROTTDS | SRS7-4501:4601 / 1:1 | ./. | 4.20 (7%RSD) | 3.82 (2%RSD) | 4.37 (2%RSD) | 4.74 (4%RSD) |
| 576ROTTDS | SRS7-4601 | 3.53 (7%RSD) | 2.22 (21%RSD) | 1.81 29%RSD) | ./. | ./. |
| 539ROTTDS | SRS7-4501:4601 / 1:3 | 4.21 (3%RSD) | 3.76 (20%RSD) | 1.18 (31 %RSD) | ./. | ./. |
| 571_568ROTTDS | RS: SRS7-4501 | 5.68 (5%RSD) | 5.68 (3%RSD) | 4.86 (4%RSD) | ./. | ./. |
| | HS: SRS7-4601 | | | | | |
| 592_591ROTTDS | RS: SRS7-4501 | 3.52 (14%RSD) | 2.23 (27%RSD) | 0.79 (8%RSD) | ./. | ./. |
| | HS: SRS7-4601 | | | | | |
| 618_617ROTTDS | RS: SRS7-4501 | 6.65 (7%RSD) | 4.60 (25%RSD) | ./. | ./. | ./. |
| | HS: SRS7-4501:SRS7-4601 / 1:3, 2.4% Paraffin | | | | | |
| 574ROTTDS | SRS7-4501 2% Paraffin | 2.90 (11%RSD) | 3.11 (11%RSD) | 1,95 (14%RSD) | ./. | ./. |
| 602ROTTDS | 7-4501:4601 / 1:3 1% Paraffin | 1.93 (4%RSD) | 0.95 (25%RSD) | 0.42 (27%RSD) | ./. | ./. |
| 611ROTTDS | 7-4501:4601 / 1:3 2% Paraffin | 3.33 (8%RSD) | 1,28 (18%RSD) | 0.95 (18%RSD) | ./. | ./. |
| 614ROTTDS | 7-4601 2% Paraffin | 3.67 (6%RSD) | 1,43 (8%RSD) | ./. | ./. | ./. |
| 616ROTTDS | SRS7-4501:4601 / 1:3 2.1% Paraffin | 4.63 (6%RSD) | ./. | ./. | ./. | ./. |

### C) Klebrigkeit

Schließlich wurde die Klebrigkeit (auch "Tack" genannt) von verschiedenen TTS-Formulierungen umfassend Rotigotin und unterschiedliche nicht-aminreistenten Silikonkleber und - klebergemische mit einer Placebo-Formulierung und dem auf dem Markt erhältlichen Neupro^{®} nach Lagerung über 0 bis 3 Monate verglichen.

Wie in Tabelle 7 und Abbildung 5 klar zu erkennen ist, führten TTS-Formulierungen mit Rotigotin in Kombination mit einem oder mehreren nicht silanolreduzierten Silikonklebern (BIO-PSA 7-4501 und BIO-PSA 7-4601 von Dow Corning^{®}) mit noch freien Silanolgruppen (nicht-silanolreduzierte nicht aminresistente Silikonkleber) zu einer geringeren Klebrigkeit (Tack) als das Neupro^{®} Produkt.

Überraschenderweise war die Klebrigkeit bei Verwendung von silanolreduzierten nicht aminresistenten Silikonklebern deutlich höher, sank bei Lagerung bei 40 °C/75% rF nach 1 Monat nur leicht ab und stabilisierte sich dann nach 3 Monaten. Außerdem ließ sich diese Klebrigkeit durch Zugabe von einer kleinen Menge an Paraffin, über die Klebrigkeit des Neupro^{®} Produkts hinaus, erhöhen und überraschenderweise sank die Klebrigkeit bei Verwendung von einer kleinen Menge an Paraffin (ca. 2 Gew.-%) weniger stark ab während der Lagerung.

**Tabelle 7: Klebrigkeit (Tack) von Silikonkleberformulierungen mit nicht silanolreduzierten Silikonklebern und mit silanolreduzierten Silikonklebern in verschieden Polymerkleber-Mischungsverhältnissen mit oder ohne Paraffin bei Lagerung über 0-3 Monaten bei 40 °C/ 75% rF.**

| Batch | Klebertyp / Mischungsverhältnis | Tack nach 0 mon | Tack nach 1 mon bei 40°C/75% rF (rh) | Tack nach 3 mon bei 40°C/75% rF (rh) |
|---|---|---|---|---|
| 583ROTTDS | 7-4601 | 1.13 (5%RSD) | 0.90 (11%RSD) | 0.60 (28%RSD) |
| 586ROTTDS | 7-4501 :4601 / 1:3 | 0.89 (15%RSD) | 0.62 (20%RSD) | 0.41 (30%RSD) |
| 577ROTTDS | SRS7-4501 | 0.54 (10%RSD) | 0.18 (18%RSD) | 0.45 (50%RSD) |
| Neupro^{®} lot: 55338204 | | 1.45 (8%RSD) | ./. | ./. |
| 534ROTTDS | SRS7-4501:4601 / 1:1 | ./. | 2.02 (16%RSD) | ./. |
| 576ROTTDS | SRS7-4601 | 1.71 (9%RSD) | 1.18 (2%RSD) | 1.14 (11%RSD) |
| 588ROTTDS | SRS7-4501:4601 / 1:3 | 1.55 (16%RSD) | 1.22 (2%RSD) | 1.06 (6%RSD) |
| 571_568ROT TDS | RS: SRS7-4501 | 2.01 (9%RSD) | ./. | 2.01 (4%RSD) |
| | HS: SRS7-4601 | | | |
| 592_591ROT TDS | RS: SRS7-4501 | 2.06 (14%RSD) | 1.41 (22%RSD) | 0.97 (6%RSD) |
| | HS: SRS7-4601 | | | |
| 618_617ROT TDS | RS: SRS7-4501 | 2.25 (9%RSD) | 1.53 (24%RSD) | ./. |
| | HS: SRS7-4501:SRS7-4601 / 1:3, 2.4% Paraffin | | | |
| 574ROTTDS | SRS7-4501 2% Paraffin | 1.69 (5%RSD) | 1.50 (3%RSD) | ./. |
| 599ROTTDS | SRS7-4501 1% Paraffin | 0.80 (28%RSD) | 0.62 (12%RSD) | 0.39 (16%RSD) |
| 611ROTTDS | 7-4501:4601 1:3 2% Paraffin | 1.32 (3%RSD) | 1.00 (36%RSD) | 0.67 (5%RSD) |
| 616ROTTDS | SRS7-4501:4601 / 1:3 2.1% Paraffin | 2.31 (1%RSD) | ./. | ./. |

### BEISPIEL 3 (Rekristallisierung bei Lagerung)

Ziel war die Untersuchung des Einflusses der Menge an PVP K90 auf die Rekristallisierung von Rotigotin und die Permeation. Bei Verwendung eines Gemisches der nicht aminresistenten Silikonkleber BIO-PSA SRS7-4501 und BIO-PSA SRS7-4601 (Dow Corning^{®}) in der Matrixschicht zusammen mit Rotigotin und PVP K90 wurde bei einem Gewichtsverhältnissen von Rotigotin : PVP K90 im Bereich von 9:3,2 bis 9:5 nach Lagerung bei 25 °C und 40 °C Kristallbildung beobachtet.

Überraschenderweise verhinderte die Verwendung des gleichen Gemisches der nicht aminresistenten Silikonklebern BIO-PSA SRS7-4501 und BIO-PSA SRS7-4601 (Dow Corning^{®}) in der Matrixschicht zusammen mit Rotigotin und PVP K90, bei Gewichtsverhältnissen von Rotigotin : PVP K90 von 9:7 oder kleiner die Kristallbildung bei den gleichen Lagerungstemperaturen.

**Tabelle 8: Rekristallisierung bei Lagerung bei 2-8°C, 25°C /60% rF oder bei 40 °C/ 75% rF**

| Batch | 506ROTTDS | 525ROTTDS | 526ROTTDS | 511ROTTDS bzw. 538ROTTDS | 513ROTTDS | 554ROTTDS |
|---|---|---|---|---|---|---|
| Flächengewicht [g/m²] | 50 | 50 | 50 | 50 | 50 | 50 |
| Rotigotin : PVP K90 Verhältnis [w : w] | 9 : 3.2 | 9 : 5 | 9 : 6 | 9 : 7 | 9 : 8 | 9 : 9 |
| Lagerdauer Kühlschrank 2-8°C | 22 Wochen **Kristalle sichtbar** | 12 Monate keine Kristalle sichtbar | 12 Monate keine Kristalle sichtbar | 15 Monate keine Kristalle sichtbar | 14.5 Monate keine Kristalle sichtbar | 5 Monate keine Kristalle sichtbar |
| Lagerdauer 25°C/60° rF | 6 Wochen **Kristalle sichtbar** | 10 Monate **Kristalle sichtbar** | 10 Monate möglicherweise **Kristalle sichtbar** | 16 Monate keine Kristalle sichtbar | 14.5 Monate keine Kristalle sichtbar | 5 Monate keine Kristalle sichtbar |
| Lagerdauer 40°C/75° rF | 3 Wochen **Kristalle sichtbar** | 10 Monate **Kristalle sichtbar** | 10 Monate **Kristalle sichtbar** | 15 Monate keine Kristalle sichtbar | 14.5 Monate keine Kristalle sichtbar | 5 Monate keine Kristalle sichtbar |

### BEISPIEL 4 (in vitro Permeation und Dissolution)

### Beschreibung der in-vitro Dissolution Experimente

Proben ohne Release Liner einer bestimmten Größe (z.B. 10 cm²) wurden per Rotating Cylinder nach Ph. Eur. 2.9.4 (Method 3) bzw. USP <724> apparatus 6 (Zylinder mit Adapter) freigesetzt. Als Freisetzungsmedium wurden je Probe 900 mL von 50 mM Phosphatpuffer (pH 4,5) verwendet. Freisetzungstemperatur war 32°C, bei einer Rotationsgeschwindigkeit der Zylinder von 50 Upm. Probenzüge erfolgten abhängig von der zu untersuchenden Formulierung bei z.B. 0,25; 0,5; 0,75; 1; 1,5; 2; 2,5; und 3 Stunden (Monolayer) und z.B. 0,5; 1; 1,5; 2; 2,5; 3; 4; und 6 Stunden (Bilayer). Die Probenlösungen wurden direkt per RP-HPLC analysiert, wie im Folgenden kurz beschrieben:
Stationäre Phase: C18 (z.B. 50x3 mm, 5 µm Partikelgröße, 35°C Ofentemperatur). Mobile Phase: 70mM Phosphatpuffer (pH 5,0)/Methanol; 55/45 (v/v), mit Fluss 0,6 mL/min. Injektionsvolumen: 20 µL, mit Detektion bei 223 nm, Retentionszeit Rotigotin ca. 3-6 Minuten, Laufzeit 8 Minuten (isokratisch). Auswertung erfolgte über 1-Punkt Kalibration mittels externer Standardlösung. Berechnung der kumulativen Freisetzung [%] auf Basis ermittelter Konzentration in Probelösungen.

### Beschreibung der in-vitro Hautpermeation für Rotigotin

Die Untersuchungen zur in-vitro Hautpermeation wurden mit einer Hautpermeationsanlage von NovoCell Schönbach nach OECD (2004) Test Guideline 428 "Skin absorption: In vitro Method & Series on testing and assessment", No. 28 "Guidance document for the conduct of skin absorption studies" durchgeführt. Eine Messzelle wurde während der gesamten Messung auf 32 ± 1 °C temperiert. Die Messzelle besteht aus einer Donor- und Akzeptor Kammer, welche voneinander durch eine auf einer Cellulose-Membran aufliegenden hitzeseparierten Epidermis menschlicher Haut mit einer effektiven Permeationsfläche von 1,05 cm² getrennt sind. Die Matrix des zu prüfenden Pflasters (Größe ca. 1,2 cm²) war auf das Stratum Corneum aufgeklebt, die freizusetzende Oberfläche war der Akzeptorkammer zugewandt. Die Messzelle beinhaltete ein Gesamtvolumen von 15 mL und war mit physiologischem Phosphatpuffer pH 5,5 gefüllt. Zu definierten Zeitpunkten (z.B. 1, 2, 3, 6, 9, 12, 15, 18, 21 und 24 Stunden) wurden Aliquote als Proben aus der Aktzeptorkammer entnommen, per RP-HPLC Analytik die Konzentration von Rotigotin bestimmt, und sogleich entnommenes Aliquot durch frischen Puffer ersetzt. Eine homogene Verteilung von Temperatur und Konzentration von Rotigotin wurde durch ein integriertes Magnetrührsystem in der Akzeptorkammer gewährleistet.

Die Durchführung der RP-HPLC Analytik und Berechnung der Probenkonzentrationen erfolgte wie bei in-vitro Dissolution (siehe oben). Im Anschluss wurde die kumulierte permeierte Menge pro Zeitpunkt berechnet und in einer Grafik gegen die Zeit aufgetragen, und dann der Steady State flux berechnet [µg/cm²/h].

### A) Monolayer: Einfluss des Verhältnisses von Rotigotin zu PVP K90 und von Parraffin auf die Permeation

Die Hautpermeation wurde durch Applikation der Testformulierungen auf humane hitzeseparierte Epidermis (HSE: heat separated epidermis) untersucht. Dabei wurden verschiedene Gewichtsverhältnisse von Rotigotin zu PVP K90, mit oder ohne Paraffin als Klebkraft- und Klebrigkeit (Tack)-erhöhende Substanz, getestet.

Bisherige Monolayer-Formulierungsansätze mit Polyacrylatklebern, Mischungen von Silikonklebern mit Polyacrylaten, Polyisobutylen/Polybutylen, Styrene-Butadien bzw. Styren-Isopren/Harzformulierungen, sowie Bilayer Formulierungen unter Nutzung einer initial wirkstofffreien Haftkleberschicht mit besagten Klebern führten ausnahmslos zu einer relevant geringeren Permeation bei Applikation auf humaner hitzeseparierter Epidermis (HSE) im Vergleich zum Neupro^{®}.

Der Einfluss verschiedener Rotigotin: PVP Verhältnisse bei fixem Rotigotin Gehalt von 9 % auf die *in vitro* Permeation bei Applikation der Monolayer-Testformulierungen auf humane HSE (heat separated epidermis) wurde untersucht.

Wie aus Abb. 6 A) ersichtlich ist, hatte die eingesetzte Menge an PVP K90 im Bereich von 6.4-9.0 % bei den verwendeten Rotigotin: PVP K90 Verhältnissen keinen relevanten Einfluss auf die *in vitro* Permeation. Außerdem zeigten die Monolayer-Formulierungen eine analoge, und sogar minimal höhere/effektivere Permeation als Neupro^{®}.

Der Einfluss verschiedener Mischungsverhältnisse von BIO-PSA SRS7-4501 zu BIO-PSA SRS7-4601 bei fixem Rotigotin Gehalt (9 Gew.-%) und PVP K90 Gehalt (7 Gew.-%) auf die *in vitro* Permeation bei Applikation von Monolayer-Testformulierungen auf humane HSE (heat separated epidermis) wurde untersucht.

Wie aus Abb. 6 B) ersichtlich ist, hatten bei den Monolayer-Formulierungen die eingesetzten Mischungsverhältnisse an BIO-PSA SRS7-4501 zu SRS7-4601 von 1:1, 1:1,5, 1:2 und 1:3 keinen relevanten Einfluss auf die *in vitro* Permeation bei Applikation der Testformulierungen auf humane HSE (heat separated epidermis). Auch zeigten die Monolayer-Formulierungen mit BIO-PSA SRS7-4501 zu SRS7-4601 Mischungsverhältnissen von 1:1 bis 1:3 eine analoge und minimal höhere/effektivere Permeation als Neupro^{®}.

Schließlich wurde der Einfluss einer kleiner Menge Paraffin auf die *in vitro* Permeation bei Applikation von Monolayer-Testformulierungen auf humane HSE (heat separated epidermis) untersucht.

Bei der Monolayer-Formulierung mit silanolreduziertem nicht aminresistenten Silikonkleber BIO-PSA SRS7-4501, bei einer Paraffinmenge von 2 Gew.-%, zeigte sich ein positiver Effekt auf die *in vitro* Permeation, wobei die Permeation im Vergleich zu Neupro^{®} leicht erhöht/effektiver war (Abb. 7 A).

Bei Monolayer-Formulierungen mit nicht silanolreduzierten nicht aminresistenten Silikonklebern BIO-PSA 7-4501 und/oder BIO-PSA 7-4601, bei einer Paraffinmenge von 1-2 Gew.-%, zeigte sich ein positiver Effekt auf die *in vitro* Permeation. Im Vergleich zu dem Neupro^{®} Produkt war die Permeation leicht erhöht/effektiver. Auch zeigten die mit Paraffin verbesserten Formulierungen eine höhere Permeation als die Formulierungen mit diesen nicht silanolreduzierten nicht aminresistenten Silikonklebern ohne Paraffin (Abb. 7 B).

### B) Bilayer: Einfluss verschiedener wirkstofffreier Haftkleberschichten auf die Permeation

Der Einfluss verschiedener initial wirkstofffreier Haftkleberschichten (Adhesive Layer) basierend auf verschiedenen Klebersystemen in Kombination mit einer wirkstoffhaltigen silikonkleberbasierten Matrixschicht (Bilayerformulierungen) auf die *in vitro* Permeation bei Applikation auf humane HSE (heat separated epidermis) im Vergleich zu Neupro^{®} wurde untersucht.

Der Einsatz von initial wirkstofffreien Haftkleberschichten beschichtet zu ca. 30 g/m² basierend auf Polyacrylatkleber, Polyisobutylen/Polybutylen oder einer Mischung aus Styrene Isobutatien, Harz und Paraffin waren der Bilayer-Formulierung basierend auf einem wirkstofffreien Haftkleberschicht aus nicht aminresistentem Silikonkleber SRS7-4601 deutlich unterlegen (vgl. Abb. 8 A). Bei allen Formulierungen wurde dieselbe Matrixschicht mit 15 Gew.-% Rotigotin, 8,5 Gew.-% PVP K90 und 76,5 Gew.-% BIO-PSA SRS7-4501 Kleber, beschichtet zu ca. 30 g/m², verwendet. Auch zeigte der Bilayer mit nicht aminresistentem Silikonkleber in der initial wirkstofffreien Haftkleberschicht und in der Matrixschicht eine erhöhte/verbesserte Permeation im Vergleich zu Neupro^{®}.

### C) In vitro Dissolution

Wie Abbildung 8 B) zeigt, hatten die eingesetzten Mischungsverhältnisse an BIO-PSA SRS7-4501 zu SRS7-4601 von 1:1 und 1:3 keinen relevanten Einfluss auf die *in vitro* Freisetzung, da sich 538ROTTDS und 539ROTTDS nahezu identisch verhielten. Jedoch zeigten die Formulierungen mit 9 Gew.-% Rotigotin und 7 Gew.-% PVP K90 eine initial leicht schnellere *in vitro* Freisetzung als Neupro^{®}, was an dem höheren Gehalt an PVP K90 liegen kann.

Abbildung 9 zeigt die Kumulativ freigesetzte Menge an Rotigotin über 6 h, bei Bilayer-Formulierung per *in vitro* Dissolution, bei der die Masse der wirkstoffhaltigen Matrixschicht (2) vor dem jeweiligen Beschichten zu ca. 50 g/m² jeweils nur gerührt wurde und dieselbe Masse vor dem jeweiligen Beschichten zu 50 g/m² zusätzlich homogenisiert wurde. Daraufhin wurden beide wirkstoffhaltigen Matrixschichten (2) mit derselben initial wirkstofffreien Haftklebeschicht (3) laminiert. Als weiterer Vergleich wurde eine Monolayer-Formulierung analog behandelt, wobei keine initial wirkstofffreie Haftklebeschicht (3) laminiert wurde. Nur langsames Rühren führte zu größeren Sphären mit breiterer Größenverteilung verglichen mit nachträglichem zusätzlichem Homogenisieren. Beide zeigten einen identischen Freisetzungsverlauf.

## Patentansprüche

1. Transdermales therapeutisches System umfassend
a) eine Rückschicht (1),
b) eine Matrixschicht (2), die einen Arzneistoff enthält, und
c) eine vor Gebrauch zu entfernende Schutzfolie (4),
wobei es sich bei dem Arzneistoff um Rotigotin handelt,
wobei die Matrixschicht (2) einen oder mehrere nicht aminresistente druckempfindliche Silikonkleber in einer Menge von mehr als 50 Gew.-% bezogen auf das Gesamtgewicht der druckempfindlichen Haftkleber der Matrixschicht (2), und Paraffin in einer Menge von mindestens 0,1 Gew.-% bezogen auf das Gesamtgewicht der Matrixschicht (2), enthält, wobei die Rückschicht eine Polyesterfolie mit einer Dicke von unter 35 µm umfasst oder aus einer Polyesterfolie mit einer Dicke von unter 70 µm besteht, und wobei das Rotigotin in der Matrixschicht (2) im Wesentlichen in einer nicht-kristallinen Form in der dispergierten Phase einer festen Dispersion umfassend ein Polyvinylpyrrolidon vorliegt.

2. Transdermales therapeutisches System nach Anspruch 1, wobei die Matrixschicht (2) Paraffin in einer Menge von 0,2 - 20,0 Gew.-%, bevorzugt 0,5 - 10,0 Gew.-%, bevorzugter 1,0 - 4,0 Gew.-%, bezogen auf das Gesamtgewicht der Matrixschicht (2), enthält.

3. Transdermales therapeutisches System nach einem der vorhergehenden Ansprüche, wobei die Matrixschicht (2) ein Flächengewicht von 40-70 g/m², bevorzugt 50-60 g/m², aufweist.

4. Transdermales therapeutisches System nach einem der Ansprüche 1-3, bei dem sich zwischen der Matrixschicht (2) und der vor Gebrauch zu entfernenden Schutzfolie (4) mindestens eine zusätzliche initial wirkstofffreie Haftkleberschicht (3) befindet, wobei die mindestens eine zusätzliche initial wirkstofffreie Haftkleberschicht (3) einen oder mehrere nicht aminresistente druckempfindliche Silikonkleber in einer Menge von mehr als 50 Gew.-% bezogen auf das Gesamtgewicht der druckempfindlichen Haftkleber der mindestens einen zusätzlichen initial wirkstofffreien Haftkleberschicht (3), und Paraffin in einer Menge von mindestens 0,1 Gew.-%, bevorzugt 0,2 - 20,0 Gew.-%, bevorzugter 1,0 - 5,0 Gew.-%, bezogen auf das Gesamtgewicht der mindestens einen zusätzlichen initial wirkstofffreien Haftkleberschicht (3), enthält.

5. Transdermales therapeutisches System nach Anspruch 4, wobei die mindestens eine zusätzliche initial wirkstofffreie Haftkleberschicht (3) ein Flächengewicht von 20-40 g/m², bevorzugt 25-35 g/m², aufweist.

6. Transdermales therapeutisches System nach einem der vorhergehenden Ansprüche, wobei die Matrixschicht (2) einen Gewichtsanteil an nicht aminresistentem druckempfindlichem Silikonkleber von mehr als 75 Gew.-%, bevorzugt mehr als 90 Gew.-%, bezogen auf das Gesamtgewicht der druckempfindlichen Haftkleber der Matrixschicht (2) aufweist.

7. Transdermales therapeutisches System nach einem der Ansprüche 4 bis 6, wobei die mindestens eine zusätzliche initial wirkstofffreie Haftkleberschicht (3) einen Gewichtsanteil an nicht aminresistentem druckempfindlichem Silikonkleber von mehr als 75 Gew.-%, bevorzugt mehr als 90 Gew.-%, bezogen auf das Gesamtgewicht der druckempfindlichen Haftkleber der mindestens einen zusätzlichen initial wirkstofffreien Haftkleberschicht (3) aufweist.

8. Transdermales therapeutisches System nach einem der vorhergehenden Ansprüche, wobei die Matrixschicht (2) und, falls vorhanden, die mindestens eine zusätzliche initial wirkstofffreie Haftkleberschicht (3), als druckempfindliche Haftkleber ausschließlich nicht aminresistente druckempfindliche Silikonkleber aufweisen.

9. Transdermales therapeutisches System nach einem der Ansprüche 1-8, wobei das Gewichtsverhältnis von Rotigotin zu Polyvinylpyrrolidon höchstens 9 : 7, vorzugsweise 9 : 7 bis 9 : 10, beträgt.

10. Transdermales therapeutisches System nach einem der Ansprüche 1-9, bei dem der Wirkstoffgehalt in der Matrixschicht (2) im Bereich von 6 Gew.-% bis 20 Gew.-%, bevorzugt im Bereich von 6,5 Gew.-% bis 11,5 Gew.-%, bezogen auf das Gesamtgewicht der Matrixschicht (2), liegt.

11. Transdermales therapeutisches System nach einem der vorhergehenden Ansprüche, wobei der eine oder die mehreren nicht aminresistenten druckempfindlichen Silikonkleber der Matrixschicht (2) und/oder der mindestens einen zusätzlichen initial wirkstofffreien Haftkleberschicht (3), falls mindestens eine zusätzliche initial wirkstofffreie Haftkleberschicht (3) vorhanden ist, zwei oder mehr nicht aminresistente druckempfindliche Silikonkleber umfasst, wobei vorzugsweise einer oder mehrere der nicht aminresistenten druckempfindlichen Silikonkleber eine mittlere Klebrigkeit und einer oder mehrere der nicht aminresistenten druckempfindlichen Silikonkleber eine hohe Klebrigkeit aufweist.

12. Transdermales therapeutisches System nach einem der vorhergehenden Ansprüche, wobei der eine oder die mehreren nicht aminresistenten druckempfindlichen Silikonkleber der Matrixschicht (2) und/oder der mindestens einen zusätzlichen initial wirkstofffreien Haftkleberschicht (3), falls mindestens eine zusätzliche initial wirkstofffreie Haftkleberschicht (3) vorhanden ist, ausschließlich aus silanolreduziertem nicht aminresistentem druckempfindlichem Silikonkleber besteht.

13. Transdermales therapeutisches System nach einem der vorhergehenden Ansprüche, wobei die Matrixschicht (2) ein oder mehrere Antioxidantien und/oder NatriumMetabisulfit enthält.

14. Transdermales therapeutisches System nach einem der Ansprüche 1-13 zur Verwendung bei der Behandlung der Parkinson-Erkrankung.

15. Verfahren zur Herstellung eines transdermalen therapeutischen Systems nach einem der Ansprüche 1-3, 6 und 8-14 als Monolayer-Formulierung, umfassend die Schritte
a) Herstellen einer homogenisierten Beschichtungsmasse durch Zusammengegeben aller Bestandteile der Matrixschicht (2) in einem geeigneten Lösemittel und Vermischen bis zur gewünschten Homogenität;
b) Aufbringen der homogenisierten Beschichtungsmasse auf eine Rückschicht (1), oder bevorzugt auf eine Schutzfolie (4), und Entfernen des Lösemittels durch Trocknen; und
c) Kaschieren der verbleibenden Schicht, also eine Schutzfolie (4) oder bevorzugt eine Rückschicht (1), auf die Matrixschicht (2), und Ausstanzen transdermaler therapeutischer Systeme geeigneter Größe.

16. Verfahren zur Herstellung eines transdermalen therapeutischen Systems nach einem der Ansprüche 4-14, umfassend die Schritte
a) Herstellen einer ersten Vorstufe des transdermalen therapeutischen Systems, umfassend
a1) Herstellen einer ersten homogenisierten Beschichtungsmasse durch Zusammengegeben aller Bestandteile der Matrixschicht (2) in einem geeigneten Lösemittel und Vermischen bis zur gewünschten Homogenität;
a2) Aufbringen der ersten homogenisierten Beschichtungsmasse auf eine Rückschicht (1), oder bevorzugt auf eine vorläufige Schutzfolie (4), und Entfernen des Lösemittels durch Trocknen; und
a3) Kaschieren der verbleibenden Schicht, also eine vorläufige Schutzfolie (4) oder bevorzugt eine Rückschicht (1), auf die Matrixschicht (2);
b) Herstellen einer zweiten Vorstufe des transdermalen therapeutischen Systems, umfassend
b1) Herstellen einer weiteren homogenisierten Beschichtungsmasse durch Zusammengegeben aller Bestandteile der mindestens einen zusätzlichen initial wirkstofffreien Haftkleberschicht (3) in einem geeigneten Lösemittel und Vermischen bis zur gewünschten Homogenität;
b2) Aufbringen der weiteren homogenisierten Beschichtungsmasse auf eine Schutzfolie (4) und Entfernen des Lösemittels durch Trocknen; und
c) Entfernen der vorläufigen Schutzfolie (4) von der ersten Vorstufe des transdermalen therapeutischen Systems aus a), Kaschieren der ersten Vorstufe und der zweiten Vorstufe des transdermalen therapeutischen Systems zu einem Gesamtlaminat umfassend, in dieser Schichtreihenfolge, eine Rückschicht (1), eine wirkstoffhaltige Matrixschicht (2), mindestens eine zusätzliche initial wirkstofffreie Haftkleberschicht (3) und eine Schutzfolie (4), und Ausstanzen transdermaler therapeutischer Systeme geeigneter Größe.

## Claims

1. A transdermal therapeutic system comprising
a) a backing layer (1),
b) a matrix layer (2) containing a drug; and
c) a release liner (4) to be removed before use,
wherein the drug is rotigotine,
wherein the matrix layer (2) contains one or more non-amine-resistant pressure sensitive silicone adhesives in an amount of more than 50% by weight, based on the total weight of the pressure sensitive adhesives of the matrix layer (2), and paraffin in an amount of at least 0.1% by weight, based on the total weight of the matrix layer (2), wherein the backing layer comprises a polyester film with a thickness of less than 35 µm or consists of a polyester film with a thickness of less than 70 µm, and wherein the rotigotine within the matrix layer (2) is essentially present in a non-crystalline form in the dispersed phase of a solid dispersion comprising a polyvinylpyrrolidone.

2. The transdermal therapeutic system according to claim 1, wherein the matrix layer (2) contains paraffin in an amount of 0.2-20.0% by weight, preferably 0.5-10.0% by weight, more preferably 1.0-4.0% by weight, based on the total weight of the matrix layer (2).

3. The transdermal therapeutic system according to any of the preceding claims, wherein the matrix layer (2) has a weight per unit area of 40-70 g/m², preferably 50-60 g/m².

4. The transdermal therapeutic system according to any of claims 1-3 in which at least one additional initially active ingredient-free pressure sensitive adhesive layer (3) is between the matrix layer (2) and the release liner (4) to be removed before use, wherein the at least one additional initially active ingredient-free pressure sensitive adhesive layer (3) contains one or more non-amine-resistant pressure sensitive silicone adhesives in an amount of more than 50% by weight, based on the total weight of the pressure sensitive adhesives of the at least one additional initially active ingredient-free pressure sensitive adhesive layer (3), and paraffin in an amount of at least 0.1% by weight, preferably 0.2-20.0% by weight, more preferably 1.0-5.0% by weight, based on the total weight of the at least one additional initially active ingredient-free pressure sensitive adhesive layer (3).

5. The transdermal therapeutic system according to claim 4, wherein the at least one additional initially active ingredient-free pressure sensitive adhesive layer (3) has a weight per unit area of 20-40 g/m², preferably 25-35 g/m².

6. The transdermal therapeutic system according to any of the preceding claims, wherein the matrix layer (2) has a weight percentage of non-amine-resistant pressure sensitive silicone adhesive of more than 75% by weight, preferably more than 90% by weight, based on the total weight of the pressure sensitive adhesive of the matrix layer (2).

7. The transdermal therapeutic system according to any of claims 4 to 6, wherein the at least one additional initially active ingredient-free pressure sensitive adhesive layer (3) has a weight percentage of non-amine-resistant pressure sensitive silicone adhesive of more than 75% by weight, preferably more than 90% by weight, based on the total weight of the pressure sensitive adhesive of the at least one additional initially active ingredient-free pressure sensitive adhesive layer (3).

8. The transdermal therapeutic system according to any of the preceding claims, wherein the matrix layer (2) and, if present, the at least one additional initially active ingredient-free pressure sensitive adhesive layer (3), exclusively have non-amine-resistant pressure sensitive silicone adhesives as the pressure sensitive adhesives.

9. The transdermal therapeutic system according to any of claims 1-8, wherein the weight ratio of rotigotine to polyvinylpyrrolidone is at most 9:7, preferably 9:7 to 9:10.

10. The transdermal therapeutic system according to any of claims 1-9, in which the content of active ingredient in the matrix layer (2) is in the range of from 6% by weight to 20% by weight, preferably in the range of from 6.5% by weight to 11.5% by weight, based on the total weight of the matrix layer (2).

11. The transdermal therapeutic system according to any of the preceding claims, wherein the one or more non-amine-resistant pressure sensitive silicone adhesives of the matrix layer (2) and/or the at least one additional initially active ingredient-free pressure sensitive adhesive layer (3), if at least one additional initially active ingredient-free pressure sensitive adhesive layer (3) is present, comprise two or more non-amine-resistant pressure sensitive silicone adhesives, wherein preferably one or more of the non-amine-resistant pressure sensitive silicone adhesives have a medium tack and one or more of the non-amine-resistant pressure sensitive silicone adhesives have a high tack.

12. The transdermal therapeutic system according to any of the preceding claims, wherein the one or more non-amine-resistant pressure sensitive silicone adhesives of the matrix layer (2) and/or the at least one additional initially active ingredient-free pressure sensitive adhesive layer (3), if at least one additional initially active ingredient-free pressure sensitive adhesive layer (3) is present, exclusively consist of a silanol-reduced non-amine-resistant pressure sensitive silicone adhesive.

13. The transdermal therapeutic system according to any of the preceding claims, wherein the matrix layer (2) contains one or more antioxidants and/or sodium metabisulfite.

14. The transdermal therapeutic system according to any of claims 1-13 for use in the treatment of the Parkinson disease.

15. A method for the preparation of a transdermal therapeutic system according to any of claims 1-3, 6, and 8-14 as a monolayer formulation comprising the steps of
a) preparing a homogenized coating mass by adding together all components of the matrix layer (2) in a suitable solvent and mixing to the desired homogeneity;
b) applying the homogenized coating mass onto a backing layer (1) or preferably onto a release liner (4) and removing the solvent by drying; and
c) laminating the remaining layer, i.e. a release liner (4) or preferably a backing layer (1), onto the matrix layer (2), and blanking transdermal therapeutic systems of a suitable size.

16. The method for the preparation of a transdermal therapeutic system according to any of claims 4-14 comprising the steps of
a) preparing a first precursor of the transdermal therapeutic system comprising
a1) preparing a first homogenized coating mass by adding together all the components of the matrix layer (2) in a suitable solvent and mixing to the desired homogeneity;
a2) applying the first homogenized coating mass onto a backing layer (1) or preferably onto a temporary release liner (4) and removing the solvent by drying; and
a3) laminating the remaining layer, i.e., a temporary release liner (4) or preferably a backing layer (1), onto the matrix layer (2);
b) preparing a second precursor of the transdermal therapeutic system comprising
b1) preparing a further homogenized coating mass by adding together all the components of the at least one additional initially active ingredient-free pressure sensitive adhesive layer (3) in a suitable solvent and mixing to the desired homogeneity;
b2) applying the further homogenized coating mass onto a release liner (4) and removing the solvent by drying; and
c) removing the temporary release liner (4) from the first precursor of the transdermal therapeutic system of a), laminating the first precursor and the second precursor of the transdermal therapeutic system to an overall laminate comprising in this layering order a backing layer (1), an active ingredient-containing matrix layer (2), at least one additional initially active ingredient-free pressure sensitive adhesive layer (3) and a release liner (4), and blanking transdermal therapeutic systems of the suitable size.

## Revendications

1. Système thérapeutique transdermique comprenant
a) une couche de support (1),
b) une couche de matrice (2) contenant un médicament, et
c) un film protecteur (4) à retirer avant utilisation,
dans lequel le médicament est la rotigotine,
dans lequel la couche de matrice (2) contient un ou plusieurs adhésifs silicone sensibles à la pression non résistants aux amines en une quantité supérieure à 50 % en poids sur la base du poids total des adhésifs sensibles à la pression de la couche de matrice (2), et de la paraffine en une quantité d'au moins 0,1 % en poids sur la base du poids total de la couche de matrice (2), dans lequel la couche de support comprend un film de polyester d'une épaisseur inférieure à 35 µm ou consiste en un film de polyester d'une épaisseur inférieure à 70 µm, et dans lequel la rotigotine dans la couche de matrice (2) est sensiblement sous une forme non cristalline dans la phase dispersée d'une dispersion solide comprenant une polyvinylpyrrolidone.

2. Système thérapeutique transdermique selon la revendication 1, dans lequel la couche de matrice (2) contient de la paraffine en une quantité de 0,2 - 20,0 % en poids, de préférence de 0,5 - 10,0 % en poids, de préférence encore de 1,0 - 4,0 % en poids, sur la base du poids total de la couche de matrice (2).

3. Système thérapeutique transdermique selon l'une des revendications précédentes, dans lequel la couche de matrice (2) a un poids surfacique de 40-70 g/m², de préférence de 50- 60 g/m².

4. Système thérapeutique transdermique selon l'une des revendications 1-3, dans lequel, entre la couche de matrice (2) et le film protecteur (4) à retirer avant utilisation, il se trouve au moins une couche adhésive supplémentaire initialement exempte de substance active (3), dans lequel la au moins une couche adhésive supplémentaire initialement exempte de substance active (3) contient un ou plusieurs adhésifs silicone sensibles à la pression non résistants aux amines en une quantité supérieure à 50 % en poids, sur la base du poids total des adhésifs sensibles à la pression, de la au moins une couche adhésive supplémentaire initialement exempte de substance active (3), et de la paraffine en une quantité d'au moins 0,1 % en poids, de préférence de 0,2 - 20,0 % en poids, de préférence encore de 1,0 - 5,0 % en poids, sur la base du poids total de la au moins une couche adhésive supplémentaire initialement exempte de substance active (3).

5. Système thérapeutique transdermique selon la revendication 4, dans lequel la au moins une couche adhésive supplémentaire initialement exempte de substance active (3) a un poids surfacique de 20-40 g/m², de préférence de 25-35 g/m².

6. Système thérapeutique transdermique selon l'une des revendications précédentes, dans lequel la couche de matrice (2) présente une proportion pondérale d'adhésif silicone sensible à la pression non résistant aux amines de plus de 75 % en poids, de préférence de plus de 90 % en poids, sur la base du poids total des adhésifs sensibles à la pression de la couche de matrice (2).

7. Système thérapeutique transdermique selon l'une des revendications 4 bis 6, dans lequel la au moins une couche adhésive supplémentaire initialement exempte de substance active (3) présente une proportion pondérale d'adhésif silicone sensible à la pression non résistant aux amines de plus de 75 % en poids, de préférence de plus de 90 % en poids, sur la base du poids total des adhésifs sensibles à la pression de la au moins une couche adhésive supplémentaire initialement exempte de substance active (3).

8. Système thérapeutique transdermique selon l'une des revendications précédentes, dans lequel la couche de matrice (2) et, le cas échéant, la au moins une couche adhésive supplémentaire initialement exempte de substance active (3), ne comportent que des adhésifs silicone sensibles à la pression non résistants aux amines comme adhésifs sensibles à la pression.

9. Système thérapeutique transdermique selon l'une des revendications 1-8, dans lequel le rapport pondéral de la rotigotine à la polyvinylpyrrolidone est d'au plus 9 : 7, de préférence de 9 : 7 à 9 : 10.

10. Système thérapeutique transdermique selon l'une des revendications 1-9, dans lequel la teneur en substance active dans la couche de matrice (2) se situe dans la plage de 6 % en poids à 20 % en poids, de préférence de dans la plage de 6,5 % en poids à 11,5 % en poids, sur la base du poids total de la couche de matrice (2).

11. Système thérapeutique transdermique selon l'une des revendications précédentes, dans lequel, si au moins une couche adhésive supplémentaire initialement exempte de substance active (3) est présente, le ou les adhésif(s) silicone sensible(s) à la pression non résistant(s) aux amines de la couche de matrice (2) et/ou de la au moins une couche adhésive supplémentaire initialement exempte de substance active (3) comprend/comprennent deux ou plusieurs adhésifs silicone sensibles à la pression non résistants aux amines, dans lequel, de préférence, un ou plusieurs des adhésifs silicone sensibles à la pression non résistants aux amines a/ont une adhérence moyenne et un ou plusieurs des adhésifs silicone sensibles à la pression non résistants aux amines a/ont une adhérence élevée.

12. Système thérapeutique transdermique selon l'une des revendications précédentes, dans lequel, si au moins une couche adhésive supplémentaire initialement exempte de substance active (3) est présente, le ou les adhésif(s) silicone sensible(s) à la pression non résistant(s) aux amines de la couche de matrice (2) et/ou de la au moins une couche adhésive supplémentaire initialement exempte de substance active (3) est/sont constitué(s) exclusivement d'adhésif silicone sensible à la pression non résistant aux amines à teneur réduit en silanol.

13. Système thérapeutique transdermique selon l'une des revendications précédentes, dans lequel la couche de matrice (2) contient un ou plusieurs antioxydants et/ou du métabisulfite de sodium.

14. Système thérapeutique transdermique selon l'une des revendications 1-13 destiné à être utilisé dans le traitement de la maladie de Parkinson.

15. Procédé de fabrication d'un système thérapeutique transdermique selon l'une des revendications 1-3, 6 et 8-14 sous forme de formulation monocouche, comprenant les étapes suivantes:
a) production d'une masse de revêtement homogénéisée en combinant tous les composants de la couche de matrice (2) dans un solvant approprié et en mélangeant jusqu'à l'homogénéité souhaitée;
b) application de la masse de revêtement homogénéisée sur une couche de support (1), ou de préférence sur un film protecteur (4), et élimination du solvant par séchage; et
c) lamination de la couche restante, c'est-à-dire un film protecteur (4) ou de préférence une couche de support (1), sur la couche de matrice (2), et poinçonnage de systèmes thérapeutiques transdermiques de taille appropriée.

16. Procédé de fabrication d'un système thérapeutique transdermique selon l'une des revendications 4-14, comprenant les étapes suivantes:
a) production d'un premier précurseur du système thérapeutique transdermique, comprenant
a1) production d'une première masse de revêtement homogénéisée en combinant tous les composants de la couche de matrice (2) dans un solvant approprié et en mélangeant jusqu'à l'homogénéité souhaitée;
a2) application de la première masse de revêtement homogénéisée sur une couche de support (1), ou de préférence sur un film protecteur (4) temporaire, et élimination du solvant par séchage; et
a3) lamination de la couche restante, c'est-à-dire un film protecteur (4) temporaire ou de préférence une couche de support (1), sur la couche de matrice (2);
b) production d'un deuxième précurseur du système thérapeutique transdermique, comprenant
b1) production d'une masse de revêtement homogénéisée additionnelle en combinant tous les composants de la au moins une couche adhésive supplémentaire initialement exempte de substance active (3) dans un solvant approprié et en mélangeant jusqu'à l'homogénéité souhaitée;
b2) application de la masse de revêtement homogénéisée additionnelle sur un film protecteur (4) et élimination du solvant par séchage; et
c) enlèvement du film protecteur (4) temporaire du premier précurseur du système thérapeutique transdermique de a), lamination du premier précurseur et du deuxième précurseur du système thérapeutique transdermique pour former un ensemble laminé comprenant, dans la séquence de couches suivante, une couche de support (1), une couche de matrice (2) contenant un principe actif, au moins une couche adhésive supplémentaire initialement exempte de substance active (3) et un film protecteur (4), et poinçonnage de systèmes thérapeutiques transdermiques de taille appropriée.
